# EUROPEAN PATENT APPLICATION

(11) **EP 2 374 508 A1**
(43) Date of publication of application: **12.10.2011**
(21) Application number: 11172579.2
(22) Date of filing: 21.03.2006
(51) Int. Cl.: A61P 9/00, A61P 11/00, A61P 25/00, A61P 43/00

(54) **Combination of angiotension receptor blockers (ARB) and NSAIDs for use in ameliorating cachexia**

(30) Priority: 21.03.2005 US 664225 P; 31.08.2005 US 713526 P; 10.11.2005 US 735432 P; 22.12.2005 US 753436 P
(62) Divisional of application: 06748577.1
(71) Applicant: Vicus Therapeutics SPE 1, LLC, Morristown NJ 07960 (US)
(72) Inventor: Young, Fredric, Morristown, NJ New Jersey 07960 (US); Maki, John, Morristown, NJ New Jersey 07960 (US); Bascomb, Newell, Morristown, NJ New Jersey 07960 (US)
(74) Representative: Roques, Sarah Elizabeth

(57) **Abstract**

The invention provides a therapeutic combination for use in the treatment of cachexia or of a maladaptive nutritional state secondary to a chronic Systemic Inflammatory Response State (SIRS), wherein the combination comprises at least one member of a first group and at least one member of a second group;
wherein members of the first group are selected from the group consisting of angiotensin receptor blockers (ARBs); and
members of the second group are selected from the group consisting of non-steroidal anti-inflammatory drugs (NSAIDs).

## Description

### FIELD OF THE INVENTION

This invention relates generally to medicine and pharmaceutical formulations. The invention provides compositions, e.g., preparations, formulations, kits and other products of manufacture (e.g., blister packs), comprising combinations of beneficial ingredients that are serviceable as therapies for treating, preventing or improving conditions, states and disease symptoms involving inflammation, excessive sympathoneural drive, cachexia, anorexia, and anorexia-cachexia, and stress or anxiety related thereto, and method of using them. Relevant conditions, states and disease symptoms treated, prevented and/or ameliorated by the compositions and methods of the invention are exemplified by, e.g., wasting and/or atrophy, such as muscle atrophy. In one aspect, the invention provides compositions and therapies comprising use of a beta adrenergic antagonist (also called "beta blockers" e.g., propranolol) in combination with an anti-inflammatory agent, e.g., a nonsteroidal anti-inflammatory drug (NSAID), an angiotensin-converting enzyme (ACE) inhibitor, an angiotensin receptor blocker (ARB), an anabolic steroid, a natural oil or fatty acid or any combination thereof.

### BACKGROUND

Single agent therapies have been used as therapies or suggested for use as therapies for disease symptoms and diseases states such as involving, inflammation, excessive sympathoneural drive, cachexia, anorexia, and anorexia-cachexia, and stress or anxiety related thereto for the purpose of improving one or more undesirable symptoms associated with the disease states or for slowing the progression (worsening) of the symptoms. However, the success achieved with the attempted doses of single agents has been limited, and the obstacles to increasing the doses include legitimate concerns about exceeding the therapeutic windows and/or concerns about the manifestation of undesirable side effects at higher doses.

Angiotensin II augments norepinephrine release from sympathetic nerve terminals by inhibiting reuptake of norepinephrine into nerve terminals and enhancing the vascular response to norepinephrine. Angiotensin II leads to direct vasoconstriction through increased norepinephrine release, decreased reuptake, increased sympathetic activation and release of catecholamines from the adrenal medulla. Compounds that inhibit the enzyme that converts angiotensin I to angiotensin II are frequently used for controlling blood pressure. Known commonly as ACE inhibitors (angiotensin converting enzyme inhibitors) there are approximately 11 structurally related compounds that are most commonly used.

The renin-angiotensin system has a large degree of overlap with the effects of beta blockers and the beta adrenergic receptor pathway has a direct effect on the regulation of rennin release. Therefore, the use of ACE inhibitors has been suggested as a means to treat cachexia. The problem with the use of ACE inhibitors is the same as that for beta blockers, specifically the need to reduce the inflammatory response in addition to autonomic nervous system dysregulation.

Cancer cachexia is a catastrophic weight-loss disorder associated with more than a 40 percent reduction in life expectancy, which limits chemotherapy efficacy and impairs patient tolerance to life-extending therapies. In addition, cachexia is one of the most feared disorders of advanced cancer and significantly affects quality of life. Despite the magnitude of the need, effective treatments for cancer cachexia are lacking, and no FDA approved treatments are available.

Cachexia affects 80% of advanced solid- tumor cancer patients and is associated with significant patient morbidity, including weakness, fatigue, gastrointestinal distress, sleep/wake disturbances, pain, listlessness, shortness of breath, lethargy, depression, malaise and fear of being a burden on family and friends. For more than 20% of terminal cancer patients, cachexia results in the direct cause of death.

Severe cachexia occurs in most patients with advanced cancer, AIDS and other end-stage chronic diseases. Cachexia is a maladaptive nutritional state secondary to a chronic systemic inflammatory response and autonomic nervous system dysregulation. Improved treatment protocols for this complex and devastating disorder are greatly needed. A safe and effective treatment that could alleviate suffering and improve outcomes would be a significant medical advance for cancer treatment and potentially for the treatment other end-stage diseases as well.

To date clinicians treat cachexia with a variety of interventions including hormones and steroids, cytokines, nutritional supplementation, and appetite stimulation. While these may be useful in treating aspects of the disease they must be administered in the correct sequence or combination. For example, adding an anabolic steroid while there is degradation of fat and muscle will cause increased cycling and more energy use since the protein is being rapidly broken down to supply other processes. Likewise, adding only nutritional supplementation will be difficult, since the patient has no appetite and will be ineffective since the use of body energy reserves and nutrients is imbalanced. Possible intervention points and therapeutics that have been suggested include: aberrant eicosanoid metabolism with nonsteroidal anti-inflammatory agents, omega-3 fatty acids; amino acid deficiencies relevant to need with tailored protein supplements, specific amino acid therapy; altered energy mechanisms in muscle cells with creatine, ATP, angiotensin-converting enzyme (ACE) inhibitors; impaired muscle synthesis and muscle repair with anabolic steroids.

### SUMMARY

This invention provides combination preparations and formulations, kits and other products of manufacture (e.g., blister packs) that can be used for treating, preventing and/or ameliorating conditions, diseases, symptoms and disease states comprising inflammation, excessive sympathoneural drive (sympathetic nerve activity), cachexia, anorexia, and anorexia-cachexia, and stress or anxiety related thereto for the purpose of improving one or more undesirable symptoms associated with the disease states or for slowing the progression (worsening) of one or more symptoms associated with these disease symptoms and disease states.

The invention provides therapeutic combinations comprising at least one member of a first group and at least one member of a second group; wherein members of the first group are selected from the group consisting of beta adrenergic receptor antagonists (beta blockers); and members of the second group are selected from the group consisting of angiotensin-converting enzyme (ACE) inhibitors, angiotensin receptor blockers (ARBs), non-steroidal anti-inflammatory drugs (NSAID), anabolic steroids, natural oils and fatty acids and a combination thereof. In one aspect, the therapeutic combination comprises: at least one beta adrenergic receptor antagonist (beta blocker) and at least one angiotensin-converting enzyme (ACE) inhibitors; at least one beta adrenergic receptor antagonist (beta blocker) and at least one non-steroidal anti-inflammatory drug (NSAID); at least one beta adrenergic receptor antagonist (beta blocker) and at least one angiotensin receptor blocker (ARB); at least one beta adrenergic receptor antagonist (beta blocker) and at least one anabolic steroid; or at least one beta adrenergic receptor antagonist (beta blocker) and at least one natural oil or fatty acid.

In one aspect, the therapeutic combinations further comprise at least one member of a third group, wherein the member of the third group is a different composition than the selected member of the first or second group, and members of the third group are selected from the group consisting of angiotensin-converting enzyme (ACE) inhibitors, angiotensin receptor blockers (ARBs), non-steroidal anti-inflamatory drugs (NSAIDs), anabolic steroids, natural oils and fatty acids and a combination thereof. The therapeutic combination can comprise: at least one beta adrenergic receptor antagonist (beta blocker), at least one angiotensin-converting enzyme (ACE) inhibitor and at least one angiotensin receptor blocker (ARB); at least one beta adrenergic receptor antagonist (beta blocker), at least one angiotensin-converting enzyme (ACE) inhibitor and at least one non-steroidal anti-inflammatory drug (NSAID); at least one beta adrenergic receptor antagonist (beta blocker), at least one angiotensin-converting enzyme (ACE) inhibitor and at least one anabolic steroid; at least one beta adrenergic receptor antagonist (beta blocker), at least one angiotensin-converting enzyme (ACE) inhibitor and at least one natural oil or fatty acid; at least one beta adrenergic receptor antagonist (beta blocker), at least one angiotensin receptor blocker (ARB) and at least one non-steroidal anti-inflammatory drug (NSAID); at least one beta adrenergic receptor antagonist (beta blocker), at least one angiotensin receptor blocker (ARB) and at least one anabolic steroid; at least one beta adrenergic receptor antagonist (beta blocker), at least one angiotensin receptor blocker (ARB) and at least one natural oil or fatty acid; at least one beta adrenergic receptor antagonist (beta blocker), at least one non-steroidal anti-inflammatory drug (NSAID) and at least one anabolic steroid; at least one beta adrenergic receptor antagonist (beta blocker), at least one non-steroidal anti-inflammatory drug (NSAID) and at least one natural oil or fatty acid; at least one beta adrenergic receptor antagonist (beta blocker), at least one anabolic steroid and at least one natural oil or fatty acid.

In one aspect, the therapeutic combinations further comprise at least one member of a fourth group, wherein the member of the fourth group is a different composition than the selected member of the first, second or third group, and members of the fourth group are selected from the group consisting of angiotensin-converting enzyme (ACE) inhibitors, angiotensin receptor blockers (ARBs), non-steroidal anti-inflammatory drugs (NSAIDs), anabolic steroids, natural oils and fatty acids and a combination thereof.

In one aspect, the therapeutic combinations further comprise at least one member of a fifth group, wherein the member of the fifth group is a different composition than the selected member of the first, second, third or fourth group, and members of the fifth group are selected from the group consisting of angiotensin-converting enzyme (ACE) inhibitors, angiotensin receptor blockers (ARBs), non-steroidal anti-inflammatory drugs (NSAIDs), anabolic steroids, natural oils and fatty acids and a combination thereof.

In one aspect, the therapeutic combinations further comprise at least one member of a sixth group, wherein the member of the sixth group is a different composition than the selected member of the first, second, third, fourth or fifth group, and members of the sixth group are selected from the group consisting of angiotensin-converting enzyme (ACE) inhibitors, angiotensin receptor blockers (ARBs), non-steroidal anti-inflammatory drugs (NSAIDs), anabolic steroids, natural oils and fatty acids and a combination thereof.

In one aspect, the therapeutic combinations comprise at least one member of the first group and the at least one member of the second group are formulated as separate compositions. In one aspect, the therapeutic combinations comprise at least one member of the first group and the at least one member of the group are formulated in the same composition. In one aspect, each member of each selected group is formulated as a separate composition, or, all selected members are formulated in the same composition, or, a combination thereof. In one aspect, each member of each selected group is manufactured in a separate package or container (e.g., a "blister package"), or, all selected members are manufactured in the same package or container, or, any combination thereof.

The invention provides pharmaceutical compositions comprising a therapeutic combination of the invention; or, therapeutic combination of the invention can be singly or multiply formulated or packaged as one or more pharmaceutical compositions. The pharmaceutical compositions can further comprise any pharmaceutically acceptable excipient. Compositions used in the therapeutic combinations of the invention, e.g., the pharmaceutical compositions of the invention, can be formulated in or as a feed, a food, a liquid, an elixir, an aerosol, a spray, a powder, a tablet, a pill, a capsule, a gel, a geltab, a nanosuspension, a nanoparticle, a microgel or a suppository.

The invention provides therapeutic combinations, e.g., pharmaceutical compositions of the invention, comprising at least one beta adrenergic receptor antagonist (beta blocker), which can comprise at least one atenolol, nadolol, metoprolol, propranolol, carteolol, carvedolol, labetalol, oxprenolol, penbutolol, pindolol, sotalol, timolol or a combination thereof.

In one aspect, invention provides therapeutic combinations, e.g., pharmaceutical compositions of the invention, comprising at least one angiotensin-converting enzyme (ACE) inhibitor, and the angiotensin-converting enzyme (ACE) inhibitor can comprise benazepril, captopril, cilazapril, enalapril, enalaprilat, fosinopril, imidapril, lisinopril, moexipril, perindopril, quinapril, ramipril, trandolapril or a combination thereof.

In one aspect, invention provides therapeutic combinations, e.g., pharmaceutical compositions of the invention, comprising an angiotensin receptor blocker, e.g., comprising candesartan, eprosartan, irbesartan, losartan, olmesartan, telmisartan, valsartan, or a combination thereof.

In one aspect, invention provides therapeutic combinations, e.g., pharmaceutical compositions of the invention, comprising non-steroidal anti-inflammatory drugs (NSAIDs), e.g., comprising aspirin, diclofenac; diflunisal, etodolac, fenoprofen, flurbiprofen, ibuprofen, indomethacin, ketoprofen; Ketorolac, meclofenamate, mefenamic acid, meloxicam, nabumetone, naproxen, oxaprozin, piroxicam, salsalate, sulindac, tolmetin, a COX-2 inhibitor (e.g., a COX-2-selective inhibitor) or a combination thereof, wherein optionally the COX-2-selective inhibitor comprises celecoxib rofecoxib, etoricoxib, valdecoxib, parecoxib, meloxicam or lumiracoxib.

In one aspect, invention provides therapeutic combinations, e.g., pharmaceutical compositions of the invention, comprising steroids, e.g., anabolic steroids, such as andarine, ethylestrenol, mesterolone, methandrostenolone, methenolone, methyltestosterone, oxandrolone, oxymetholone stanozolol, boldenone, hexoxymestrolum, methandrostenolone, methenolone enanthate, nandrolone decanoate, nandrolone phenproprionate, stanozolol, stenbolone, testosterone cypionate, testosterone enanthate, testosteron, testosterone nicotinate, therobolin, trenbolone, trenbolone, trophobolene or a combination thereof.

In one aspect, invention provides therapeutic combinations, e.g., pharmaceutical compositions of the invention, comprising a natural oil or fatty acid, e.g., comprising an omega-3 fatty acid, a fish oil, a long-chain polyunsaturated fatty acid, an n-3 and/or n-6 highly unsaturated fatty acid, eicosapentaenoic acid (EPA), docosahexaenoic acid (DHA) or a combination thereof.

In one aspect, invention provides therapeutic combinations, e.g., pharmaceutical compositions of the invention, and methods using these therapeutic combinations, wherein the therapeutic combinations are formulated for use as a medicament in the treatment of chronic systemic inflammatory stress; burns, chronic obstructive pulmonary disease; congestive heart failure; chronic kidney disease; surgery; cancer; sepsis; ageing; acute respiratory distress syndrome; acute lung injury; infection; a CNS disorder or injury; anemia; immunosuppression; insulin resistance; anorexia; anxiety; sleep disturbances; weakness; fatigue; gastrointestinal distress; sleep disturbances; wake disturbances; pain; listlessness; shortness of breath; lethargy; depression; malaise; or, a combination thereof. The therapeutic combinations can be formulated for use in the treatment or amelioration of a condition or disease comprising a chronic Systemic Inflammatory Response State (SIRS).

The therapeutic combinations can be formulated for or used in the manufacture of a medicament or pharmaceutical composition for treating or ameliorating a trauma, condition or disease comprising: a chronic Systemic Inflammatory Response State (SIRS); chronic systemic inflammatory stress; burns, chronic obstructive pulmonary disease; congestive heart failure; chronic kidney disease; surgery; cancer; sepsis; ageing; acute respiratory distress syndrome; acute lung injury; infection; a CNS disorder or injury; anemia; immunosuppression; insulin resistance; anorexia; anxiety; sleep disturbances; weakness; fatigue; gastrointestinal distress; sleep disturbances; wake disturbances; pain; listlessness; shortness of breath; lethargy; depression; malaise; or, a combination thereof. In one aspect of the use, the trauma, condition or disease comprises a maladaptive nutritional state secondary to the SIRS. In one aspect of the use, the maladaptive nutritional state comprises cachexia, and optionally the cachexia comprises cachexia secondary to cancer. In one aspect of the use, the CNS disorder comprises Parkinson's disease or Alzheimer's disease.

The invention provides methods for treating or ameliorating a trauma, condition or disease comprising a chronic Systemic Inflammatory Response State (SIRS), chronic systemic inflammatory stress; burns, chronic obstructive pulmonary disease; congestive heart failure; chronic kidney disease; surgery; cancer; sepsis; ageing; acute respiratory distress syndrome; acute lung injury; infection; a CNS disorder or injury; anemia; immunosuppression; insulin resistance; anorexia; anxiety; sleep disturbances; weakness; fatigue; gastrointestinal distress; sleep disturbances; wake disturbances; pain; listlessness; shortness of breath; lethargy; depression; malaise; or, a combination thereof, the method comprising the steps of: (a) providing the therapeutic combination of the invention, or the pharmaceutical composition of the invention; and (b) administering a therapeutically effective amount of the therapeutic combination of step (a), thereby treating or ameliorating the trauma, condition or disease.

In one aspect of the methods, the condition or disease comprises a maladaptive nutritional state secondary to the SIRS, or the maladaptive nutritional state comprises cachexia, such as cachexia secondary to cancer.

In one aspect of the methods, the therapeutic combination of the invention, or the pharmaceutical composition of the invention, is administered in single or multiple doses, and optionally the pharmaceutical compositions are packaged in a single or a plurality of packages or packets.

In one aspect of the methods, the therapeutic combination of the invention, or the pharmaceutical composition of the invention, is administered intravenously, topically, orally, by inhalation, by infusion, by injection, intraperitoneally, intramuscularly, subcutaneously, intra-aurally, by intra-articular administration, by intra-mammary administration, by topical administration or by absorption through epithelial or mucocutaneous linings.

The invention provides kits comprising the therapeutic combination of the invention, or the pharmaceutical composition of the invention. The kit can comprise at least one package, e.g., a blister pack, containing any one or more of a therapeutic combination of any of the invention, or the pharmaceutical composition of the invention.

In alternative aspects, the therapeutic combination of the invention, or the pharmaceutical composition of the invention, is formulated for administration intravenously, topically, orally, by inhalation, by infusion, by injection, intraperitoneally, intramuscularly, subcutaneously, intra-aurally, for intra-articular administration, for intra-mammary administration, for topical administration or for absorption through epithelial or mucocutaneous linings.

The therapeutic combination of the invention, or the pharmaceutical composition of the invention, can be packaged for administration intravenously, topically, orally, by inhalation, by infusion, by injection, intraperitoneally, intramuscularly, subcutaneously, intra-aurally, for intra-articular administration, for intra-mammary administration, for topical administration or for absorption through epithelial or mucocutaneous linings.

In one aspect, therapeutic combinations of the invention, or pharmaceutical compositions of the invention (e.g., the multi-ingredient combinations of drugs of the invention), are formulated for chrono-dosing, or the methods of the invention comprise use of chrono-dosing or escalating dosage regimens. For example, in one aspect the therapeutic combinations of the invention, or pharmaceutical compositions of the invention, are formulated for administration once a day, b.i.d. or t.i.d or more often, or for continuous administration. In one aspect, therapeutic combinations of the invention, or pharmaceutical compositions of the invention, are formulated and dosaged as set forth in any one of exemplary ingredient combinations 1 to 90.

The invention provides preparations therapeutic combinations comprising at least one member of a first group and at least one member of a second group; wherein members of the first group are selected from the group consisting of beta adrenergic receptor antagonists (or beta blockers); and members of the second group are selected from the group consisting of angiotensin converting enzyme inhibitors (or ACE inhibitors).

The invention provides preparations therapeutic combinations comprising at least one member of a first group and at least one member of a second group; wherein members of the first group are selected from the group consisting of beta adrenergic receptor antagonists (or beta blockers); and members of the second group are selected from the group consisting of angiotensin receptor blockers (or ARB's).

The invention provides preparations therapeutic combinations comprising at least one member of a first group and at least one member of a second group; wherein members of the first group are selected from the group consisting of beta adrenergic receptor antagonists (or beta blockers); and members of the second group are selected from the group consisting of non-steroidal anti-inflammatory drugs (NSAID's).

The invention provides preparations therapeutic combinations comprising at least one member of a first group and at least one member of a second group; wherein members of the first group are selected from the group consisting of beta adrenergic receptor antagonists (or beta blockers); and members of the second group are selected from the group consisting of a steroid, e.g., an anabolic steroid, such as a steroid as described in Table 1, below.

The invention provides preparations therapeutic combinations comprising at least one member of a first group and at least one member of a second group; wherein members of the first group are selected from the group consisting of beta adrenergic receptor antagonists (or beta blockers); and members of the second group are selected from the group consisting of natural oils and fatty acids.

The invention provides preparations therapeutic combinations comprising at least one member of a first group, at least one member of a second group, and at least one member of a third group; wherein members of the first group are selected from the group consisting of beta adrenergic receptor antagonists (or beta blockers); members of the second group are selected from the group consisting of angiotensin converting enzyme inhibitors (or ACE inhibitors); and members of the third group are selected from the group consisting of angiotensin receptor blockers (or ARB's).

The invention provides preparations therapeutic combinations comprising at least one member of a first group, at least one member of a second group, and at least one member of a third group; wherein members of the first group are selected from the group consisting of beta adrenergic receptor antagonists (or beta blockers); members of the second group are selected from the group consisting of angiotensin converting enzyme inhibitors (or ACE inhibitors); and members of the third group are selected from the group consisting of non-steroidal anti-inflammatory drugs (NSAID's).

The invention provides preparations therapeutic combinations comprising at least one member of a first group, at least one member of a second group, and at least one member of a third group; wherein members of the first group are selected from the group consisting of beta adrenergic receptor antagonists (or beta blockers); members of the second group are selected from the group consisting of angiotensin converting enzyme inhibitors (or ACE inhibitors); and members of the third group comprising a steroid, e.g., an anabolic steroid, such as a steroid as described in Table 1, below.

The invention provides preparations therapeutic combinations comprising at least one member of a first group, at least one member of a second group, and at least one member of a third group; wherein members of the first group are selected from the group consisting of beta adrenergic receptor antagonists (or beta blockers); members of the second group are selected from the group consisting of angiotensin converting enzyme inhibitors (or ACE inhibitors); and members of the third group are selected from the group consisting of natural oils and fatty acids.

The invention provides preparations therapeutic combinations comprising at least one member of a first group, at least one member of a second group, and at least one member of a third group; wherein members of the first group are selected from the group consisting of beta adrenergic receptor antagonists (or beta blockers); members of the second group are selected from the group consisting of angiotensin receptor blockers (or ARB's); and members of the third group are selected from the group consisting of non-steroidal anti-inflammatory drugs (NSAID's).

The invention provides preparations therapeutic combinations comprising at least one member of a first group, at least one member of a second group, and at least one member of a third group; wherein members of the first group are selected from the group consisting of beta adrenergic receptor antagonists (or beta blockers); members of the second group are selected from the group consisting of angiotensin receptor blockers (or ARB's); and members of the third group comprising steroids, e.g., an anabolic steroid, such as a steroid as described in Table 1, below.

The invention provides preparations therapeutic combinations comprising at least one member of a first group, at least one member of a second group, and at least one member of a third group; wherein members of the first group are selected from the group consisting of beta adrenergic receptor antagonists (or beta blockers); members of the second group are selected from the group consisting of angiotensin receptor blockers (or ARB's); and members of the third group are selected from the group consisting of natural oils and fatty acids.

The invention provides preparations therapeutic combinations comprising at least one member of a first group, at least one member of a second group, and at least one member of a third group; wherein members of the first group are selected from the group consisting of beta adrenergic receptor antagonists (or beta blockers); members of the second group are selected from the group consisting of non-steroidal anti-inflammatory drugs (NSAID's); and members of the third group comprising steroids, e.g., an anabolic steroid, such as a steroid as described in Table 1, below.

The invention provides preparations therapeutic combinations comprising at least one member of a first group, at least one member of a second group, and at least one member of a third group; wherein members of the first group are selected from the group consisting of beta adrenergic receptor antagonists (or beta blockers); members of the second group are selected from the group consisting of non-steroidal anti-inflammatory drugs (NSAID's); and members of the third group are selected from the group consisting of natural oils and fatty acids.

The invention provides preparations therapeutic combinations comprising at least one member of a first group, at least one member of a second group, and at least one member of a third group; wherein members of the first group are selected from the group consisting of beta adrenergic receptor antagonists (or beta blockers); members of the second group comprising steroids, e.g., an anabolic steroid, such as a steroid as described in Table 1, below, and members of the third group are selected from the group consisting of natural oils and fatty acids.

The invention provides preparations therapeutic combinations comprising at least one member of a first group, at least one member of a second group, at least one member of a third group, and at least one member of a fourth group; wherein members of the first group are selected from the group consisting of beta adrenergic receptor antagonists (or beta blockers); members of the second group are selected from the group consisting of angiotensin converting enzyme inhibitors (or ACE inhibitors); members of the third group are selected from the group consisting of angiotensin receptor blockers (or ARB's); and members of the fourth group are selected from the group consisting of non-steroidal anti-inflammatory drugs (NSAID's).

The invention provides preparations therapeutic combinations comprising at least one member of a first group, at least one member of a second group, at least one member of a third group, and at least one member of a fourth group; wherein members of the first group are selected from the group consisting of beta adrenergic receptor antagonists (or beta blockers); members of the second group are selected from the group consisting of angiotensin converting enzyme inhibitors (or ACE inhibitors); members of the third group are selected from the group consisting of angiotensin receptor blockers (or ARB's); and members of the fourth group comprising steroids, e.g., an anabolic steroid, such as a steroid as described in Table 1, below.

The invention provides preparations therapeutic combinations comprising at least one member of a first group, at least one member of a second group, at least one member of a third group, and at least one member of a fourth group; wherein members of the first group are selected from the group consisting of beta adrenergic receptor antagonists (or beta blockers); members of the second group are selected from the group consisting of angiotensin converting enzyme inhibitors (or ACE inhibitors); members of the third group are selected from the group consisting of non-steroidal anti-inflammatory drugs (NSAID's); and members of the fourth group comprising steroids, e.g., an anabolic steroid, such as a steroid as described in Table 1, below.

The invention provides preparations therapeutic combinations comprising at least one member of a first group, at least one member of a second group, at least one member of a third group, and at least one member of a fourth group; wherein members of the first group are selected from the group consisting of beta adrenergic receptor antagonists (or beta blockers); members of the second group are selected from the group consisting of angiotensin receptor blockers (or ARB's); members of the third group are selected from the group consisting of non-steroidal anti-inflammatory drugs (NSAID's); and members of the fourth group comprising steroids, e.g., an anabolic steroid, such as a steroid as described in Table 1, below.

The invention provides preparations therapeutic combinations comprising at least one member of a first group, at least one member of a second group, at least one member of a third group, and at least one member of a fourth group; wherein members of the first group are selected from the group consisting of beta adrenergic receptor antagonists (or beta blockers); members of the second group are selected from the group consisting of angiotensin converting enzyme inhibitors (or ACE inhibitors); members of the third group are selected from the group consisting of angiotensin receptor blockers (or ARB's); and members of the fourth group are selected from the group consisting natural oils and fatty acids.

The invention provides preparations therapeutic combinations comprising at least one member of a first group, at least one member of a second group, at least one member of a third group, and at least one member of a fourth group; wherein members of the first group are selected from the group consisting of beta adrenergic receptor antagonists (or beta blockers); members of the second group are selected from the group consisting of angiotensin converting enzyme inhibitors (or ACE inhibitors); members of the third group are selected from the group consisting of non-steroidal anti-inflammatory drugs (NSAID's); and members of the fourth group are selected from the group consisting natural oils and fatty acids.

The invention provides preparations therapeutic combinations comprising at least one member of a first group, at least one member of a second group, at least one member of a third group, and at least one member of a fourth group; wherein members of the first group are selected from the group consisting of beta adrenergic receptor antagonists (or beta blockers); members of the second group are selected from the group consisting of angiotensin receptor blockers (or ARB's); members of the third group are selected from the group consisting of non-steroidal anti-inflammatory drugs (NSAID's); and members of the fourth group are selected from the group consisting natural oils and fatty acids.

The invention provides preparations therapeutic combinations comprising at least one member of a first group, at least one member of a second group, at least one member of a third group, and at least one member of a fourth group; wherein members of the first group are selected from the group consisting of beta adrenergic receptor antagonists (or beta blockers); members of the second group are selected from the group consisting of angiotensin converting enzyme inhibitors (or ACE inhibitors); members of the third group comprising steroids, e.g., an anabolic steroid, such as a steroid as described in Table 1, below; and members of the fourth group are selected from the group consisting natural oils and fatty acids.

The invention provides preparations therapeutic combinations comprising at least one member of a first group, at least one member of a second group, at least one member of a third group, and at least one member of a fourth group; wherein members of the first group are selected from the group consisting of beta adrenergic receptor antagonists (or beta blockers); members of the second group are selected from the group consisting of angiotensin receptor blockers (or ARB's); members of the third group comprising steroids, e.g., an anabolic steroid, such as a steroid as described in Table 1, below; and members of the fourth group are selected from the group consisting natural oils and fatty acids.

The invention provides preparations therapeutic combinations comprising at least one member of a first group, at least one member of a second group, at least one member of a third group, and at least one member of a fourth group; wherein members of the first group are selected from the group consisting of beta adrenergic receptor antagonists (or beta blockers); members of the second group are selected from the group consisting of non-steroidal anti-inflammatory drugs (NSAID's); members of the third group comprising steroids, e.g., an anabolic steroid, such as a steroid as described in Table 1, below; and members of the fourth group are selected from the group consisting natural oils and fatty acids.

The invention provides preparations therapeutic combinations comprising at least one member of a first group, at least one member of a second group, at least one member of a third group, at least one member of a fourth group, and at least one member of a fifth group; wherein members of the first group are selected from the group consisting of beta adrenergic receptor antagonists (or beta blockers); members of the second group are selected from the group consisting of angiotensin converting enzyme inhibitors (or ACE inhibitors); and members of the third group are selected from the group consisting of angiotensin receptor blockers (or ARB's); members of the fourth group are selected from the group consisting of non-steroidal anti-inflammatory drugs (NSAID's); and members of the fifth group comprising steroids, e.g., an anabolic steroid, such as a steroid as described in Table 1, below.

The invention provides preparations therapeutic combinations comprising at least one member of a first group, at least one member of a second group, at least one member of a third group, at least one member of a fourth group, and at least one member of a fifth group; wherein members of the first group are selected from the group consisting of beta adrenergic receptor antagonists (or beta blockers); members of the second group are selected from the group consisting of angiotensin converting enzyme inhibitors (or ACE inhibitors); and members of the third group are selected from the group consisting of angiotensin receptor blockers (or ARB's); members of the fourth group are selected from the group consisting of non-steroidal anti-inflammatory drugs (NSAID's); and members of the fifth group are selected from the group consisting of natural oils and fatty acids.

The invention provides preparations therapeutic combinations comprising at least one member of a first group, at least one member of a second group, at least one member of a third group, at least one member of a fourth group, and at least one member of a fifth group; wherein members of the first group are selected from the group consisting of beta adrenergic receptor antagonists (or beta blockers); members of the second group are selected from the group consisting of angiotensin converting enzyme inhibitors (or ACE inhibitors); and members of the third group are selected from the group consisting of angiotensin receptor blockers (or ARB's); members of the fourth group comprising steroids, e.g., an anabolic steroid, such as a steroid as described in Table 1, below; and members of the fifth group are selected from the group consisting of natural oils and fatty acids.

The invention provides preparations therapeutic combinations comprising at least one member of a first group, at least one member of a second group, at least one member of a third group, at least one member of a fourth group, and at least one member of a fifth group; wherein members of the first group are selected from the group consisting of beta adrenergic receptor antagonists (or beta blockers); members of the second group are selected from the group consisting of angiotensin converting enzyme inhibitors (or ACE inhibitors); and members of the third group are selected from the group consisting of non-steroidal anti-inflammatory drugs (NSAID's); members of the fourth group comprising steroids, e.g., an anabolic steroid, such as a steroid as described in Table 1, below; and members of the fifth group are selected from the group consisting of natural oils and fatty acids.

The invention provides preparations therapeutic combinations comprising at least one member of a first group, at least one member of a second group, at least one member of a third group, at least one member of a fourth group, and at least one member of a fifth group; wherein members of the first group are selected from the group consisting of beta adrenergic receptor antagonists (or beta blockers); members of the second group are selected from the group consisting of angiotensin receptor blockers (or ARB's); and members of the third group are selected from the group consisting of non-steroidal anti-inflammatory drugs (NSAID's); members of the fourth group are selected from the group consisting of anabolic steroids; and members of the fifth group are selected from the group consisting of natural oils and fatty acids.

The invention provides preparations therapeutic combinations comprising at least one member of a first group, at least one member of a second group, at least one member of a third group, at least one member of a fourth group, at least one member of a fifth group, and at least one member of a sixth group; wherein members of the first group are selected from the group consisting of beta adrenergic receptor antagonists (or beta blockers); members of the second group are selected from the group consisting of angiotensin converting enzyme inhibitors (or ACE inhibitors); and members of the third group are selected from the group consisting of angiotensin receptor blockers (or ARB's); members of the fourth group are selected from the group consisting of non-steroidal anti-inflammatory drugs (NSAID's); members of the fifth group comprising steroids, e.g., an anabolic steroid, such as a steroid as described in Table 1, below; and members of the sixth group are selected from the group consisting of natural oils and fatty acids.

The invention provides methods wherein a formulation or preparation is administered by oral means, inhalation, infusion or injection, topical application or by absorption through epithelial or mucocutaneous linings.

The invention provides liquids comprising the preparation of the invention, or formulation of the invention. The invention provides capsules, sprays, powders, lotions, tablets or pills comprising a preparation of the invention or formulation of the invention.

The invention provides foods or food supplements comprising a preparation of the invention or a formulation of the invention. The food or food supplement can comprise a flavored bar, a power bar, a diet bar, an energy bar or a nutritional bar.

The invention provides methods for maintaining the health of a tissue comprising administering an effective amount of a preparation of the invention or a formulation of the invention. The tissue can be skeletal muscle tissue, fat tissue, or more than one body tissue type, including the majority of the body. The invention provides methods for ameliorating a disease or condition in an individual comprising administering an effective amount of a preparation of the invention or a formulation of the invention. The disease or condition can affect skeletal muscle tissue or fat tissue.

In separate embodiments, the product this invention provides different products that comprise (contain at least) all the combinations and permutations of any ingredients provided herein by specific mention. This invention also provides different products that comprise (contain at least) all the combinations and permutations of any ingredients provided herein, if not by specific mention of said ingredients, then by knowledge in the art that said ingredients are part of one or more category or group of ingredients provided herein.

In separate embodiments, this invention provides each of the products herein for use as therapies for improving states and disease symptoms such as involving inflammation, excessive sympathoneural drive, cachexia, anorexia, and anorexia-cachexia, and stress or anxiety related thereto. In separate embodiments, this invention provides the use of each the products herein as therapies for improving states and disease symptoms such as involving inflammation, excessive sympathoneural drive, cachexia, anorexia, and anorexia-cachexia, and stress or anxiety related thereto.

In separate embodiments, this invention provides different products that comprise (contain at least) all the combinations and permutations of ingredients selected from members of Group 1 (e.g. in Table 1), members of Group 2 (e.g. in Table 1), members of Group 3 (e.g. in Table 1), members of Group 4 (e.g. in Table 1), members of Group 5 (e.g. in Table 1), members of Group 6 (e.g. in Table 1), members of Group 7 (e.g. in Table 1), members of Group 8 (e.g. in Table 1), members of Group 9 (e.g. in Table 1), members of Group 10 (e.g. in Table 1), members of Group 6 (e.g. in Table 1), members of Group 11 (e.g. in Table 1), members of Group 12 (e.g. in Table 1), members of Group 13 (e.g. in Table 1), members of Group 14 (e.g. in Table 1), members of Group 15 (e.g. in Table 1), members of Group 16 (e.g. in Table 1), members of Group 17 (e.g. in Table 1), members of Group 18 (e.g. in Table 1), members of Group 19 (e.g. in Table 1), members of Group 20 (e.g. in Table 1), and members of Group 21 (e.g. in Table 1).

By way of illustration, in separate embodiments, this invention provides kits and packages containing therapeutic preparations, which therapeutic preparations that comprise (contain at least) all the combinations and permutations of from any one ingredient to any 100 ingredients selected from members of Group 1 (e.g. in Table 1), members of Group 2 (e.g. in Table 1), members of Group 3 (e.g. in Table 1), members of Group 4 (e.g. in Table 1), members of Group 5 (e.g. in Table 1), members of Group 6 (e.g. in Table 1), members of Group 7 (e.g. in Table 1), members of Group 8 (e.g. in Table 1), members of Group 9 (e.g. in Table 1), members of Group 10 (e.g. in Table 1), members of Group 6 (e.g. in Table 1), members of Group 11 (e.g. in Table 1), members of Group 12 (e.g. in Table 1), members of Group 13 (e.g. in Table 1), members of Group 14 (e.g. in Table 1), members of Group 15 (e.g. in Table 1), members of Group 16 (e.g. in Table 1), members of Group 17 (e.g. in Table 1), members of Group 18 (e.g. in Table 1), members of Group 19 (e.g. in Table 1), members of Group 20 (e.g. in Table 1), and members of Group 21 (e.g. in Table 1). The following embodiments illustrate these combinations and permutations.

In one embodiment, the invention provides novel preparations comprising the following components, for which non-limiting examples are listed in Table 1: a) one or more members selected from Group 1 (e.g. carvedolol or propanolol); and b) one or more members selected from Group 2 (e.g. captopril or enalapril).

In one embodiment, this invention provides novel preparations comprising the following components, for which non-limiting examples are listed in Table 1: a) one or more members selected from Group 1 (e.g. carvedolol or propanolol); and b) one or more members selected from Group 3 (e.g. losartan).

In one embodiment, this invention provides novel preparations comprising the following components, for which non-limiting examples are listed in Table 1: a) one or more members selected from Group 1 (e.g. carvedolol or propanolol); and b) one or more members selected from Group 4 (e.g. aspirin or ibuprofen or naproxen).

In one embodiment, this invention provides novel preparations comprising the following components, for which non-limiting examples are listed in Table 1: a) one or more members selected from Group 1 (e.g. carvedolol or propanolol); and b) one or more members selected from Group 5 (e.g. oxandrolone or testosterone).

In one embodiment, this invention provides novel preparations comprising the following components, for which non-limiting examples are listed in Table 1: a) one or more members selected from Group 1 (e.g. carvedolol or propanolol); and b) one or more members selected from Group 6 (e.g. EPA or DHA).

In one embodiment, this invention provides novel preparations comprising the following components, for which non-limiting examples are listed in Table 1: a) one or more members selected from Group 2 (e.g. captopril or enalapril); and b) one or more members selected from Group 3 (e.g. losartan).

In one embodiment, this invention provides novel preparations comprising the following components, for which non-limiting examples are listed in Table 1: a) one or more members selected from Group 2 (e.g. captopril or enalapril); and b) one or more members selected from Group 4 (e.g. aspirin or ibuprofen or naproxen).

In one embodiment, this invention provides novel preparations comprising the following components, for which non-limiting examples are listed in Table 1: a) one or more members selected from Group 2 (e.g. captopril or enalapril); and b) one or more members selected from Group 5 (e.g. oxandrolone or testosterone).

In one embodiment, this invention provides novel preparations comprising the following components, for which non-limiting examples are listed in Table 1: a) one or more members selected from Group 3 (e.g. losartan); and b) one or more members selected from Group 4 (e.g. aspirin or ibuprofen or naproxen).

In one embodiment, this invention provides novel preparations comprising the following components, for which non-limiting examples are listed in Table 1: a) one or more members selected from Group 3 (e.g. losartan); and b) one or more members selected from Group 5 (e.g. oxandrolone or testosterone).

In one embodiment, this invention provides novel preparations comprising the following components, for which non-limiting examples are listed in Table 1: a) one or more members selected from Group 2 (e.g. captopril or enalapril); and b) one or more members selected from Group 6 (e.g. EPA or DHA).

In one embodiment, this invention provides novel preparations comprising the following components, for which non-limiting examples are listed in Table 1: a) one or more members selected from Group 4 (e.g. aspirin or ibuprofen or naproxen); and b) one or more members selected from Group 5 (e.g. oxandrolone or testosterone).

In one embodiment, this invention provides novel preparations comprising the following components, for which non-limiting examples are listed in Table 1: a) one or more members selected from Group 3 (e.g. losartan); and b) one or more members selected from Group 6 (e.g. EPA or DHA).

In one embodiment, this invention provides novel preparations comprising the following components, for which non-limiting examples are listed in Table 1: a) one or more members selected from Group 1 (e.g. carvedolol or propanolol); b) one or more members selected from Group 2 (e.g. captopril or enalapril); and c) one or more members selected from Group 3 (e.g. losartan).

In one embodiment, this invention provides novel preparations comprising the following components, for which non-limiting examples are listed in Table 1: a) one or more members selected from Group 1 (e.g. carvedolol or propanolol); b) one or more members selected from Group 2 (e.g. captopril or enalapril); and c) one or more members selected from Group 4 (e.g. aspirin or ibuprofen or naproxen).

In one embodiment, this invention provides novel preparations comprising the following components, for which non-limiting examples are listed in Table 1: a) one or more members selected from Group 1 (e.g. carvedolol or propanolol); b) one or more members selected from Group 2 (e.g. captopril or enalapril); and c) one or more members selected from Group 5 (e.g. oxandrolone or testosterone).

In one embodiment, this invention provides novel preparations comprising the following components, for which non-limiting examples are listed in Table 1: a) one or more members selected from Group 4 (e.g. aspirin or ibuprofen or naproxen); and b) one or more members selected from Group 6 (e.g. EPA or DHA).

In one embodiment, this invention provides novel preparations comprising the following components, for which non-limiting examples are listed in Table 1: a) one or more members selected from Group 1 (e.g. carvedolol or propanolol); b) one or more members selected from Group 3 (e.g. losartan); and c) one or more members selected from Group 4 (e.g. aspirin or ibuprofen or naproxen).

In one embodiment, this invention provides novel preparations comprising the following components, for which non-limiting examples are listed in Table 1: a) one or more members selected from Group 1 (e.g., carvedolol or propanolol); b) one or more members selected from Group 3 (e.g. losartan); and c) one or more members selected from Group 5 (e.g. oxandrolone or testosterone).

In one embodiment, this invention provides novel preparations comprising the following components, for which non-limiting examples are listed in Table 1: a) one or more members selected from Group 1 (e.g., carvedolol or propanolol); b) one or more members selected from Group 2 (e.g., captopril or enalapril); and c) one or more members selected from Group 6 (e.g. EPA or DHA).

In one embodiment, this invention provides novel preparations comprising the following components, for which non-limiting examples are listed in Table 1: a) one or more members selected from Group 1 (e.g. carvedolol or propanolol); b) one or more members selected from Group 4 (e.g. aspirin or ibuprofen or naproxen); and c) one or more members selected from Group 5 (e.g. oxandrolone or testosterone).

In one embodiment, this invention provides novel preparations comprising the following components, for which non-limiting examples are listed in Table 1: a) one or more members selected from Group 1 (e.g. carvedolol or propanolol); b) one or more members selected from Group 3 (e.g. losartan); and c) one or more members selected from Group 6 (e.g. EPA or DHA).

In one embodiment, this invention provides novel preparations comprising the following components, for which non-limiting examples are listed in Table 1: a) one or more members selected from Group 2 (e.g. captopril or enalapril); b) one or more members selected from Group 3 (e.g. losartan); and c) one or more members selected from Group 4 (e.g. aspirin or ibuprofen or naproxen).

In one embodiment, this invention provides novel preparations comprising the following components, for which non-limiting examples are listed in Table 1: a) one or more members selected from Group 2 (e.g, captopril or enalapril); b) one or more members selected from Group 3 (e.g. losartan); and c) one or more members selected from Group 5 (e.g. oxandrolone or testosterone).

In one embodiment, this invention provides novel preparations comprising the following components, for which non-limiting examples are listed in Table 1: a) one or more members selected from Group 1 (e.g. carvedolol or propanolol); b) one or more members selected from Group 4 (e.g. aspirin or ibuprofen or naproxen); and c) one or more members selected from Group 6 (e.g. EPA or DHA).

In one embodiment, this invention provides novel preparations comprising the following components, for which non-limiting examples are listed in Table 1: a) one or more members selected from Group 2 (e.g. captopril or enalapril); b) one or more members selected from Group 4 (e.g. aspirin or ibuprofen or naproxen); and c) one or more members selected from Group 5 (e.g. oxandrolone or testosterone).

In one embodiment, this invention provides novel preparations comprising the following components, for which non-limiting examples are listed in Table 1: a) one or more members selected from Group 2 (e.g. captopril or enalapril); b) one or more members selected from Group 3 (e.g. losartan); and c) one or more members selected from Group 6 (e.g. EPA or DHA).

In one embodiment, this invention provides novel preparations comprising the following components, for which non-limiting examples are listed in Table 1: a) one or more members selected from Group 3 (e.g. losartan); b) one or more members selected from Group 4 (e.g. aspirin or ibuprofen or naproxen); and c) one or more members selected from Group 5 (e.g. oxandrolone or testosterone).

In one embodiment, this invention provides novel preparations comprising the following components, for which non-limiting examples are listed in Table 1: a) one or more members selected from Group 2 (e.g. captopril or enalapril); b) one or more members selected from Group 4 (e.g. aspirin or ibuprofen or naproxen); and c) one or more members selected from Group 6 (e.g. EPA or DHA).

In one embodiment, this invention provides novel preparations comprising the following components, for which non-limiting examples are listed in Table 1: a) one or more members selected from Group 1 (e.g. carvedolol or propanolol); b) one or more members selected from Group 2 (e.g. captopril or enalapril); c) one or more members selected from Group 3 (e.g. losartan); and d) one or more members selected from Group 4 (e.g. aspirin or ibuprofen or naproxen).

In one embodiment, this invention provides novel preparations comprising the following components, for which non-limiting examples are listed in Table 1: a) one or more members selected from Group 1 (e.g. carvedolol or propanolol); b) one or more members selected from Group 2 (e.g. captopril or enalapril); c) one or more members selected from Group 3 (e.g. losartan); and d) one or more members selected from Group 5 (e.g. oxandrolone or testosterone).

In one embodiment, this invention provides novel preparations comprising the following components, for which non-limiting examples are listed in Table 1: a) one or more members selected from Group 3 (e.g. losartan); b) one or more members selected from Group 4 (e.g. aspirin or ibuprofen or naproxen); and c) one or more members selected from Group 6 (e.g. EPA or DHA).

In one embodiment, this invention provides novel preparations comprising the following components, for which non-limiting examples are listed in Table 1: a) one or more members selected from Group 1 (e.g. carvedolol or propanolol); b) one or more members selected from Group 2 (e.g. captopril or enalapril); c) one or more members selected from Group 4 (e.g. aspirin or ibuprofen or naproxen); and d) one or more members selected from Group 5 (e.g. oxandrolone or testosterone).

In one embodiment, this invention provides novel preparations comprising the following components, for which non-limiting examples are listed in Table 1: a) one or more members selected from Group 1 (e.g. carvedolol or propanolol); b) one or more members selected from Group 2 (e.g. captopril or enalapril); c) one or more members selected from Group 3 (e.g. losartan); and d) one or more members selected from Group 6 (e.g. EPA or DHA).

In one embodiment, this invention provides novel preparations comprising the following components, for which non-limiting examples are listed in Table 1: a) one or more members selected from Group 1 (e.g. carvedolol or propanolol); b) one or more members selected from Group 3 (e.g. losartan); c) one or more members selected from Group 4 (e.g. aspirin or ibuprofen or naproxen); and d) one or more members selected from Group 5 (e.g. oxandrolone or testosterone).

In one embodiment, this invention provides novel preparations comprising the following components, for which non-limiting examples are listed in Table 1: a) one or more members selected from Group 1 (e.g. carvedolol or propanolol); b) one or more members selected from Group 2 (e.g. captopril or enalapril); c) one or more members selected from Group 4 (e.g. aspirin or ibuprofen or naproxen); and d) one or more members selected from Group 6 (e.g. EPA or DHA).

In one embodiment, this invention provides novel preparations comprising the following components, for which non-limiting examples are listed in Table 1: a) one or more members selected from Group 1 (e.g. carvedolol or propanolol); b) one or more members selected from Group 2 (e.g. captopril or enalapril); c) one or more members selected from Group 3 (e.g. losartan); d) one or more members selected from Group 4 (e.g. aspirin or ibuprofen or naproxen); and e) one or more members selected from Group 5 (e.g. oxandrolone or testosterone).

In one embodiment, this invention provides novel preparations comprising the following components, for which non-limiting examples are listed in Table 1: a) one or more members selected from Group 1 (e.g. carvedolol or propanolol); b) one or more members selected from Group 3 (e.g. losartan); c) one or more members selected from Group 4 (e.g. aspirin or ibuprofen or naproxen); and d) one or more members selected from Group 6 (e.g. EPA or DHA).

In one embodiment, this invention provides novel preparations comprising the following components, for which non-limiting examples are listed in Table 1: a) one or more members selected from Group 1 (e.g. carvedolol or propanolol); b) one or more members selected from Group 3 (e.g. losartan); c) one or more members selected from Group 4 (e.g. aspirin or ibuprofen or naproxen); and d) one or more members selected from Group 6 (e.g. EPA or DHA).

In one embodiment, this invention provides novel preparations comprising the following components, for which non-limiting examples are listed in Table 1: a) one or more members selected from Group 1 (e.g. carvedolol or propanolol); b) one or more members selected from Group 2 (e.g. captopril or enalapril); c) one or more members selected from Group 3 (e.g. losartan); d) one or more members selected from Group 4 (e.g. aspirin or ibuprofen or naproxen); and e) one or more members selected from Group 6 (e.g. EPA or DHA).

In one embodiment, this invention provides novel preparations comprising the following components, for which non-limiting examples are listed in Table 1: a) one or more members selected from Group 1 (e.g. carvedolol or propanolol); b) one or more members selected from Group 2 (e.g. captopril or enalapril); c) one or more members selected from Group 3 (e.g. losartan); d) one or more members selected from Group 4 (e.g. aspirin or ibuprofen or naproxen); e) one or more members selected from Group 5 (e.g. oxandrolone or testosterone); and f) one or more members selected from Group 6 (e.g. EPA or DHA).

In one aspect, the term "at least one member" in reference to exemplary alternative embodiments comprises minimally every integer value from one to about 20 or more, inclusive; i.e. in one aspect it means at least one member, in another aspect it means at least two members, in another aspect it means at least three members, ..., etc, and in another aspect it means at least 20 members. Alternative embodiments can comprise more than 20 members.

In one embodiment, this invention provides every combination and permutation of ingredients exemplified in Table 1 (i.e. Groups 1-20). Examples of such combinations and permutations provided herein include the possibilities where ingredients are selected from at least 1 group, where the at least 1 group is selected from any of Groups 1-20. In alternative embodiments there may be at least one, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, and at least 20 different members selected from any to each of Groups 1-20.

Examples of such combinations and permutations provided herein include the possibilities where ingredients are selected from at least 2 groups, where the at least 2 groups are selected from any of Groups 1-20. In alternative embodiments there may be at least one, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, and at least 20 different members selected from any to each of Groups 1-20.

Examples of such combinations and permutations provided herein include the possibilities where ingredients are selected from at least 3 groups, where the at least 3 groups are selected from any of Groups 1-20. In alternative embodiments there may be at least one, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, and at least 20 different members selected from any to each of Groups 1-20.

Examples of such combinations and permutations provided herein include the possibilities where ingredients are selected from at least 4 groups, where the at least 4 groups are selected from any of Groups 1-20. In alternative embodiments there may be at least one, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, and at least 20 different members selected from any to each of Groups 1-20.

Examples of such combinations and permutations provided herein include the possibilities where ingredients are selected from at least 5 groups, where the at least 5 groups are selected from any of Groups 1-20. In alternative embodiments there may be at least one, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, and at least 20 different members selected from any to each of Groups 1-20.

Examples of such combinations and permutations provided herein include the possibilities where ingredients are selected from at least 6 groups, where the at least 6 groups are selected from any of Groups 1-20. In alternative embodiments there may be at least one, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, and at least 20 different members selected from any to each of Groups 1-20.

Examples of such combinations and permutations provided herein include the possibilities where ingredients are selected from at least 7 groups, where the at least 7 groups are selected from any of Groups 1-20. In alternative embodiments there may be at least one, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, and at least 20 different members selected from any to each of Groups 1-20.

Examples of such combinations and permutations provided herein include the possibilities where ingredients are selected from at least 8 groups, where the at least 8 groups are selected from any of Groups 1-20. In alternative embodiments there may be at least one, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, and at least 20 different members selected from any to each of Groups 1-20.

Examples of such combinations and permutations provided herein include the possibilities where ingredients are selected from at least 9 groups, where the at least 9 groups are selected from any of Groups 1-20. In alternative embodiments there may be at least one, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, and at least 20 different members selected from any to each of Groups 1-20.

Examples of such combinations and permutations provided herein include the possibilities where ingredients are selected from at least 10 groups, where the at least 10 groups are selected from any of Groups 1-20. In alternative embodiments there may be at least one, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, and at least 20 different members selected from any to each of Groups 1-20.

Examples of such combinations and permutations provided herein include the possibilities where ingredients are selected from at least 11 groups, where the at least 11 groups are selected from any of Groups 1-20. In alternative embodiments there may be at least one, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, and at least 20 different members selected from any to each of Groups 1-20.

Examples of such combinations and permutations provided herein include all possibilities where ingredients are selected from at least 12 groups, where the at least 12 groups are selected from any of Groups 1-20. In alternative embodiments there may be at least one, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, and at least 20 different members selected from any to each of Groups 1-20.

Examples of such combinations and permutations provided herein include all possibilities where ingredients are selected from at least 13 groups, where the at least 13 groups are selected from any of Groups 1-20. In alternative embodiments there may be at least one, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, and at least 20 different members selected from any to each of Groups 1-20.

Examples of such combinations and permutations provided herein include all possibilities where ingredients are selected from at least 14 groups, where the at least 14 groups are selected from any of Groups 1-20. In alternative embodiments there may be at least one, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, and at least 20 different members selected from any to each of Groups 1-20.

Examples of such combinations and permutations provided herein include all possibilities where ingredients are selected from at least 15 groups, where the at least 15 groups are selected from any of Groups 1-20. In alternative embodiments there may be at least one, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, and at least 20 different members selected from any to each of Groups 1-20.

Examples of such combinations and permutations provided herein include all possibilities where ingredients are selected from at least 16 groups, where the at least 16 groups are selected from any of Groups 1-20. In alternative embodiments there may be at least one, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, and at least 20 different members selected from any to each of Groups 1-20.

Examples of such combinations and permutations provided herein include all possibilities where ingredients are selected from at least 17 groups, where the at least 17 groups are selected from any of Groups 1-20. In alternative embodiments there may be at least one, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, and at least 20 different members selected from any to each of Groups 1-20.

Examples of such combinations and permutations provided herein include all possibilities where ingredients are selected from at least 18 groups, where the at least 18 groups are selected from any of Groups 1-20. In alternative embodiments there may be at least one, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, and at least 20 different members selected from any to each of Groups 1-20.

Examples of such combinations and permutations provided herein include all possibilities where ingredients are selected from at least 19 groups, where the at least 19 groups are selected from any of Groups 1-20. In alternative embodiments there may be at least one, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, and at least 20 different members selected from any to each of Groups 1-20.

Examples of such combinations and permutations provided herein include all possibilities where ingredients are selected from at least 20 groups, where the at least 20 groups are selected from Groups 1-20. In alternative embodiments there may be at least one, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, and at least 20 different members selected from any to each of Groups 1-20.

The invention provides product of manufactures comprising a blister package, a clamshell, a tray or a shrink wrap comprising a therapeutic combination of the invention, or a pharmaceutical composition of the invention, e.g., a pills, tabs, geltabs, capsules and the like. In one aspect the product of manufacture comprises at least one beta adrenergic receptor antagonist (a beta blocker) and an angiotensin-converting enzyme (ACE) inhibitor, an angiotensin receptor blocker (ARB), a non-steroidal anti-inflammatory drug (a NSAID), an anabolic steroid, a natural oil, a fatty acid or a combination thereof. In one aspect the product of manufacture comprises at least one beta adrenergic receptor antagonist (a beta blocker) and at least one non-steroidal anti-inflammatory drug (a NSAID). The at least one non-steroidal anti-inflammatory drug (a NSAID) can comprise an aspirin, dichlofenac, diflunisal, etodolac, fenoprofen, flurbiprofen, ibuprofen, indomethacin, ketoprofen; ketorolac, meclofenamate, mefenamic acid, meloxicam, nabumetone, naproxen, oxaprozin, piroxicam, salsalate, sulindac, tolmetin, a COX-2 inhibitor (e.g., a COX-2-selective inhibitor) or a combination thereof. In one aspect, the COX-2 inhibitor (e.g., a COX-2-selective inhibitor) comprises celecoxib rofecoxib, etoricoxib, valdecoxib, parecoxib, meloxicam and/or lumiracoxib. The invention also provides products of manufacture comprising a blister package, a clamshell, a tray or a shrink wrap comprising a therapeutic combination comprising an atenolol, nadolol, metoprolol, propranolol, carteolol, carvedolol, labetalol, oxprenolol, penbutolol, pindolol, sotalol, timolol or a combination thereof, and at least one non-steroidal anti-inflammatory drug (a NSAID).

The invention provides therapeutic combinations of the invention for use as a medicament, e.g., for use in the treatment of cachexia. In one aspect, the cachexia is defmed as at least two of the symptoms selected from the group consisting of: 1) a hyper-inflammatory state, 2) altered hormone levels and/or cytokine levels (over normal); 3) sustained heart rate or rhythm variability (over normal); 4) weight loss, and 5) sustained increased heart rate (over normal), wherein optionally the sustained increased heart rate is having a sustained elevated heart rate of at least about 6 bpm over normal (over what is considered normal for a particular healthy individual). In one aspect, the cachexia is defined by an individual having at least a sustained elevated heart rate of at least about 6 bpm (over what is considered normal for a particular healthy individual) and weight loss.

In one aspect, sustained heart rate or rhythm variability over normal is measured over a period of at least 10, 20, 30, 40, 50, 60 or more minutes, or, alternatively, over a 24 hour period, or alternatively over a period of 200, 300, 400 or 500 or more beats, or a combination thereof; or alternatively, where the heart rate (or rhythm) variability is statistically significant with a p value of at least 0.01, 0.02, 0.03, 0.04, 0.05, 0.06, 0.07, 0.08, 0.09 or 1.0, or more, or a p value of less than 0.1. In one aspect, the "normalcy" or "abnormalcy" of heart rate or rhythm - the variability of heart rate or rhythm - is determined by comparing a patient suspected of having SIRS, e.g., cachexia, with what would be considered normal values for a similar individual (e.g., an individual of similar age, health, weight, sex, race and the like) (in other words, it is not necessary that the individual's heart rate be evaluated in a healthy state). In one aspect, practicing the invention (e.g., administering the therapeutic combinations of the invention, or practicing the methods of the invention) result in a less normal to more normal heart rate or rhythm, less variability of heart rate or rhythm, hormone level and/or cytokine level and the like.

In another aspect, the variability of heart rate or rhythm is measured using R to R variability, QRS patterns, or a combination thereof, or any method known in the art to determine heart rate or rhythm variability; see, e.g., U.S. Patent Nos. 7,009,492; 6,678,547; 6,574,491; 6,549,804; 6,223,073; 5,241,967. Normal versus abnormal heart rate or rhythm are determined by comparing values from similar individuals, e.g., individuals of the same age, weight, sex, race and the like.

The invention provides uses of the therapeutic combinations of the invention in the manufacture of a medicament or pharmaceutical composition for treating, ameliorating or preventing a trauma, condition or disease comprising: a chronic Systemic Inflammatory Response State (SIRS); chronic systemic inflammatory stress; burns, chronic obstructive pulmonary disease; congestive heart failure; chronic kidney disease; surgery; cancer; sepsis; ageing; acute respiratory distress syndrome; acute lung injury; infection; a CNS disorder or injury; anemia; immunosuppression; insulin resistance; anorexia; anxiety; sleep disturbances; weakness; fatigue; gastrointestinal distress; sleep disturbances; wake disturbances; pain; listlessness; shortness of breath; lethargy; depression; malaise; or, a combination thereof. In one aspect, the trauma, condition or disease comprises a maladaptive nutritional state secondary to the SIRS. In one aspect, the maladaptive nutritional state comprises cachexia, and optionally the cachexia comprises cachexia secondary to cancer. In one aspect, the cachexia is defined as at least two of the symptoms selected from the group consisting of: 1) a hyper-inflammatory state, 2) altered hormone levels and/or cytokine levels; 3) sustained heart rate variability (more variability than normal); 4) weight loss, and 5) sustained increased heart rate, wherein optionally the sustained increased heart rate is having a sustained elevated heart rate of at least about 6 bpm (over what would be considered normal for a particular individual). In one aspect, the cachexia is defined by an individual having at least a sustained elevated heart rate of at least about 6 bpm (over what would be considered normal for a particular individual) and weight loss.

The invention provides kits comprising a therapeutic combination of the invention, or a pharmaceutical composition of the invention. In one aspect, the kit comprises at least one blister pack comprising a therapeutic combination of the invention, or a pharmaceutical composition of the invention.

The invention provides kits for the treatment, amelioration or prevention of a chronic Systemic Inflammatory Response State (SIRS) or a maladaptive nutritional state in a patient population, the kit comprising a therapeutic combination of the invention, or a pharmaceutical composition of the invention, and instructions for use of the therapeutic combination or pharmaceutical composition. In one aspect, the maladaptive nutritional state comprises cachexia, and optionally the cachexia comprises cachexia secondary to cancer, and in one aspect, the instructions for use indicate that the patient population for practicing the methods and compositions of the invention are individuals having a chronic Systemic Inflammatory Response State (SIRS) or a maladaptive nutritional state, wherein optionally the cachexia is defined as at least two of the symptoms selected from the group consisting of: 1) a hyper-inflammatory state, 2) altered hormone levels and/or cytokine levels; 3) sustained increased heart rate variability (more variability than normal); 4) weight loss, and 5) sustained increased heart rate, wherein optionally the increased heart rate is having a sustained elevated heart rate of at least about 6 bpm over what would be considered normal for that particular individual. In one aspect, the cachexia is defined by an individual having at least a sustained elevated heart rate of at least about 6 bpm (over what would be considered normal for that particular individual) and weight loss.

The invention provides kits for the treatment, amelioration or prevention of a CNS disorder, wherein the kit comprises a therapeutic combination of the invention, or a pharmaceutical composition of the invention. In one aspect, the CNS disorder comprises Parkinson's disease or Alzheimer's disease.

The invention provides computer implemented methods for diagnosing cachexia, wherein the computer implemented method comprises analysis of data representing measurements of at least two of symptoms selected from the group consisting of: 1) a hyper-inflammatory state, 2) hormone levels and/or cytokine levels; 3) heart rate variability over normal; 4) weight loss, and 5) sustained increased heart rate over normal are analyzed and the presence of at least two of the symptoms indicates a readout of a diagnosis of cachexia. In one aspect, the cachexia diagnosis comprises diagnosis of early onset cachexia or early stage of cachexia.

The invention also provides computer program products for implementing the computer implemented methods of the invention, and computers comprising the computer program products of the invention.

The details of one or more aspects of the invention are set forth in the description below. Other features, objects, and advantages of the invention will be apparent from the description and from the claims.

All publications, patents and patent applications cited herein are hereby expressly incorporated by reference for all purposes.

### DETAILED DESCRIPTION

The invention provides compositions, e.g., pharmaceutical compounds, preparations, formulations, kits and products of manufacture (e.g., blister packages, shrink wraps, etc.), comprising combinations of beneficial ingredients that are serviceable as therapies for improving, treating, ameliorating and/or preventing conditions, states and/or disease symptoms involving inflammation, excessive sympathoneural drive, cachexia, anorexia, and anorexia-cachexia, and stress or anxiety related thereto, and method of using them. Relevant symptoms improved, treated, ameliorated and/or prevented by the compositions and methods of the invention are exemplified by, e.g., wasting and/or atrophy, such as muscle atrophy, or anorexia-cachexia, e.g., as cachexia related to chronic and/or wasting diseases, such as cancer.

Table 1 provides serviceable components, i.e., exemplary combinations of drugs and/or pharmaceuticals of the invention. These components (e.g., exemplary combinations of drugs) are presented as members of groups with non-limiting examples provided that can be used in the compositions (e.g., pharmaceutical compounds, preparations, formulations, kits and products of manufacture) and methods of the invention.

For example, in one aspect, the invention provides compositions and therapies comprising use of a beta adrenergic antagonist (also called "beta blockers") in combination with an anti-inflammatory agent, e.g., a nonsteroidal anti-inflammatory drug (NSAID), an angiotensin-converting enzyme (ACE) inhibitor, an angiotensin receptor blocker (ARB), an anabolic steroid, a natural oil or fatty acid or any combination thereof.

**Table 1: Exemplary ingredients used in the methods and compositions of the invention.**

| Group | Table 1. Group Members (The examples are listed for each group are non-limiting examples. The sub-groups are not necessarily mutually exclusive.) |
|---|---|
| 1 | Beta adrenergic antagonists (Beta Blockers or BBs) Beta blockers in general, including a) Beta blockers with binding at both beta1 and beta 2 receptors, e.g. Carteolol, Carvedilol Labetalol, Nadolol, Oxprenolol, Penbutolol, Pindolol, Propranolol, Sotalol, Timolol; b) Beta blockers that have selectivity for beta1 receptors (over beta2 receptors), e.g. Acebutolol, Atenolol, Betaxolol, Bisoprolol, Esmolol, Metoprolol (including Metoprolol tartrate and Metoprolol succinate), Nebivolol; and c) Beta blockers that have selectivity for beta 3 receptors (over beta1 receptors or over beta2 receptors). |
| 2 | Angiontensin Converting Enzyme Inhibitors (ACE Inhibitors or ACE-Is) Benazepril; Captopril; Cilazapril; Enalapril; Enalaprilat; Fosinopril Sodium; Imidapril, Lisinopril; Moexipril; Perindopril; Quinapril; Ramipril; Trandolapril. |
| 3 | Angiotensin Receptor Blockers (ARBs) Candesartan; Eprosartan; Irbesartan; Losartan; Olmesartan; Telmisartan; Valsartan. |
| 4 | Non-Steroidal Anti-Inflammatory Drugs (NSAIDs) Traditional NSAIDs, e.g. Aspirin, Diclofenac; Diflunisal; Etodolac; Fenoprofen; Flurbiprofen; Ibuprofen; Indomethacin; Ketoprofen; Ketorolac Tromethamine; Meclofenamate; Mefenamic Acid; Meloxicam; Nabumetone; Naproxen; Oxaprozin; Piroxicam; Salsalate, Sulindac; Tolmetin. COX2-selective NSAIDs, e.g. Celecoxib; Rofecoxib; Etoricoxib; Valdecoxib and Parecoxib; Meloxicam; Lumiracoxib. |
| 5 | Steroids Examples of steroids that can be administered orally: Andarine, Ethylestrenol; Mesterolone; Methandrostenolone; Methenolone; Methyltestosterone; Oxandrolone; Oxymetholone; Stanozolol. Examples of steroids that can be administered by intramuscular injection: Boldenone (veterinary); Hexoxymestrolum; Methandrostenolone; Methenolone enanthate; Nandrolone decanoate; Nandrolone phenproprionate; Stanozolol (veterinary); Stenbolone; Testosterone cypionate; Testosterone enanthate; Testosteron (esters); Testosterone nicotinate; Therobolin; Trenbolone (Finajet); Trenbolone hexahydrobenzylcarbonate; Trophobolene. |
| 6 | Fatty acids Natural fatty acids such as omega-3 fatty acids, fish oils, long-chain polyunsaturated fatty acids (these may be found in fish and fish oils), n-3 and n-6 highly unsaturated fatty acids, including specific examples such as eicosapentaenoic acid (EPA), and docosahexaenoic acid (DHA). In non-limiting examples, these fatty acids may be administered orally or intravenously. |
| 7 | TNF inhibitors Anti-TNF agents, anti-TNF mAbs, pentoxifylline, thalidomide. |
| 8 | Immunomodulators (IMMs) Rapamycin, lenalidomide. |
| 9 | Antidepressant Agents and Mood Altering Drugs (MADs) include: alternative and herbal antidepressants (AHAs) monoamine oxidase inhibitors (MAOIs) "novel" and "other" antidepressants (NOAs) noradrenaline reuptake inhibitors (NARIs). reversible monoamine oxidase inhibitors (RMAOIs) serotonin and noradrenaline reuptake inhibitors (SNRIs). serotonin reuptake inhibitors (SRIs) and/or selective serotonin reuptake inhibitors (SSRIs), tricyclic antidepressants (TCAs), tetracyclic antidepressants (TTCAs) AHAs include St John's Wort (Hypericum), Valerian root (Valerian officinalis), Damiana (Tumera diffusa), Ginkgo (Ginkgo biloba) Ginseng (Panax ginseng). MAOIs include:phenelzine, tranylcypromine NOAs or include alprazolam, amoxapine, bupropion, buspirone, carbamazepine, cyclobenzaprine, dexanabinol, lithium, maprotiline, mirtazapine, oxcarbazepine, reserpine, rolipram, serzone, trazodone, and venlafaxine. NARIs include reboxetine RMAOIs include moclobemide SNRIs include duloxetine, milnacipran, nefazodone, venlafaxine. SRIs &/or SSRIs include citalopram, escitalopram, fluoxetine, fluvoxamine, paroxetine and sertraline. TCAs include amitriptyline, amoxapine, clomipramine, desipramine, doxepin, imipramine, nortryptyline, protriptyline, trimipramine TTCAs include maprotiline, mianserin, and mirtazapine |
| 10 | Cannabinoid receptor ligands (CARLs), including plant-derived cannabinoids, endogenous cannabinoids (endocannabinoids), synthetic cannabinoids, compounds that have cannabinoid receptor agonist activity (agonist activity includes direct, modulating, and potentiating), compounds that have cannabinoid receptor antagonist activity, and compounds that have both cannabinoid receptor agonist activity and cannabinoid receptor antagonist activity, compounds that have CB 1 receptor-selective activity, compounds that have CB2 receptor-selective activity, and compounds that have selectivity for cannabinoid receptors that are not CB1 or CB2. Anandamide, 2-AG, delta (9)-tetrahydrocannabinol (delta 9-THC), cannabidiol (CBD), cannabinol (CBN), Cannabigerol (CBG), Cannabichromene (CBC), Cannabicyclol (CBL), Cannabivarol (CBV), Tetrahydrocannabivarin (THCV), Cannabidivarin (CBDV), Cannabichromevatin (CBCV), Cannabigerovarin (CBGV), Cannabigerol Monoethyl Ether (CBGM), Dronabinol, WIN55,212-2, CP-47,497, CP55,940, HU210, SR-144526, Nabilone, 1-pentyl-3-(2-methylphenylacetyl)indole (JWH-251) and 1-pentyl-3-(3-methoxyphenylacetyl)indole (JWH-302), JWH133, L768242, Dexanabinol, LY320135, Marinol, Rimonabant (or SR141716A), URB597, UCM707, and molecules with potentiating activity such as Org 27569, Org 27759 and Org 29647. Analogs of the aforementioned; e.g. 7-hydroxy-delta-6-tetrahydrocannabinol and HU210 are analogs; e.g. docosatetraenylethanolamide and homo-γ-linoenylethanolamide are analogs of anandamide; e.g. there are manay analogs of CP-47,497, e.g. there are many analogs of CP55,940; e.g. there are a number of delta 8-THC derivatives, such as 1-methoxy-delta(8)-THC derivatives, 1-methoxy-delta(8)-THC-DMH (L759633), 1-methoxy-delta(9(11))-THC-DMH (L759656), 1-methoxy-3-(1',1'-dimethylhexyl)-Delta(8)-THC (JWH-229), plus a number of 1-deoxy-delta(8)-THC analogues, such as 1-deoxy-3-(1',1'-dimethylbutyl)-Delta(8)-THC (JWH-133); and e.g. Arvanil is a cannabinoid analog that is a hybrid endocannabinoid and vanilloid compound. |
| 11 | Anti-Oxidants (AOs) N¹-acetyl-N²-formyl-5-methoxykynuramine (AFMK), alkylglycerols (or AKGs, e.g. from shark liver oil), alpha lipoic acid, p-amino-benzoic acid (PABA), anthocyanidins (e.g. apigeninidin, aurentinidin, capensinidin, L-carnitine (including acetyl-L-cartinite), columnidin, cyanidin, delphinidin, diosmetinidin, europinidin, guibourtinidin, fisetinidin, hirsutinidin, luteolinidin, malvidin, pelargonidin, peonidin, 5-desoxy-malvidin, 5-desoxy-peonidin, petunidin, proanthocyanidins, robinetinidin, rosinidin, tricetinidin), anthocyadins, anthocyanins, astrone, bioflavonoids (e.g. catechin, citrin, eriodictin, ginko biloba, hesperetin, hesperidin, nobiletin, quercetin, rutin, sinensetin, and tangeretin), carnosine, carotenes, carotenoids, carvedilol (and other carbazole-containing antioxidant compounds), catechins, centrophenoxine (Lucidril™, meclofenoxate), chlorogenic acid, coenzyme Q10 (CoQ10 or ubiquinone), p-coumaric acid (CA), CPI-1189, curcumin, cysteine, dehydroepiandrosterone (DHEA, and analogs such as e.g., 16-alpha-fluoro-5-androsten-17-one DHEA, 16 alpha-fluoro-5 alpha-androstan-17-one DHEA, DHEA sulfate, 7alpha-OH-DHEA, 7beta-OH-DHEA, and 7-keto DHEA), deprenyl (selegiline), dimethylaminoethanol (DMAE), dimethylglycine (DMG), dioscorea, L-DOPA, ellagic acid, ellagitannins, epicatechin (EC), epigallocatechin (EGC), epicatechin gallate (ECG) and epigallocatechin gallate (EGCG), famotidine, ferulic acid, flavonoids, falvonols, flavonolgnans (e.g. silibinin, silychristin, and silydianin, sometimes collectively called silymarin), Gallic acid (3,4,5-trihydroxy benzoic acid), gamma-oryzanol, glutathione (GSH), hydergine (codergocrine mesylate), idebenone, lutein, lycopenes, MDL 29311, melatonin, N₂-Mercaptopropionylglycine(N₂-MPG), N-acetyl-L-cysteine (NAC), NADH (Q-1), N-acetylserotonin (NAS), nifedipine, nimodipine, oligomeric proanthocyanidins (OPCs), omeprazole, OPC-14117, organogermanium compounds, pangamic acid (sometimes called vitamin B 15), pentoxifylline, phenolic compounds, phloretin, phloridzin, phytosterols, piracetam, plants and plant extracts (e.g. preparations of and extracts derived from acerola, ashwagandha, bacopa, berries, bilberries, blackberries, blueberries, citrus, cranberries, garlic, grapes, grape seeds, grape skins, green teas, kale, mangosteen, milk thistle, origanum vulgare, pine barks, pomegranate, prunes, raspberries, red wine, strawberries, teas, tumeric, wolfberry, xango), polyphenols, proanthocyanidins, proanthocyanidoles, probucol, pycogenols, pyritinol (also known as pyrithioxine and pyridoxine disulfide), quercetin (sometimes may also be called xanthaurine; meletin; quercetol; quertine; or sophoretin), resveratrol, rutin, selenium, silica hydride, squalene, tannins (e.g. profisetinidin type), taurine, tocopherol, uric acid, vincamine, vinpocetine (e.g. periwinkle extract), vitamins with antioxidant properties, vitamin A (e.g. retinal, retinol, retinoic acid), vitamin B (e.g.), vitamin C (including provitamin C or ascorbign, vitamin C1 or ascorbic acid, vitamin C2 or dehydroascorbic acid,vitamin C3 or ascorbyl palmitate, vitamin C4 or calcium ascorbate, vitamin C5 or isoascorbic acid, vitamin C6 or calcium isoascorbate), vitamin E (e.g. tocotrienols and tocopherols, including alpha-, gamma-, and delta-versions thereof), vitamin P (citrin), xanthones, zeaxanthin. |
| 12 | Reducing agents Niacin, tryptophan. |
| 13 | Amino Acids The eight "essential" amino acids: isoleucine, leucine, lysine, methionine, phenylalanine, threonine,tryptophan and valine. The "non-essential" amino acids: alanine, arginine, asparagin, aspartic acid, carnitine, citrulline, cysteine, cystine, gamma-amino butyric acid (gaba), glutamic acid, glutamine, glycine, ornithine, proline, serine, taurine, and tyrosine. All amino acids based on the compositions of proteins (e.g. fibrinogen) in acute phase protein responses (APPRs), e.g., alanine, arginine, cysteine, glutamine, glycine, phenylalanine, proline, serine, and tryptophan. |
| 14 | Statins Atorvastatin, fluvastatin, lovastatin, pravastatin, rosuvastatin, simvastatin. |
| 15 | Calcium Channel Blockers Amlodipine besylate, diltiazem, gallopamil, lercanidipine, menthol (mint oil), nicardipine, nifedipine, nimodipine, nitrendipine, nisoldipine, verapamil. |
| 16 | Bisphosphonates Alendronate, etidronate, disodium pamidronate, ibandronate, risedronate, sodium clodronate, and zoledronate. |
| 17 | Tissue Factors and Hormones IGF-1, hGH, GHRF and analogs |
| 18 | Tissue Factor Inhibitors Anti-tissue factor mAb's, TFPI. |
| 19 | Melanocortin Blockers Melanocortin mAb's, Melanocortin receptor (MC4) antagonists. |
| 20 | Vitamins Water soluble vitamins, such as vitamin C and the B vitamins (e.g. folic acid and folinic acid): Vitamin C including ascorbic acid and ascorbates, such as calcium ascorbate, chromium ascorbate, magnesium ascorbate, manganese ascorbate, molybdenum ascorbate, sodium ascorbate, zinc ascorbate, 1-ascorbic acid, 1-(+)-ascorbic acid, 1,3-ketothreohexuronic acid; also including metabolites such as dehydroascorbate (oxidized ascorbic acid), calcium threonate, xylonate and lyxonate; also including precursors and derivatives such as ascorbyl pahnitate (a vitamin C ester, likely to hydrolyzed to ascorbic acid and palmitic acid). B-Vitamins including B1 (thiamine), B2 (riboflavin), B3 (niacin, niacinamide, inositol hexaniacinate, nicotinamide, nicotinic acid), B5 (pantothenates, such as pantothenic acid, calcium pantothenate, pantethine), B6 (pyridoxine, PLP, Pyridoxal-5'-Phosphate), B7 (biotin), B9 (folinic acid, folic acid, folates such as methylfolate, or 5-formyl tetrahydrofolate), and B12 (cobalamin and derivatives such as, hydroxycobalamin, methylcobalamin, adenosylcobalamin, cyanocobalamin, hydroxycyanocobalamin). Products containing variations of these vitamins are also included herein (for patent protection) and include, e.g. folinic acid (e.g. as calcium folinate). Fat soluble vitamins such as vitamins A, D, E, and K: Vitamin A (including retinol and retinol derivatives such as retinoic acid) and carotenoids (which includes over 600 members such as lycopene and lutein). Vitamin D including 1,25-dihydroxyvitamin D, calciferol, calcipotriol, cholecalciferol, ergocalciferol (vitamin D2), irradiated ergocalciferol. Vitamin E including alpha tocopherol, tocopherol, tocopheryl acetate, tocopheryl succinate. Vitamin K including forms such as phylloquinones, menaquinones, menadione, and menatetrenone (vitamin K2). |
| 21 | ATP. Creatine |

In alternative aspects, subgroups (categories within each Group) in Table 1 are not necessarily mutually exclusive. By way of non-limiting exemplification, desipramine is listed as a tricyclic antidepressant, and is chemically categorized as such; however, it works on both serotonin and norepinephrine, so it can also be considered an SNRI. In separate aspects, this invention provides protection for each subgroup, and each subgroup may include members of another subgroup; thus, using this non-limiting example, the subgroup of SNRIs includes desipramine.

In alternative aspects, some ingredients of the invention (e.g., exemplary combinations of drugs) provided herein can be administered by more than one route. By non-limiting exemplification, in alternative aspects a number of steroids (see, for example, Table 1, Group 5) can be administered by more than one route, and their mention herein under one list of examples with a common route of administration (e.g. oral administration) does not preclude the understanding that said ingredient may also be administered by a different route; this invention provides that, for each ingredient provided herein, that all possible routes of administration are provided herein and intended for protection herein; likewise, intended for protection herein are kits containing each ingredient provided including ingredient forms suitable for different modes of administration (e.g. a form of said ingredient that can be administered orally, a form of said gradient that can be administered topically, etc.). In separate aspects, this invention provides protection for each subgroup, and each subgroup may include members of another subgroup; thus, using this non-limiting illustration, the subgroup of steroids that can be administered orally, include members of the subgroup of steroids that can be administered by intramuscular injection.

The invention provides alternative embodiments wherein the preparations of the invention comprise ingredients of the invention (e.g., exemplary combinations of drugs of the invention) as exemplified in Table 1, and, in one aspect, are orally ingestible. In a non-limiting exemplification these preparations can be liquids (e.g. that can be orally ingested with the help of a spoon), or powders, capsules, tablets, and pills. In non-limiting exemplifications, they can also be formed into flavored bars (e.g. similar to what bars that are marketed as "power bars", "diet bars", "energy bars", and "nutritional bars").

This invention provides that the ingredients of the invention (e.g., exemplary combinations of drugs of the invention), such as the ingredients exemplified in Table 1 (for making the preparations of the invention) are ingredients that are commercially (and thus can be purchased or manufactured).

Antioxidants. The exemplary combinations of drugs of the invention can use any known antioxidant; thus, specifically provided herein are the thousands of antioxidants currently known. Provided herein are any substances with a measurable positive antioxidant activity. As used herein, a measurable antioxidant value may be determined by test such as ORAC (see discussion, below), HORAC, NORAC, as well as by other tests or methods including the trolox equivalent antioxidant capacity (TEAC) method, the 2,2-azinobis-3-ethylbenzothiazoline-6-sulphonate (ABTS) method, the total radical-trapping antioxidant (TRAP) method, the 2,2-diphenyyl-1-picrylhydrazl (DPPH) method, the dichlorofluorescin-diacetate (DCFH-DA) method and the N,N-dimethyl-p-phenylenediamine (DMPD) method, to name just a few.

Provided herein (e.g., for use in the exemplary combinations of drugs of the invention) are also any substances that have the ability to contribute to an increase in antioxidant activity whether directly or indirectly, e.g. by acting through another molecule or substance, such as by contributing to an increase in catalase activity or in superoxide dismutase (SOD) activity. Ingredients provided herein (e.g., for use in the exemplary combinations of drugs of the invention) include the aforementioned members listed in Table 1 as well as analogs and derivatives thereof that have antioxidant activity, specifically a measurable positive antioxidant activity or value.

In alternative aspects, ingredients used to practice this invention (e.g., for use in the exemplary combinations of drugs of the invention), and in one aspect, methods and compositions of the invention used to treat, prevent and/or ameliorate wasting and/or atrophy, such as muscle atrophy, or methods and compositions of the invention used for protection against oxidants (e.g., used as an antioxidant), as discussed herein, include any single known or groups of ingredients that have a measurable positive ORAC value (or similar value using another comparable test) (ORAC assays and ORAC value discussed in detail, below) of at least about 1 ORAC unit/gram, or, at least about 5 ORAC units/gram, or, at least about 10 ORAC units/gram, or, at least about 20 ORAC units/gram, or, at least about 30 ORAC units/gram, or, at least about 40 ORAC units/gram, or, at least about 50 ORAC units/gram, or, at least about 60 ORAC units/gram, or, at least about 70 ORAC units/gram, or, at least about 80 ORAC units/gram, or, at least about 90 ORAC units/gram, or, at least about 100 ORAC units/gram, or, at least about 200 ORAC units/gram, or, at least about 300 ORAC units/gram, or, at least about 400 ORAC units/gram, or, at least about 500 ORAC units/gram, or, at least about 600 ORAC units/gram, or, at least about 700 ORAC units/gram, or, at least about 800 ORAC units/gram, or, at least about 900 ORAC units/gram, or, at least about 1000 ORAC units/gram, or, at least about 1000 ORAC units/gram, or, at least about 1500 ORAC units/gram, or, at least about 2000 ORAC units/gram, or, at least about 2500 ORAC units/gram, or, at least about 3000 ORAC units/gram, or, at least about 3500 ORAC units/gram, or, at least about 4000 ORAC units/gram, or, at least about 4500 ORAC units/gram, and or, at least about 5000 ORAC units/gram.

### Exemplary ingredient combinations and amounts

Exemplary ingredient combination 1. VB-122

| Ingredients: Beta Blocker NSAID | Exemplary amount: (Absolute amount, mg) or (other unit of measure, e.g. RDA) |
|---|---|
| 1) Propranolol | 20 to 120 mg |
| 2) Etodolac | 200 to400 mg |

### Exemplary ingredient combination 2. VB-122 "Chronopak I"

| Ingredients: Beta Blocker NSAID | Exemplary amount: (Absolute amount, mg) or (other unit of measure, e.g. RDA) |
|---|---|
| 1) Propranolol | 40mg AM 20mg Afternoon 10mg PM |
| 2) Etodolac | 200 mg AM 200 mg Afternoon 400 mg PM |

### Exemplary ingredient combination 3. VB-122 "Chronopak II"

| Ingredients: Beta Blocker NSAID | Exemplary amount: (Absolute amount, mg) or (other unit of measure, e.g. RDA) |
|---|---|
| 1) Propranolol | 80mg AM 40 mg Afternoon 20 mg PM |
| 2) Etodolac | 200 mg AM 200 mg Afternoon 400 mg PM |

### Exemplary ingredient combination 4. VB-132

| Ingredients: Beta Blocker NSAID | Exemplary amount: (Absolute amount, mg) or (other unit of measure, e.g. RDA) |
|---|---|
| 1) Atenolol | 25 to 100 mg |
| 2) Etodolac | 200 to 400 mg |

### Exemplary ingredient combination 5. VB-142

| Ingredients: Beta Blocker NSAID | Exemplary amount: (Absolute amount, mg) or (other unit of measure, e.g. RDA) |
|---|---|
| 1) Metoprolol | 50 to 200 mg |
| 2) Etodolac | 200 to 400 mg |

### Exemplary ingredient combination 6. VB-153

| Ingredients: Beta Blocker NSAID | Exemplary amount: (Absolute amount, mg) or (other unit of measure, e.g. RDA) |
|---|---|
| 1) Nadolol | 40 to 240 mg |
| 2) Naprosyn | 125 to 750 mg |

### Exemplary ingredient combination 7

| Ingredients: ARB NSAID | Exemplary amount: (Absolute amount, mg) or (other unit of measure, e.g. RDA) |
|---|---|
| 1) Losartan | 25 to 100mg |
| 2) Etodolac | 20 to 400mg |

### Exemplary ingredient combination 8

| Ingredients: Beta Blocker ACE Inhibitor | Exemplary amount: (Absolute amount, mg) or (other unit of measure, e.g. RDA) |
|---|---|
| 1) Propranolol | 20 to 120 mg |
| 2) Captopril | 50 mg |

### Exemplary ingredient combination 9

| Ingredients: Beta Blocker ACE Inhibitor | Exemplary amount: (Absolute amount, mg) or (other unit of measure, e.g. RDA) |
|---|---|
| 1) Metoprolol | 50 to 200mg |
| 2) Enalapril | 10 to 40mg |

### Exemplary ingredient combination 10

| Ingredients: Beta Blocker ACE Inhibitor | Exemplary amount: (Absolute amount, mg) or (other unit of measure, e.g. RDA) |
|---|---|
| 1) Carvedilol | 3 to 50mg |
| 2) Quinapril | 10 to 80mg |

### Exemplary ingredient combination 11

| Ingredients: Beta Blocker ARB | Exemplary amount: (Absolute amount, mg) or (other unit of measure, e.g. RDA) |
|---|---|
| 1) Propranolol | 20 to 120mg |
| 2) Losartan | 25 to 100mg |

### Exemplary ingredient combination 12

| Ingredients: Beta Blocker ARB | Exemplary amount: (Absolute amount, mg) or (other unit of measure, e.g. RDA) |
|---|---|
| 1) Metoprolol | 50 to 200 mg |
| 2) Losartan | 25 to 100mg |

### Exemplary ingredient combination 13

| Ingredients: Beta Blocker ARB | Exemplary amount: (Absolute amount, mg) or (other unit of measure, e.g. RDA) |
|---|---|
| 1) Carvedilol | 3 to 50 mg |
| 2) Losartan | 25 to 100 mg |

### Exemplary ingredient combination 14

| Ingredients: Beta Blocker Steroid | Exemplary amount: (Absolute amount, mg) or (other unit of measure, e.g. RDA) |
|---|---|
| 1) Carvedilol | 3 to 50mg |
| 2) Oxandrolone | 5 to 10mg |

### Exemplary ingredient combination 15

| Ingredients: Beta Blocker Steroid | Exemplary amount: (Absolute amount, mg) or (other unit of measure, e.g. RDA) |
|---|---|
| 1) Propranolol | 20 to 120mg |
| 2) Methyltestosterone | 50 mg |

### Exemplary ingredient combination 16

| Ingredients: Beta Blocker Fatty Acid | Exemplary amount: (Absolute amount, mg) or (other unit of measure, e.g. RDA) |
|---|---|
| 1) Propranolol | 20 Mg |
| 2) EPA | 900 Mg |

### Exemplary ingredient combination 17

| Ingredients: Beta Blocker Fatty Acid | Exemplary amount: (Absolute amount, mg) or (other unit of measure, e.g. RDA) |
|---|---|
| 1) Carvedilol | 20 Mg |
| 2) EPA | 900 Mg |

### Exemplary ingredient combination 18

| Ingredients: Beta Blocker NSAID | Exemplary amount: (Absolute amount, mg) or (other unit of measure, e.g. RDA) |
|---|---|
| 1) Carvedilol | 12.5 mg |
| 2) Indomethacin | 75 mg |

### Exemplary ingredient combination 19

| Ingredients: ACEI NSAID | Exemplary amount: (Absolute amount, mg) or (other unit of measure, e.g. RDA) |
|---|---|
| 1) Quinapril | 20 mg |
| 2) Etodolac | 400mg |

### Exemplary ingredient combination 20

| Ingredients: ACEI NSAID | Exemplary amount: (Absolute amount, mg) or (other unit of measure, e.g. RDA) |
|---|---|
| 1) Imidapril | 1.5 mg |
| 2) Naproxen | 500 mg |

### Exemplary ingredient combination 21

| Ingredients: ACE Inhibitor NSAID | Exemplary amount: (Absolute amount, mg) or (other unit of measure, e.g. RDA) |
|---|---|
| 1) Imidapril | 5 mg |
| 2) Celecoxib | 100 to 400mg |

### Exemplary ingredient combination 22

| Ingredients: ACE Inhibitor Steroid | Exemplary amount: (Absolute amount, mg) or (other unit of measure, e.g. RDA) |
|---|---|
| 1) Enalapril | 10 to 40mg |
| 2) Methyltestosterone | 50mg |

### Exemplary ingredient combination 23

| Ingredients: ACE Inhibitor Steroid | Exemplary amount: (Absolute amount, mg) or (other unit of measure, e.g. RDA) |
|---|---|
| 1) Imidapril | 4mg |
| 2) Oxandrolone | 5 to 10mg |

### Exemplary ingredient combination 24

| Ingredients: ARB NSAID | Exemplary amount: (Absolute amount, mg) or (other unit of measure, e.g. RDA) |
|---|---|
| 1) Losartan | 25 to 100mg |
| 2) Etodolac | 200 to 400mg |

### Exemplary ingredient combination 25

| Ingredients: ARB NSAID | Exemplary amount: (Absolute amount, mg) or (other unit of measure, e.g. RDA) |
|---|---|
| 1) Valsartan | 80 to 160mg |
| 2) Ibuprofen | 800mg |

### Exemplary ingredient combination 26

| Ingredients: ARB Steroid | Exemplary amount: (Absolute amount, mg) or (other unit of measure, e.g. RDA) |
|---|---|
| 1) Losartan | 25 to 100mg |
| 2) Oxandrolone | 5 to 10mg |

### Exemplary ingredient combination 27

| Ingredients: ARB Steroid | Exemplary amount: (Absolute amount, mg) or (other unit of measure, e.g. RDA) |
|---|---|
| 1) Candesartan | 16mg |
| 2) Methyltestosterone | 50mg |

### Exemplary ingredient combination 28

| Ingredients: NSAID Steroid | Exemplary amount: (Absolute amount, mg) or (other unit of measure, e.g. RDA) |
|---|---|
| 1) Etodolac | 200 to 400mg |
| 2) Oxandrolone | 5 to 10mg |

### Exemplary ingredient combination 29

| Ingredients: NSAID Steroid | Exemplary amount: (Absolute amount, mg) or (other unit of measure, e.g. RDA) |
|---|---|
| 1) Rofecoxib | 25mg |
| 2) Methyltestosterone | 50mg |

### Exemplary ingredient combination 30

| Ingredients: NSAID Steroid | Exemplary amount: (Absolute amount, mg) or (other unit of measure, e.g. RDA) |
|---|---|
| 1) Celecoxib | 100mg |
| 2) Oxandrolone | 10mg |

### Exemplary ingredient combination 31

| Ingredients: ARB Fatty Acid | Exemplary amount: (Absolute amount, mg) or (other unit of measure, e.g. RDA) |
|---|---|
| 1) Losartan | 50mg |
| 2) EPA | 950mg |

### Exemplary ingredient combination 32

| Ingredients: Beta Blocker ACEI NSAID | Exemplary amount: (Absolute amount, mg) or (other unit of measure, e.g. RDA) |
|---|---|
| 1) Nadolol | 80mg |
| 2) Enalapril | 10 to 40mg |
| 3) Naproxen | 500 |

### Exemplary ingredient combination 33

| Ingredients: Beta Blocker ACE Inhibitor NSAID | Exemplary amount: (Absolute amount, mg) or (other unit of measure, e.g. RDA) |
|---|---|
| 1) Metoprolol | 50mg |
| 2) Imidapril | 3.5mg |
| 3) Etodolac | 300mg |

### Exemplary ingredient combination 34

| Ingredients: Beta Blocker ACE Inhibitor Steroid | Exemplary amount: (Absolute amount, mg) or (other unit of measure, e.g. RDA) |
|---|---|
| 1) Metoprolol | 50mg |
| 2) Quinapril | 20mg |
| 3) Methyltestosterone | 50mg |

### Exemplary ingredient combination 35

| Ingredients: Beta Blocker ACE Inhibitor Steroid | Exemplary amount: (Absolute amount, mg) or (other unit of measure, e.g. RDA) |
|---|---|
| 1) Carvedilol | 6.25mg |
| 2) Captopril | 50mg |
| 3) Stanozol | 10mg |

### Exemplary ingredient combination 36

| Ingredients: Beta Blocker ACE Inhibitor Steroid | Exemplary amount: (Absolute amount, mg) or (other unit of measure, e.g. RDA) |
|---|---|
| 1) Propranolol | 120mg |
| 2) Quinapril | 20mg |
| 3) Oxandrolone | 10mg |

### Exemplary ingredient combination 37

| Ingredients: ARB NSAID Beta Blocker | Exemplary amount: (Absolute amount, mg) or (other unit of measure, e.g. RDA) |
|---|---|
| 1) Losartan | 50mg |
| 2) Etodolac | 300mg |
| 3)Propranolol | 80 mg |

### Exemplary ingredient combination 38

| Ingredients : Beta Blocker ARB NSAID | Exemplary amount: (Absolute amount, mg) or (other unit of measure, e.g. RDA) |
|---|---|
| 1) Metoprolol | 100mg |
| 2) Valdesartan | 80mg |
| 3) Celecoxib | 200 |

### Exemplary ingredient combination 39

| Ingredients: Beta Blocker ARB NSAID | Exemplary amount: (Absolute amount, mg) or (other unit of measure, e.g. RDA) |
|---|---|
| 1) Carvedilol | 12.5mg |
| 2) Candesartan | 16mg |
| 3) Ibuprofen | 800mg |

### Exemplary ingredient combination 40

| Ingredients: Beta Blocker ARB Steroid | Exemplary amount: (Absolute amount, mg) or (other unit of measure, e.g. RDA) |
|---|---|
| 1) Propranolol | 120mg |
| 2) Losartan | 25mg |
| 3) Oxandrolone | 10 |

### Exemplary ingredient combination 41

| lngredients: Beta Blocker ARB Steroid | Exemplary amount: (Absolute amount, mg) or (other unit of measure, e.g. RDA) |
|---|---|
| 1) Atenolol | 50mg |
| 2) Candesartan | 16mg |
| 3) Methyltestosterone | 25mg |

### Exemplary ingredient combination 42

| Ingredients: Beta Blocker ARB Steroid | Exemplary amount: (Absolute amount, mg) or (other unit of measure, e.g. RDA) |
|---|---|
| 1) Carvedilol | 6.25mg |
| 2) Losartan | 25mg |
| 3) Stanozol | 10mg |

### Exemplary ingredient combination 43

| Ingredients: Beta Blocker ACE Inhibitor Fatty Acid | Exemplary amount: (Absolute amount, mg) or (other unit of measure, e.g. RDA) |
|---|---|
| 1) Propranolol | 80mg |
| 2) Enalapril | 20mg |
| 3) EPA | 800mg |

### Exemplary ingredient combination 44

| Ingredients: Beta Blocker ACE Inhibitor Fatty Acid | Exemplary amount: (Absolute amount, mg) or (other unit of measure, e.g. RDA) |
|---|---|
| 1) Nadolol | 80mg |
| 2) Imidapril | 3.25mg |
| 3) EPA | 800mg |

### Exemplary ingredient combination 45

| Ingredients: Beta Blocker ACE Inhibitor Fatty Acid | Exemplary amount: (Absolute amount, mg) or (other unit of measure, e.g. RDA) |
|---|---|
| 1) Metoprolol | 100mg |
| 2) Quinapril | 20mg |
| 3) EPA | 750mg |

### Exemplary ingredient combination 46

| Ingredients: Beta Blocker NSAID Steroid | Exemplary amount: (Absolute amount, mg) or (other unit of measure, e.g. RDA) |
|---|---|
| 1) Propranolol | 60 mg |
| 2) Etodolac | 400 mg |
| 3) Oxandrolone | 10 mg |

### Exemplary ingredient combination 47

| Ingredients: Beta Blocker Bisphosphonate | Exemplary amount: (Absolute amount, mg) or (other unit of measure, e.g. RDA) |
|---|---|
| 1) carvedilol | 5mg |
| 2) clodronate | 800 mg |

### Exemplary ingredient combination 48

| Ingredients: Beta Blocker NSAID Cannabinoid | Exemplary amount: (Absolute amount, mg) or (other unit of measure, e.g. RDA) |
|---|---|
| 1) Metoprolol | 100 mg |
| 2) Celecoxib | 200 mg |
| 3) Dronabinol | 5 mg |

### Exemplary ingredient combination 49

| Ingredients: Anti-TNF Beta Blocker | Exemplary amount: (Absolute amount, mg) or (other unit of measure, e.g. RDA) |
|---|---|
| 1) Thalidomide | 200 mg |
| 2) Carvedilol | 10 mg |

### Exemplary ingredient combination 50

| Ingredients: Immunomodulator NSAID | Exemplary amount: (Absolute amount, mg) or (other unit of measure, e.g. RDA) |
|---|---|
| 1) Tacrolimus | 5 mg |
| 2) Celecoxib | 200 mg |

### Exemplary ingredient combination 51

| Ingredients: Herbal aDA Anti-oxidant Beta Blocker NSAID | Exemplary amount: (Absolute amount, mg) or (other unit of measure, e.g. RDA) |
|---|---|
| 1) St. John's Wort | 5mg |
| 2) Ellagic Acid | 30mg |
| 3) Propranolol | 80mg |
| 4) Etodolac | 200 mg |

### Exemplary ingredient combination 52

| Ingredients: ARB Thalidomide | Exemplary amount: (Absolute amount, mg) or (other unit of measure, e.g. RDA) |
|---|---|
| 1) Losartan | 25mg |
| 2) Thalidomide | 200mg |

### Exemplary ingredient combination 53

| Ingredients: Bisphosphonate Beta Blocker N SAID | Exemplary amount: (Absolute amount, mg) or (other unit of measure, e.g. RDA) |
|---|---|
| 1) Clodronate | 800mg |
| 2) Propranolol | 40mg |
| 3) Naproxen | 150mg |

### Exemplary ingredient combination 54

| Ingredients: Bisphosphanate Beta Blocker NSAID Cannabinoid | Exemplary amount: (Absolute amount, mg) or (other unit of measure, e.g. RDA) |
|---|---|
| 1) Alendronate | 10mg |
| 2) Propranolol | 120mg |
| 3) Etodolac | 300mg |
| 4) Dronabinol | 5 mg |

### Exemplary ingredient combination 55

| Ingredients: Thalidomide Oxandrolone | Exemplary amount: (Absolute amount, mg) or (other unit of measure, e.g. RDA) |
|---|---|
| 1) Thalidomide | 250mg |
| 2) Methyltestosterone | 50mg |

### Exemplary ingredient combination 56

| Ingredients: Thalidomide Oxandrolone | Exemplary amount: (Absolute amount, mg) or (other unit of measure, e.g. RDA) |
|---|---|
| 1) Pentoxifyline | 900mg |
| 2) Stanozol | 10 mg |

### Exemplary ingredient combination 57

| Ingredients: Thalidomide Bisphosphonate Beta Blocker NSAID | Exemplary amount: (Absolute amount, mg) or (other unit of measure, e.g. RDA) |
|---|---|
| 1) Thalidomide | 200mg |
| 2) Alendronate | 10mg |
| 3) Carvedilol | 12.5mg |
| 4) Indomethacin | 75 mg |

### Exemplary ingredient combination 58

| Ingredients: Beta Blocker NSAID Cannabinoid | Exemplary amount: (Absolute amount, mg) or (other unit of measure, e.g. RDA) |
|---|---|
| 1) Propranolol | 120mg |
| 2) Etodolac | 300mg |
| 3) Dronabinol | 5 mg |

### Exemplary ingredient combination 59

| Ingredients: Beta Blocker NSAID Cannabinoid | Exemplary amount: (Absolute amount, mg) or (other unit of measure, e.g. RDA) |
|---|---|
| 1) Nadolol | 120mg |
| 2) Naproxen | 400mg |
| 3) Cannabinol | 30mg |

### Exemplary ingredient combination 60

| Ingredients: Beta Blocker NSAID Cannabinoid | Exemplary amount: (Absolute amount, mg) or (other unit of measure, e.g. RDA) |
|---|---|
| 1) Propranolol | 80mg |
| 2) Celecoxib | 200mg |
| 3) Dronabinol | 10mg |

### Exemplary ingredient combination 61

| Ingredients: Beta Blocker Fatty Acid ACEI | Exemplary amount: (Absolute amount, mg) or (other unit of measure, e.g. RDA) |
|---|---|
| 1) Propranolol | 120mg |
| 2) EPA | 800mg |
| 3) Imidapril | 3.25mg |

### Exemplary ingredient combination 62

| Ingredients: NSAID Cannabinoid | Exemplary amount: (Absolute amount, mg) or (other unit of measure, e.g. RDA) |
|---|---|
| 1) Etodolac | 200mg |
| 2) Dronabinol | 10mg |

### Exemplary ingredient combination 63

| Ingredients: Herbal aDA ARB Beta Blocker Thalidomide | Exemplary amount: (Absolute amount, mg) or (other unit of measure, e.g. RDA) |
|---|---|
| 1) St. Johns Wort | 10mg |
| 2) Candesartan | 20mg |
| 3) Carvedilol | 6.25mg |
| 4) Thalidomide | 200mg |

### Exemplary ingredient combination 64

| Ingredients: Beta Blocker ACE inhibitor Steroid | Exemplary amount: (Absolute amount, mg) or (other unit of measure, e.g. RDA) |
|---|---|
| 1) Propranolol | 60mg |
| 2) Enalapril | 40mg |
| 3) Oxandrolone | 20mg |

### Exemplary ingredient combination 65

| Ingredients: Beta Blocker NOA NSAID | Exemplary amount: (Absolute amount, mg) or (other unit of measure, e.g. RDA) |
|---|---|
| 1) Carteolol | 5 mg |
| 2) Reserpine | 0.1mg |
| 3) Indomethacn | 75mg |

### Exemplary ingredient combination 66

| Ingredients: Beta Blocker TCA NSAID | Exemplary amount: (Absolute amount, mg) or (other unit of measure, e.g. RDA) |
|---|---|
| 1) Atenolol | 50mg |
| 2) Protriptyline | 15 mg |
| 3) Piroxicam | 20mg |

### Exemplary ingredient combination 67

| Ingredients: Beta Blocker NOA NSAID | Exemplary amount: (Absolute amount, mg) or (other unit of measure, e.g. RDA) |
|---|---|
| 1) Nadolol | 80mg |
| 2) Ginseng | 300mg |
| 3) Meloxicam | 7.5 mg |

### Exemplary ingredient combination 68

| Ingredients: ACEI NOA NSAID | Exemplary amount: (Absolute amount, mg) or (other unit of measure, e.g. RDA) |
|---|---|
| 1) Captopril | 50mg |
| 2) Doxepin | 150mg |
| 3) Diclofenac | 600mg |

### Exemplary ingredient combination 69

| Ingredients: ARB NOA NSAID | Exemplary amount: (Absolute amount, mg) or (other unit of measure, e.g. RDA) |
|---|---|
| 1) Valsartan | 100mg |
| 2) Desipramine | 200mg |
| 3) Ketoprofen | 200mg |

### Exemplary ingredient combination 70

| Ingredients: Beta Blocker MAOI Steroid | Exemplary amount: (Absolute amount, mg) or (other unit of measure, e.g. RDA) |
|---|---|
| 1) Propranolol | 60mg |
| 2) Phenelzine | 45mg |
| 3) Methyltestosterone | 50mg |

### Exemplary ingredient combination 71

| Ingredients: NSAID NARI Cannabinoid Anti-oxidant | Exemplary amount: (Absolute amount, mg) or (other unit of measure, e.g. RDA) |
|---|---|
| 1) Etodolac | 400mg |
| 2) Reboxitine | 8 mgmg |
| 3) Dronabinol | 10 mg |
| 4) Alpha lipoic acid | 150mg |

### Exemplary ingredient combination 72

| Ingredients: Anti-TNF Beta blocker TCA Reducing Agent | Exemplary amount: (Absolute amount, mg) or (other unit of measure, e.g. RDA) |
|---|---|
| 1) Thalidomide | 100mg |
| 2) Propranolol | 120mg |
| 3)Imipramine | 150mg |
| 4) Niacin | 200mg |

### Exemplary ingredient combination 73

| Ingredients: Beta Blocker NSAID Reducing Agent | Exemplary amount: (Absolute amount, mg) or (other unit of measure, e.g. RDA) |
|---|---|
| 1) Propranolol | 80mg |
| 2) Etodolac | 400mg |
| 3) Niacin | 200 |

### Exemplary ingredient combination 74

| Ingredients: ARB NSAID Amino Acids Cannabinoid | Exemplary amount: (Absolute amount, mg) or (other unit of measure, e.g. RDA) |
|---|---|
| 1) Olmesartan | 5mg |
| 2) Etodolac | 150mg |
| 3) Essential AA mix | 750mg |

### Exemplary ingredient combination 75

| Ingredients: ACEI Statin NSAID | Exemplary amount: (Absolute amount, mg) or (other unit of measure, e.g. RDA) | |
|---|---|---|
| 1) Enalapril | 20mg | |
| 2) Fluvastatin | 20mg | |
| 3) Aspirin | 325mg | |

### Exemplary ingredient combination 76

| Ingredients: Beta Blocker Statin NSAID | Exemplary amount: (Absolute amount, mg) or (other unit of measure, e.g. RDA) | |
|---|---|---|
| 1) Propranolol | 120mg | |
| 2) Rosuvastatin | 20mg | |
| 3) Etodolac | 300mg | |

### Exemplary ingredient combination 77

| Ingredients: Beta Blocker NSAID Amino Acids Reducing Agent Steroid | Exemplary amount: (Absolute amount, mg) or (other unit of measure, e.g. RDA) |
|---|---|
| 1) Metoprolol | 50mg |
| 2) Celecoxib | 200mg |
| 3) AA Mix | 500mg |
| 4) Niacin | 250mg |
| 5) Stanozol | 6mg |

### Exemplary ingredient combination 78

| Ingredients: Amino Acids Vitamins ARB Fatty Acid | Exemplary amount: (Absolute amount, mg) or (other unit of measure, e.g. RDA) |
|---|---|
| 1) AA Mix | 500mg |
| 2) Vitamin Mix | 200mg |
| 3) Candesartan | 15mg |
| 4) EPA | 50000gm |

### Exemplary ingredient combination 79

| Ingredients: ACEI NSAID Vitamins Anti-oxidant Fatty Acid | Exemplary amount: (Absolute amount, mg) or (other unit of measure, e.g. RDA) |
|---|---|
| 1) Imidapril | 1.67mg |
| 2) Etodolac | 800mg |
| 3) Vitamin mix | 1500mg |
| 4) Niacin | 200 mg |
| 5) EPA | 5000mg |

### Exemplary ingredient combination 80

| Ingredients: Calcium Channel Blocker NSAID | Exemplary amount: (Absolute amount, mg) or (other unit of measure, e.g. RDA) |
|---|---|
| 1) Diltiazem | 120mg |
| 2) Ibuprofen | 800mg |

### Exemplary ingredient combination 81

| Ingredients: Calcium Channel Blocker NSAID | Exemplary amount: (Absolute amount, mg) or (other unit of measure, e.g. RDA) |
|---|---|
| 1) Nisoldipine | 20mg |
| 2) Diclofenac | 600mg |

### Exemplary ingredient combination 82

| Ingredients: Calcium Channel Blocker NSAID | Exemplary amount: (Absolute amount, mg) or (other unit of measure, e.g. RDA) |
|---|---|
| 1) Verapamil | 100mg |
| 2) Celecoxib | 200 mg |

### Exemplary ingredient combination 83

| Ingredients: Calcium Channel Blocker NSAID | Exemplary amount: (Absolute amount, mg) or (other unit of measure, e.g. RDA) |
|---|---|
| 1) Menthol | 5 mg |
| 2) Aspirin | 325mg |

### Exemplary ingredient combination 84

| Ingredients: Calcium Channel Blocker Fatty Acid Immunomodulator | Exemplary amount: (Absolute amount, mg) or (other unit of measure, e.g. RDA) |
|---|---|
| 1) Verapamil | 100mg |
| 2) EPA | 700mg |
| 3) Lenalidomide | 10mg |

### Exemplary ingredient combination 85

| Ingredients: Calcium Channel Block Antioxidant Bisphosphonate | Exemplary amount: (Absolute amount, mg) or (other unit of measure, e.g. RDA) |
|---|---|
| 1) Diltiazem | 120mg |
| 2) Resveratrol | 40mg |
| 3) Risedronate | 30mg |

### Exemplary ingredient combination 86

| Ingredients: Beta blocker NSAID Steroid Cannabinoid Amino Acids Vitamins Anti-oxidant | Exemplary amount: (Absolute amount, mg) or (other unit of measure, e.g. RDA) |
|---|---|
| 1) Propranolol | 120mg- |
| 2) Rofecoxib | 25 mg |
| 3)Oxandrolone | 10mg |
| 4) Dronabinol | 10 mg |
| 5) Essential Amino Acid Mix | 2000mg |
| 6) Vitamins | 1000mg |
| 7) Niacin | 250mg |

### Exemplary ingredient combination 87

| Ingredients: ARB Anti-TNF Cannabinoid Statin Vitamins | Exemplary amount: (Absolute amount, mg) or (other unit of measure, e.g. RDA) |
|---|---|
| 1) Valsartan | 100 mg |
| 2) Thalidomide | 200mg |
| 3) Cannabidiol | 30mg |
| 4) Atorvastatin | 60mg |
| 5)Vitamin Mix | 600mg |

### Exemplary ingredient combination 88

| Ingredients: Beta Blocker NSAID Growth Hormone Cannabinoid | Exemplary amount: (Absolute amount, mg) or (other unit of measure, e.g. RDA) |
|---|---|
| 1) Timolol | 20 mg |
| 2) Indomethacin | 75 mg |
| 3) rHGH | .0125mg.kg |
| 4) Dronabinol | 10mg |

### Exemplary ingredient combination 89

| Ingredients: ACEI Fatty Acid Bisphosphonate | Exemplary amount: (Absolute amount, mg) or (other unit of measure, e.g. RDA) |
|---|---|
| 1) Quinapril | 20mg |
| 2) EPA | 900mg |
| 3) Ibandronate | 2.5 mg |

### Exemplary ingredient combination 90

| Ingredients: Beta blocker TFPI | Exemplary amount: (Absolute amount, mg) or (other unit of measure, e.g. RDA) |
|---|---|
| 1) Nadolol | 80mg |
| 2) TFPI | 0.05mg/kg bolus, 0.2mg/kg/hour infusion for 6 hours |

### Methods of administration

This invention provides compositions (e.g., for use in the exemplary combinations of drugs of the invention), e.g., pharmaceutical compositions, preparations and kits, that can be administered by several routes, including intravenous, topical and oral. Furthermore, in separate embodiments, this invention provides forms of compositions, preparations and kits that can be administered by inhalation, infusion or injection, (e.g., intraperitoneal, intramuscular, subcutaneous, intra-aural, intra-articular, intra-mammary, etc.), topical application (e.g., on areas, such as eyes, ears, skin or on afflictions such as wounds, bums, etc.), and by absorption through epithelial or mucocutaneous linings (e.g. vaginal and other epithelial linings, gastrointestinal mucosa, etc.). Methods are known for making compositions, preparations and kits containing the present components that are suitable for each of these methods of administration as well as other methods of administration that are know in the art.

For example, in alternative embodiments, this invention provides compositions, preparations and kits in liquid forms that can be administered orally. The compositions, preparations and kits can be also prepared as capsules, gels, geltabs, tablets, powders, sprays, aerosols, pellets (e.g. for animal consumption), suppositories, or creams and ointments. The compositions, preparations and kits can be also prepared as physiological solutions suitable for I.V. administration or other parenteral administration.

In as many separate aspects, this invention also provides all the possible combinations of component quantities that are possible (e.g. the total of all the components does not surpass 100% of the relevant total dosage compositions, preparations and kits, and admixing or solubility limitations are not exceeded).

In one aspect, a multi-ingredient kit, as provided herein, may contain two or more ingredients in approximately equal amounts. An amount may be determined, e.g. by mass or by weight or by molar amount. In another aspect, a multi-ingredient kit, as provided herein, may contain two or more ingredients in unequal amounts. In another aspect, a multi-ingredient kit, as provided herein, may contain two or more ingredients in approximately equal amounts as well as one or more ingredients that are not in unequal amounts.

Thus, in one aspect, a multi-ingredient kit, as provided herein, may contain two or more ingredients in approximately equimolar amounts. In another aspect, a multi-ingredient kit, as provided herein, may contain two or more ingredients that are not in equimolar amounts. In another aspect, a multi-ingredient kit, as provided herein, may contain two or more ingredients that are in approximately equimolar amounts as well as one or more ingredients that are not in equimolar amounts.

In another aspect, said multi-ingredient kit, as provided herein, may contain two or more ingredients that are admixed. In another aspect, said multi-ingredient kit, as provided herein, may contain two or more ingredients that are not admixed. In another aspect, said multi-ingredient kit, as provided herein, may contain two or more ingredients that are partially admixed. In another aspect, said multi-ingredient kit, as provided herein, may contain two or more ingredients that are at least partially admixed, as well as one or more ingredients that are not admixed. An ingredient in a multi-ingredient kit, as provided herein, may be liquid forms that can be administered orally.

An ingredient in a multi-ingredient kit, as provided herein, may also be in delivery forms such as capsules, tablets, powders, sprays, aerosols, pellets (e.g. for animal consumption), suppositories, or creams and ointments. An ingredient in a multi-ingredient kit, as provided herein, may also be in delivery forms such as physiological solutions suitable for I.V. administration or other parenteral administration.

The ingredients in a multi-ingredient kit, as provided herein, may be separated by physically compartmentalization (e.g. in separate compartments that are part of said kit, where said kit is a multi-compartment kit). Thus, for example, it is provided that the ingredients may be admixed or not admixed. For example, a single pill or capsule may contain more than one key ingredient (e.g. a BB and an NSAID). Alternatively, separate compartments, as may be found in a "blister pack" type of packaging, may contain different ingredients.

### Methods for treating or ameliorating a condition or disease

The invention provides methods for treating or ameliorating a condition or disease comprising a chronic Systemic Inflammatory Response State (SIRS) comprising the steps of administering a therapeutically effective amount of a therapeutic combination of the invention or a pharmaceutical composition of the invention, thereby treating or ameliorating the condition or disease. The invention provides combination pharmaceutical compositions, preparations, formulations, kits and products of manufacture that can be used for treating or ameliorating disease symptoms and diseases states comprising inflammation, excessive sympathoneural drive (sympathetic nerve activity), cachexia, anorexia, and anorexia-cachexia, and stress or anxiety related thereto for the purpose of improving one or more undesirable symptoms associated with the disease states or for slowing the progression (worsening) of one or more symptoms associated with these disease symptoms and disease states.

Thus, the invention provides combination compositions (products of manufacture), e.g., pharmaceutical compositions, preparations, formulations, kits, for treating or ameliorating the symptoms and/or effects of cachexia, which is a severe and debilitating muscle wasting disorder that affects many terminal cancer patients. Thus, the compositions and methods of the invention, by treating or ameliorating the symptoms and/or effects of cachexia, can decrease the significant patient morbidity seen in cachexia, and ameliorate or abrogate the weakness, fatigue, gastrointestinal distress, sleep/wake disturbances, pain, listlessness, shortness of breath, lethargy, depression and/or malaise associated with cachexia. Additionally, administration of compositions of the invention have additional psychological effects on the individual, e.g., by ameliorating the fear of being a burden on family and friends. In addition, administration of compositions of the invention ameliorate the condition where cachectic patients have substantially poorer tolerance to therapy and higher mortality. Cachectic cancer patients have a 50% reduction in life expectancy relative to non-cachectic cancer patients. Cachexia results in the direct cause of death in more than 20% of terminal cancer patients.

Additionally, the compositions and methods of the invention, by treating or ameliorating the symptoms and/or effects of the complex syndrome of cachexia, including treating or ameliorating the weight loss, lipolysis, loss of muscle and visceral protein, anorexia, chronic nausea and weakness associated with cachexia. In addition, the compositions and methods of the invention the can treat or ameliorate the anemia, immunosuppression, insulin resistance, anxiety and sleep disturbances associated with cachexia.

The syndromes, conditions and diseases that can be treated or ameliorated by administering the compositions of the invention, or practicing the methods of the invention, include syndromes, conditions and diseases characterized as Chronic Systemic Inflammatory Response Syndrome (SIRS) or Chronic SIRS, or associated with Chronic SIRS. Because severe cachexia occurs in most patients with advanced cancer, AIDS and end-stage chronic diseases, in one aspect administering the compositions of the invention, or practicing the methods of the invention, can treat or ameliorate advanced cancer (e.g., tumors of the pancreas, stomach, upper gastrointestinal system, and lung), AIDS and end-stage chronic diseases and their symptoms. More than 80% of patients with cancer (particularly those with tumors of the pancreas, stomach, upper gastrointestinal system, and lung) or AIDS develop cachexia before death. At the moment of diagnosis, about 80% of patients with upper gastrointestinal cancers and 60% of patients with lung cancer have substantial weight loss. In general, patients with solid tumors (with the exception of breast cancer) have a higher frequency of cachexia. Cachexia is also more common in children and elderly patients and becomes more pronounced as disease progresses.

In identifying a patient population that would benefit by the administration of the compositions of the invention, or practicing the methods of the invention, any clinically acceptable protocols can be used, e.g., for diagnosing cachexia, e.g., cancer cachexia, AIDS or other end-stage chronic diseases, congestive heart failure (CHF), severe injuries (e.g., bums) can be used. For example, in one aspect, the methods and compositions of the invention are used on a patient population suffering from cachexia, e.g., cancer cachexia, as diagnosed by symptoms comprising having a sustained elevated heart rate of at least about 6 beats per minute (BPM) (over what would be considered normal for that particular individual) and having weight loss.

In alternative aspects, diagnosis of severe inflammation, excessive sympathoneural drive (sympathetic nerve activity), anorexia and anorexia-cachexia, severe stress or anxiety also provides an indication to administer the compositions of the invention or practice the methods of the invention.

In alternative aspects, diagnosis of any conditions (physical or psychological) or diseases comprising an escalating stress response, a maladaptive immune system feedback and/or a severe dysregulated psycho-neuroendocrine-immune state also provide an indication to administer the compositions of the invention or practice the methods of the invention. Thus, the invention provides specific criteria for diagnosing a patient population for practicing this invention, including specific criteria for identifying individuals having cachexia, e.g., cancer cachexia (versus anorexia), who are appropriate recipients of the treatments and compositions of this invention. Accordingly, in one aspect this invention provides a) signature markers to distinguish cachexia from anorexia (e.g., symptoms comprising having an elevated sustained heart rate (over what would be considered normal for that particular individual) of at least about 6 beats per minute (BPM) and having weight loss), and b) effective therapies for treating cachexia.

Thus, in one aspect, cachexia is defined as a "maladaptive nutritional state characterized by hyper-catabolism and deficient or impaired protein-sparing mechanisms" (normally, in starvation or nutritionally-deprived states the body spares protein over fat; this "protein sparing" is lost when an individual is in a "cachexic" state). In one aspect, cachexia is distinguished (e.g. from anorexia) by characteristic parameters that are exemplified in non-limiting fashion by 1) a hyper-inflammatory state, 2) altered hormone levels and/or cytokine levels; 3) increased sustained heart rate variability (over what would be considered normal for a particular individual); and/or 4) sustained increased heart rate (over what would be considered normal for a particular individual), e.g., having an elevated heart rate of at least about 6 bpm. For example, a sustained (i.e. not an acute exercise-induced short term change) heart rate increase of about 6 bpm or more is one easily measurable parameter that is used to determine a patient population to practice this invention, e.g., to distinguish cachexia from anorexia.

In contrast to the present invention, in the North Central Cancer Treatment Group (NCCTG) research trials involving over 2,300 patients, a very simple criteria for anorexia/cachexia was used:
■ A 5-lb weight loss in the preceding 2 months and/or an estimated daily caloric intake < 20 calories/kg.
■ A desire by the patient to increase his or her appetite and gain weight.
■ The physician's opinion that weight gain would be beneficial for the patient.

Thus, the invention provides specific easily defmed and measurable criteria for diagnosing cachexia, including cancer cachexia, to identify individuals to which it is appropriate to administer the compositions of the invention, or practice the methods of the invention.

In another aspect, the compositions and methods (and uses) of the invention are used to treat, ameliorate or prevent anorexia. The invention's methods for distinguishing between anorexia and cachexia allows for a differential diagnosis of (identifying the distinction between) anorexia and cachexia. For example, in one aspect, the invention provides methods for ameliorating cachexia, anorexia, anorexia-cachexia, stress and/or anxiety related to a cancer comprising administering to an individual in need thereof a pharmaceutically effective amount of the therapeutic combination of the invention, or the pharmaceutical composition of the invention.

In one aspect, in practicing the invention cachexia is identified as a maladaptive nutritional state secondary to a chronic systemic inflammatory response and autonomic dysregulation. While the invention is not limited by any particular mechanism of action, in one aspect, the natural history of a healthy inflammatory response is characterized by a sequence of feedback responses beginning with tissue breakdown leading to a stress response of inflammation and immune activation, and finally restoration of tissue integrity. If the sequence of events is within normal adaptive range, both in terms of the extent of trauma and duration of the response, then tissue integrity is restored. However, in some trauma (e.g., cancer, congestive heart failure (CHF), AIDS, burn, etc) a positive feedback cycle of escalating stress response and maladaptive immune system feedback leads to a severe dysregulated psycho-neuroendocrine-immune state.

Administering the compositions of the invention or practicing the methods of the invention can effectively help address the complexity of the treatment and management of these diseases, e.g., cachexia in its many forms and many causes; practicing the invention will make these easier to manage. While the invention is not limited by any particular mechanism of action, in one aspect the autonomic nervous system (ANS) and the inflammatory response are underlying causes of diseases treated by this invention. Thus, the invention comprises use of compounds that regulate the autonomic nervous system, in one aspect the adrenergic system, with beta blockers and in combination with other drugs, e.g., anti-inflammatory drugs, such as non-steroidal anti-inflammatory drugs (NSAID) and eicosapentaenoic acid (EPA). In one aspect, the compositions of the invention or practicing the methods of the invention are a treatment for chronic SIRS, e.g., cachexia.

While the invention is not limited by any particular mechanism of action, in some situations the cachexia syndrome may be associated with malign patterns of eicosanoid production and the presence of a chaotic population of cytokines with consequent aberrations in neuroendocrine-immune control systems, hypothalamic control of appetite, and intermediary metabolism. Thus, detection or diagnosis of aberrations in neuroendocrine-immune control systems, hypothalamic control of appetite, and intermediary metabolism can be used as an indication for administration of compositions of the invention, or to practice the methods of the invention.

Metabolic changes also may be associated with changes in autonomic control and an increase in body energy consumption, with further mismatches of food intake with need. The systemic aberrations are similar to the acute stress reaction commonly observed in patients with life-threatening infections or major trauma. When this potentially life-saving "switch" is left on, over time the chronic stress inflammatory reaction may enhance tumor growth while inducing a devastating effect on a variety of organs and tissues. Thus, chronic stress, life-threatening infections or major trauma can be used as an indication for administration of compositions of the invention, or to practice the methods of the invention.

Wasting cancer patients, or individuals diagnosed with "wasting syndrome", are also a patient population for administration of compositions of the invention, or to practice the methods of the invention. In wasting cancer patients, muscle synthesis and repair decline, whereas increased muscle proteolysis occurs, possibly due to tumor-produced proteolysis factors. The wasting syndrome commonly noted at the time of diagnosis in many forms of cancer, notably those of the upper gastrointestinal tract and lung, progresses through the course of illness, finally leaving the patient in a severe state of malnutrition. A recent review details current concepts of this pathophysiology and the relevance of these concepts to anorexia-cachexia research.

Because markers of chronic inflammation (e.g., C reactive protein, or CRP) and increased production of certain cytokines, notably interleukin (IL)-6, TNFα and interferon (IFN)-γ correlate with both cachexia and with poor prognosis in cancer patients and other illnesses, non-normal levels of these markers can be used to determine whether an individual would benefit from administration of compositions of the invention, or to practice the methods of the invention. Although evidence has been presented for circulatory levels of tumor catabolic products in humans, there is less evidence for circulating cytokines. In many cases where serum levels of cytokines such as TNFα are elevated, these levels correlate with the stage of the disease, reflecting tumor size and metastasis and not specifically with cachexia. Chronic inflammation is also an indicator that an individual would benefit from administration of compositions of the invention, or to practice the methods of the invention, and it correlates with other symptoms, such as fatigue, which are common in cancer and other chronic illnesses.

The autonomic nervous system helps to control arterial pressure, gastrointestinal motility, urinary bladder emptying, sweating, body temperature, and many other activities. The autonomic nervous system is primarily activated by centers in the spinal cord, brain stem, and hypothalamus. The efferent autonomic signals are transmitted to the various organs of the body through either the sympathetic nervous system or the parasympathetic nervous system. The neurotransmitter of all preganglionic autonomic fibers, all postganglionic parasympathetic fibers, and a few postganglionic sympathetic fibers is acetylcholine. These are referred to as cholinergic fibers. The adrenergic fibers compose the majority of the postganglionic sympathetic fibers and use the neurotransmitter norepinephrine (noradrenaline).

In cancer patients elevated resting energy expenditure is not likely due to tumor metabolism, but more likely it is due to an interaction between the cancer and the neuroendocrine-immune system. While the invention is not limited by any particular mechanism of action, the elevated resting energy metabolism secondary to tumor metabolism is primarily due to the increased adrenergic activity in cancer patients, and this increased adrenergic activity is ameliorated by administration of compositions of the invention, or to practice the methods of the invention.

Administration of compositions of the invention, or practicing the methods of the invention, can be in conjunction with other treatments for cachexia, e.g., a variety of interventions including hormones and steroids, cytokines, nutritional supplementation, and appetite stimulation. In administering hormones and steroids, cytokines, nutritional supplementation, and appetite stimulation, are useful in treating aspects of the disease, but should be administered in the correct sequence or combination. For example, adding an anabolic steroid while there is degradation of fat and muscle will cause increased cycling and more energy use since the protein is being rapidly broken down to supply other processes. Likewise, adding only nutritional supplementation will be difficult, since the patient has no appetite and will be ineffective since the use of body energy reserves and nutrients is imbalanced. In some aspects, administration of compositions of the invention, or practicing the methods of the invention, addresses this problem.

Administration of compositions of the invention, or practicing the methods of the invention, can be used in conjunction with diagnoses using the following diagnostic markers (intervention points), and therapeutics:
■ aberrant eicosanoid metabolism with nonsteroidal anti-inflammatory agents [NSAIDs], omega-3 fatty acids;
■ amino acid deficiencies relevant to need with tailored protein supplements, specific amino acid therapy;
■ altered energy mechanisms in muscle cells with creatine, ATP, angiotensin-converting enzyme (ACE) inhibitors;
■ impaired muscle synthesis and muscle repair with anabolic steroids;

As discussed above, administration of compositions of the invention, or practicing the methods of the invention, can be used to treat or ameliorate conditions or symptoms associated with maladaptive immune responses, maladaptive infection responses (e.g., those dominated by gamma interferon), maladaptive trauma response (e.g., dominated by IL-6) or maladaptive wound healing response (e.g., dominated by TNF or IL-1), for example, including cancer cachexia as a maladaptive nutritional state secondary to a chronic Systemic Inflammatory Response State (SIRS). The natural history of healthy inflammation is characterized by a sequence of flux imbalances and positive feedback responses. The sequence proceeds with (1) a breakdown in tissue integrity due to trauma, infection, cancer, etc.; (2) stress, with associated imbalanced metabolic and signaling fluxes; (3) a stress response characterized by a cycle of immune activation, inflammation and healing, and (4) ultimate restoration of tissue integrity, balanced fluxes (homeostasis), and immune inactivation. As long as the trauma is within the adaptive range and the immune system correctly partitions, escalates and de-escalates its immune response, the process is adaptive and restores tissue integrity.

However, in cancer, for example, the immune response is maladaptive and does not restore tissue homeostasis. Instead, the cancer progresses, leading to a positive feedback cycle of escalating cancer induced flux imbalances and escalating maladaptive immune system feedback responses. The escalating flux imbalances and maladaptive feedback responses leads to a severe dysregulated neuroendocrine-immune state. The specific pattern of neuroendocrine-immune imbalances appears to partition into three broad classes of maladaptive infection response (dominated by gamma interferon), maladaptive trauma response (dominated by IL-6) or maladaptive wound healing response (dominated by TNF or IL-1). The overall state is characterized by a chronic SIRS.

In the normal state a weight-stable adult takes in energy taken that matches the amount used by the body. If energy supply is too low (fasting state) the body compensates by releasing orexigenic ("increasing the appetite") hormones to stimulate feeding and reducing total energy expenditure through the sympathetic nervous system, or more generally the neuroendocrine-immune system. If energy supply is in excess (overfed state) the body stores the excess primarily as fat. In normal adaptive nutritional states, control of the use of energy stores (protein, fat and carbohydrates) is by a number of factors including insulin/glucagon, catecholamines, ghrelin and related orexigenic and anorectic peptide signals from the neuroendocrine-immune system. The result of this regulation is the use of carbohydrates first, followed by mobilization and oxidation of fatty acids and finally degradation of protein and release of gluconeogenic amino acids.

In contrast, in cachexia, a stressed state, there is a dysregulation of this system such that protein is not spared at the expense of carbohydrates or fat and there is no reduction in metabolic rate. Administration of compositions of the invention, or practicing the methods of the invention, can be used to treat or ameliorate dysregulation associated with a stressed state, e.g., cachexia.

The autonomic nervous system is central to regulation of the immune and nutritional systems and throughout the body. Antagonists of β-adrenergic receptors (beta-blockers) address the underlying autonomic disorder. These drugs also reduce the inflammatory cytokines, but the additions of NSAIDS or EPA are needed to effectively reduce this response. Beta blockers also reduce the signaling pathway to lipolysis and muscle degradation. Beta blockade has reduced REE in cardiac and burn cachexia and has demonstrated short term effects in cancer cachexia. Since the underlying imbalances are the same for cachexia due to cancer, HIV/AIDS, CHF, burn, COPD and others, as indicated by similar inflammatory and autonomic nervous system dysregulation, they should all respond well to treatment that targets both systems.

In addition, NSAIDs tend to increase blood pressure and therefore provide synergistic effects with beta-blockers by offsetting the potentially adverse hypotensive effect of beta-blockers. Many NSAIDS and EPA also show a direct effect on the cancer itself by regulating angiogenesis, apoptosis, metastasis and energy utilization. Therefore, these agents will be particularly beneficial for cancer patients.

Once autonomic nervous system dysregulation is brought under control by beta blockade and inflammation is reduced by NSAIDs, EPA or other agents then other therapies can become effective. The purpose of the beta-blockers is to reduce overall flux and the energy demand of the system so it is not beyond normal adaptive ranges. When the demand is reduced to within normal ranges the body is able to resolve residual signal and will return to a normal state. Once this state is reached, the process of building muscle can begin. This process can be enhanced by drugs such as anabolic steroids and beta2 agonists.

While the invention is not limited by any particular mechanism of action, the embodiments of the invention comprising a combination treatment, or a therapeutic combination, or a pharmaceutical composition, comprising a non-selective beta blocker and an NSAID will provide the reduction in energy use and the activity of the autonomic nervous system while also providing a means to reduce inflammatory response by reducing prostaglandin biosynthesis.

In previous clinical trials on cachectic severely burned children propranolol was associated with reduction in elevated inflammatory cytokines, elevated resting energy expenditure and rate of loss of lean body mass. In cachectic metastatic cancer patients propranolol showed a reduction in elevated inflammatory cytokines and elevated resting energy expenditure. No adverse events were reported in those trials.

In a large retrospective study, etodolac demonstrated the lowest GI complications rate among non-specific Cox inhibitors without the reported increase in cardiovascular events associated with specific cox2 inhibitors. In addition, etodolac is currently in an investigator-lead Phase I/II clinical trial adjunct to standard chemotherapy for breast cancer and the R(-) isomer of etodolac is in a commercial Phase II clinical trial for treatment of Chronic Lymphocytic Leukemia.

While the invention is not limited by any particular mechanism of action, in this combination of the invention (a non-selective beta blocker and an NSAID) the relative activity against the different receptor subtypes is balanced, thereby providing the broadest therapeutic effect. The presence of the etodolac can serve to maintain blood pressure and to reduce the inflammatory response caused by the disease. In addition, this combination of the invention will provide for reduced anxiety which will help deal with the social aspects of cachexia and in combination with etodolac will allow the patient to sleep easier. Therefore, this aspect of the invention, including methods and compositions for treating and ameliorating cachexia, addresses both the neuroendocrine-immune and the inflammatory disorder underlying the disease.

The following table (from Gilman 2001) provides guidelines to practice the compositions and methods of the invention: it gives an overview of the different major organ systems and sub-systems, what the predominant receptor type is and how it reacts to adrenergic impulse.

| **Organ** | | **Receptor Type** | **Adrenergic Impulse Response** |
|---|---|---|---|
| **Heart** | | | |
| | SA node | beta1, beta2 | increase heart rate |
| | Atria | beta1, beta2 | increase contractility |
| | AV node | beta1, beta2 | increase conduction velocity |
| | His-Purkinje | beta1, beta2 | increase conduction velocity |
| | Ventricles | beta1, beta2 | increase contractility, conduction velocity, pacemaker rate |
| **Arterioles** | | | |
| | Coronary | alpha1, alpha2, beta2 | constriction / dilation |
| | Skin and Mucosa | alpha1, alpha2 | constriction |
| | Skeletal Muscle | alphal, beta2 | constriction / dilation |
| | Cerebral | alpha1 | constriction |
| | Pulmonary | alphal, beta2 | constriction |
| | Abdominal Viscera | alphal, beta2 | constriction / dilation |
| | Renal | alphal, alpha2, beta1, beta2 | constriction / dilation |
| **Veins** | | | |
| | Systemic | alphal, alpha2, beta2 | constriction/dilation |
| **Lung** | | beta2 | relaxation/contraction |
| | Tracheal and bronchial muscle | beta2 | relaxation |
| | Bronchial Glands | alphal, beta2 | decreased/increased secretion |
| **Stomach, Intestine** | | | |
| | Motility, tone | alpha1, alpha2, beta2 | decrease |
| | Sphincters | alpha1 | contraction |
| | Secretion | | inhibition |
| **Kidney** | | | |
| | Renin Secretion | alpha1, beta1 | decrease/increase |
| **Skeletal Muscle** | | beta2 | increase contraction, glycogenolysis |
| **Liver** | | alphal, beta2 | Glycogenolysis, gluconeogenesis |
| **Pancreas** | | alpha2, beta2 | secretion |
| | Acini | alpha | decreased secretion |
| | Islets (beta cells) | alpha2 | decreased secretion |
| | | beta2 | increaed secretion |
| **Adipose** | | alpha2, beta1, beta2, beta3 | lipolysis (thermogenesis)/inhibi tion of lipolysis |

The importance of the beta1 in energy metabolism of the heart is well documented as is the role of beta2 effect on the skeletal muscle and visceral organs and beta3 as the receptor of prime importance in the adipose.

The following table provides guidelines to practice the compositions and methods of the invention: it gives highlights some of the key features of various beta blockers including primary receptor subtype targeted and pharmacokinetic and pharmacodynamic properties. This table is not meant to include all beta blockers that can be used in practicing the compositions and methods of the invention, including the treatment of cachexia, but represents some exemplary drugs, which are some of the more common drugs, and their important characteristics.

| Non-selective Beta Blockers | | | | | | | |
|---|---|---|---|---|---|---|---|
| Drug | Site of Effect | Oral Absorp. (%) | Biotrans-formation | Half-life (hr) | Time to Peak Effect Single dose (hr) | Elimination (% un-changed) | Removable by Hemodialysis |
| Carteolol | Beta-1; Beta-2 | 85 | Hepatic (minimal) | 6 | 1-3 | Renal (50-70) | ? |
| Labetalo1 | Beta-1; Beta-2 | 100 | Hepatic | 6-8 oral ~5.5 IV | 2-4 (oral); 5 min (IV) | 55-60%, <5 Renal; Biliary/fecal | No |
| Nadolol | Beta-1; Beta-2 | 30 | None | 20-24 | 4 | Renal(70) | Yes |
| Oxprenolol | Beta-1; Beta-2 | 90 | Hepatic | 1.3-1.5 | ? | Renal (<5) | ? |
| Penbutolol | Beta-1; Beta-2 | 100 | Hepatic | 5 | 1.5-3 | 90% (0) Renal | No |
| Pindolol | Beta-1; Beta-2 | 90-100 | Hepatic | 3-4 | 1-2 | Renal (40) | ? |
| Propranolol | Beta-1; Beta-2 | 90 | Hepatic | 3-5 | 1-1.5 | Renal (<1) | No |
| Sotalol | Beta-1; Beta-2 | > 80 | Hepatic | 7-18 | 2-3 | Renal (75) | Yes |
| Timolol | Beta-1; Beta-2 | 90 | Hepatic | 4 | 1-2 | Renal (20); Fecal | No |
| Carvedilol | Beta-1 Beta-2 alpha1 | 25-35 | Hepatic | 7-10 | ? | < 2% Renal | No |

| Beta1 Selective Beta Blockers | | | | | | | |
|---|---|---|---|---|---|---|---|
| Drug | Site of Effect | Oral Absorp. (%) | Biotrans-formation | Half-life (hr) | Time to Peak Effect Single dose (hr) | Elimination (% un-changed) | Removable by Hemodialys is |
| Acebutolol | Beta-1 | 70 | Hepatic | 3-4 | 2.5 | 30-40% Renal; 50-60% Biliary/fecal | Yes |
| Atenolol | Beta-1 | 50-60 | Hepatic (minimal) | 6-7 | 2-4 | 85-100% Renal | Yes |
| Betaxolol | Beta-1 | 80-89 | Hepatic | 14-22 | 3-4 | >80% (15) Renal | No |
| Bisoprolol | Beta-1 | 80-90 | Hepatic | 9-12 | | Renal (50) <2% Fecal | No |
| Metoprolol | Beta-1 | 95 | Hepatic | 3-7 | 1-2 (std); 6-12 (LA); 20 min (IV) | Renal (3-10) | No |

The following table describes exemplary dosing guidelines, and selected physicochemical properties, and for some selected beta blockers that are used in practicing the compositions and methods of the invention:

### Pharmacokinetic and Dosing Characteristics of Common Beta Blockers

| **Name** | **Lipophilic/ Hydrophilic** | **Start Dose** | **Max Dose** |
|---|---|---|---|
| Propranolol | Lipo (+++) | 40-80 mg/day | 320 mg LA OD |
| Nadolol | Hydr (+++) | 40 mg/day | 120 mg bid |
| Timolol | Lipo (++) | 20-40 mg/day | 20 mg tid |
| Pindolol | Lipo (++) | 10 mg/day | 20 mg bid |
| Metoprolol | Lipo (++) | 50-100mg/day | 200 mg bid |
| Atenolol | Hydr (+++) | 50-100mg/day | 200 mg od |
| Carvedilol | Lipo (+++) | 12.5mg/day | 50 mg bid |

Although several beta blockers have been used to examine the effect on REE in cachexia associated with CHF, and a few have been used on patients with cancer, any beta blocker can be used to practice the combination therapy of the invention, and it is important to identify the best beta blocker and exemplary combination for each patient. Specific considerations as guidelines include:
Liver - If a patient has liver problems then it would be advisable to prescribe drugs that are secreted directly from the kidney, such as nadolol.
Kidney - Patients with kidney problems, (perhaps from chemotherapy or radiotherapy), would be better off with a compound metabolized by the liver such as propranolol, atenolol, etc.
Pulmonary - The cardioselective beta blockers are advised to patients who have pulmonary or respiratory complications. Agents such as acebutolol, atenolol, bisoprolol or metoprolol would be preferred for these patients. In addition, these patients must be advised of the potential for pulmonary constriction and doses will be lower than for patients without these complications.
Cardiac - Patients that have low blood pressure or low heart rate will have more safety concerns with cardioselective drugs and would probably do better with drugs that impact heart rate and blood pressure less significantly.

In practicing the compositions and methods of this invention, there are several NSAIDs that can be used alone (as a single NSAID) or in combination (more than one NSAID) with a beta blocker. Examples of non-selective NSAIDs that can be used in the compositions and methods of this invention, and selective cox-2 inhibitors that can be used in the compositions and methods of this invention, are listed in the following Tables:

### Non-selective (traditional) NSAIDS

| **Generic Name** | **Common Name** |
|---|---|
| Diclofenac | Voltaren, Cataflam |
| Diflunisal | Dolobid |
| Etodolac | Lodine |
| Flurbiprofen | Ansaid |
| Ibuprofen | Motrin, Advil |
| Indomethacin | Indocin |
| Ketoprofen | Orudis, Oruvail |
| Ketorolac | Toradol |
| Nabumetone | Relafen |
| Naproxen | Naprosyn, Alleve |
| Oxaprozin | Daypro |
| Piroxicam | Feldene |
| Salsalate | Disalcid |
| Sulindac | Clinoril |
| tolmetin | Tolectin |

### Cox-2 selective NSAIDs

| **Generic Name** | **Common Name** |
|---|---|
| celecoxib | Celebrex |
| rofecoxib | Vioxx |
| etoricoxib | Arcoxia |
| valdecoxib | Bextra |
| Meloxicam | Mobic |

Omega-3 (omega-3) fatty acids (FAs), e.g., eicosapentaenoic acid, or EPA, can be used in practicing the combination compositions and methods of this invention; EPA is a well-known agent for reducing an inflammatory response; it is a direct inhibitor of adipocyte lipid mobilization and has been used with varying success in the treatment of cachexia. Variability in success appears to be driven by ability to tolerate an effective dose and high rate of metabolism by the liver. EPA is generally administered orally and is in combination with DHA in fish oil in about a 2:1 ratio. Large doses of fish oil are known to cause unpleasant side effects and patients are often unable to tolerate a therapeutic dose. Oral EPA must be absorbed by the gut, which may be impaired in cachexia patients. Once EPA enters the blood stream it goes to the liver where it is cleared as much as 90% in the first pass, making adequate dosing problematic. Recent formulations of EPA may make dosing less of a problem.

The relatively recent results on the use of intravenous EPA (OMEGAVEN™) for the treatment of psoriasis are very promising and address a number of the pharmacokinetic and pharmacodynamic issues discussed above. Coupled with biomarkers of likely responding patients, Omegaven (currently approved only in Europe) could be a very attractive therapeutic agent in combination with beta blockers. Use of different formulations of EPA that allow adequate dosing in combination with beta blockers will provide effective the most effective therapy by impacting both the autonomic nervous system and the inflammatory signals.

One of the problems in treating cachexia patients, particularly patients with cancer cachexia, is the fear of unknown interaction between new cancer drugs and treatments for cachexia. While this is a problem administratively, it should not be a factor when treating patients. A key to addressing this issue is identifying patients that have cachexia (chronic SIRS) and who could benefit from early intervention. Systemic inflammation and REE are known predictors of advancing cancer and weight loss; however, additional parameters are important to provide non-invasive, relatively easy assays to indicate early cachexia and its onset.

The invention comprises use of cytokine and/or hormone levels as well as heart rate variability (over what would be considered normal for a particular individual) as key factors in the diagnostics of early cachexia.

### Cytokine and Hormone Levels

It is well established that elevated inflammatory cytokines are associated with cachexia. Whether these are a cause or effect is still unclear, but nevertheless they may be somewhat useful in aiding early diagnosis. The hormone that is particularly important in identifying the cachectic patient is epinephrine. Several studies have incidentally reported a significant elevation in epinephrine levels in cachexia patients. In addition, C-reactive protein, an acute phase response protein, is significantly elevated in cachexia patients. A diagnostic assay including IL-1, IL-6, TNFα, IFNγ, C-RP and epinephrine will be useful in the identification of potential cachexia patients.

### Heart Rate Variability

One component to cachexia is the imbalance of the autonomic nervous system. Analysis of heart rate variation (HRV) can serve as a noninvasive tool for assessing the activities of the autonomic nervous system (ANS). HRV analysis is based on the concept that fast fluctuations may reflect changes of sympathetic activity. The heart rate is analyzed using time domain parameters, frequency domain parameter and nonlinear parameters. Recently, the multifractal nature of HRV has been attributed to the critical balance between the antagonistic activity of the parasympathetic nervous system and the sympathetic nervous system.

Heart rate variation can be used to identify patients that have cachexia or are trending towards cachexia, as well as patients with more generally described chronic and acute SIRS. By first screening patients for HRV through a simple measurement of heart rate, then profiling the blood markers, it will be possible to identify patients at risk for cachexia, or in the early stages of cachexia, with a high degree of certainty.

### Exemplary Protocols for Diagnosing and Treating Cachexia Patients

In some aspects, the invention provides compositions that have the ideal profile of a drug to treat the patient with cachexia, e.g., a composition that is easy to take, such as a once a day pill or liquid formulation. In one aspect, the drug is effective but does not cause depression, tiredness, bradycardia or hypotension. The impact is to reduce the metabolic flux rate, balance the muscle and fat utilization, and to reduce inflammation.

The instant invention recognizes that simply supplying nutrients or providing signals to encourage increase of lean muscle mass is not beneficial until the high metabolic and energy flux through the muscle tissue is brought into normal range. Once metabolism has been normalized it is possible to use agents such as beta2 adrenergic agonists and anabolic hormones to start adding muscle mass. During the administration of beta blockers it is important to monitor blood pressure, heart rate and fatigue.

*Administration and Dosing*: Since patients with cachexia are often taking many other medications it is important that the therapy for cachexia not interfere with, and ideally should benefit, ongoing therapy.

*Frequency of dosing*: It is important to minimize the frequency and number of medications a patient must take. Patients who are required to take a medication only once or twice a day will have much higher compliance compared to a those who require medication of multiple pills three or more times a day. Formulation to a once a day dose is an advantage for all reformulations of the product.

*Dose Form*: In some aspects, unless there is a highly significant therapeutic reason, medication should be administered orally in pill or tablet form, or as a liquid that can be combined with a drink so taking the medication is not unpleasant. Since the patients with cachexia are frequently on a number of other medications it is important to make administration uncomplicated. In one aspect, the invention provides two pills in a single package, e.g., a single blister-pack, to assure that the both drugs are taken and at the correct time.

Many patients object to taking too many pills, so in one aspect, a product is provided as an oral liquid formulation that will be desired by many patients. In the situation where more than one drug needs to be administered both dugs can be provided in a single cartridge that is easily opened and which then allows both drugs to be administered at the same time.

### Antioxidant assays

### Oxygen Radical Absorbance Capacity (ORAC) values and assays

In one aspect, ingredients used to practice this invention (the multi-ingredient combinations of drugs of the invention), and methods and compositions of the invention used to treat, prevent and/or ameliorate wasting and/or atrophy, such as muscle atrophy, or methods and compositions of the invention used for protection against oxidants (e.g., used as an antioxidant), include any single known or groups of ingredients that have a measurable positive ORAC "Oxygen Radical Absorbance Capacity" (ORAC) value (or similar value using another comparable test) of at least about 1 ORAC unit/gram, or, at least about 5 ORAC units/gram, etc. as set forth, above.

ORAC assays are well known in the art, e.g., the Total Antioxidant Capacity Assay, or "Oxygen Radical Absorbance Capacity" (ORAC/NORAC), from Brunswick Laboratories, Wareham, MA; thus, the one of skill in the art would understand how to practice an ORAC assay and understand how to interpret a positive, or negative, ORAC assay in the context of this invention. The ORAC assay depends on the free radical damage to a fluorescent probe through the change in its fluorescence intensity. The change of fluorescence intensity is an index of the degree of free radical damage. In the presence of antioxidant, the inhibition of free radical damage by an antioxidant, which is reflected in the protection against the change of probe fluorescence in the ORAC assay, is a measure of its antioxidant capacity against the free radical. The ORAC assays can be performed *in vitro,* and if performed *in vitro,* do not determine the bioavailability within the body. A high ORAC value indicates that the tested sample possesses a high potency of antioxidant activity chemically.

With the exception of the fluorescent probe, the "improved" ORAC assay of Brunswick Laboratories, Wareham, MA, used in one aspect of this invention is exactly the same as the "original" ORAC assay in terms of experimental conditions, AUC technique and chemical principles. In one aspect, the "original" assay's fluorescent probe, beta-phycoerythrin (PE) is replaced with fluorescein (FL)4. FL as compared to PE does not interact with antioxidants, shows excellent photostability and reduces the cost of experiments. The ORAC value obtained by this "improved" ORAC is higher than that obtained by the original ORAC assay. The reason for this is due to the fact that in the "improved" ORAC reaction, the fluorescent probe is exclusively damaged by the peroxyl radical, and therefore the new assay measures the antioxidant capacity more accurately. See also Cao (1995) "Automated Assay of Oxygen Radical Absorbance Capacity with the COBAS FARA II." Clinical Chemistry 41(12):1738-1745; Bank (2002) "Oxygen Radical Absorbance Capacity, Standardizing the Way We Look at Antioxidants." Nutraceuticals World September 2002; 42-45; Cao (1993) "Oxygen Radical Absorbency Capacity Assay for Antioxidants." Free Radical Biol. Med. 14(3):303-311.

### TEAC assay

In one aspect, ingredients used to practice this invention (the multi-ingredient combinations of drugs of the invention), and methods and compositions of the invention used to treat, prevent and/or ameliorate wasting and/or atrophy, such as muscle atrophy, or methods and compositions of the invention used for protection against oxidants (e.g., used as an antioxidant), include any single known or groups of ingredients that measure positive as antioxidants on the Trolox Equivalent Antioxidant Capacity (TEAC) assay.

The TEAC assay was developed by Miller et al., 1993, and uses the COBAS BIO™ spectrophotometric analyzer (Hoffmann-La Roche Inc., Nutley,. NJ). This assay is based on the observation that when 2,2'-azinobis-(3-ethylbenzothiazoline-6-sulphonic acid) (ABTS) is incubated in the presence of a peroxidase and hydrogen peroxide or in the presence of hydroxyl, peroxyl, alkoxyl, and inorganic radicals, the slightly more stable ABTS⁺ radical cation is generated. From the time the ABTS, metmyoglobin, buffer, and hydrogen peroxide are added together, the absorbance of infra red radiation is measured at a wavelength of 734nm with respect to time. As the ABTS⁺ radical cation begins to form, the absorbance increases. When antioxidants are added before the addition of hydrogen peroxide, the antioxidants scavenge the radicals formed by the hydrogen peroxide, delaying the formation of the ABTS⁺ radical cation, thus inducing an increase in the percentage of inhibition of the absorbance. The unit of measure in this assay is the TEAC, which is the concentration (mmol/l) of Trolox with the equivalent antioxidant capacity to a 1.0 mmol/l solution of the substance being tested.

The TEAC assay can detect the antioxidant capacity of water-soluble drugs or drugs that can be solubilized. Also, due to the ability of the TEAC assay to detect the contribution of other components, the TEAC assay can be used to measure the antioxidant capacity of the system. The TEAC method can be used for pharmacological and nutritional studies.

### TRAP Assay

In one aspect, ingredients used to practice this invention (the multi-ingredient combinations of drugs of the invention), and methods and compositions of the invention used to treat, prevent and/or ameliorate wasting and/or atrophy, such as muscle atrophy, or methods and compositions of the invention used for protection against oxidants (e.g., used as an antioxidant), include any single known or groups of ingredients that measure positive as antioxidants on the Total Radical-Trapping Antioxidant Parameter, TRAP, method.

Aqueous dispersions of oxidizable organic compounds that can be readily and reproducibly initiated at a constant rate, Ri, by peroxidation using the water-soluble azo compound 2,2'-azo-bis-(2-amidipropropane hydrochloride), ABAP, is the basis of the Total Radical-Trapping Antioxidant Parameter, TRAP, method. The TRAP method was developed by Wayner et al. and published in 1985. Upon addition of the ABAP to the plasma, the length of time that oxygen uptake by peroxidizable plasma is inhibited is measured with an oxygen probe, and this value is referred to as the TRAP. Trolox is used in this method by creating a second induction period after the natural antioxidants have been depleted. This second induction period is used to calculate an Ri value, which is used to calculate the TRAP value. The TRAP value is reported as the number of moles of peroxyl radicals trapped per liter of fluid.

The duration of time is the only thing measured in the TRAP method, which is a limiting factor in its analysis. The time taken to prevent maximum oxygen uptake cannot be measured easily and precisely, and the total radical trapping capability per mole of some antioxidants is dependent on their initial concentration. This leaves out the extent of inhibition. The TRAP method shows the contribution of the serum components to the total radical-trapping antioxidant content, which is a problem if the antioxidant contribution of a specific chemical is the desired information. The TRAP method is more specific than the TEAC method due to the involvement of a number of different radical species in the TEAC method as opposed to just the chain-carrying peroxyl species in the TRAP method. The TRAP method requires high levels of dilution of plasma to produce the required lag phase, and the process to accomplish this shortens the lipid chain length necessary for a rapid chain reaction.

### Packaging

The invention provides compositions, including preparations, formulations and/or kits, comprising combinations of ingredients, as described above (including the multi-ingredient combinations of drugs of the invention), that are serviceable as therapies for treating, preventing or improving conditions, states and disease symptoms involving inflammation, excessive sympathoneural drive, cachexia, anorexia, and anorexia-cachexia, and stress or anxiety related thereto. In one aspect, each member of the combination of ingredients is manufactured in a separate package, kit or container; or, all or a subset of the combinations of ingredients are manufactured in a separate package or container. In alternative aspects, the package, kit or container comprises a blister package, a clamshell, a tray, a shrink wrap and the like.

In one aspect, the package, kit or container comprises a "blister package" (also called a blister pack, or bubble pack). In one aspect, the blister package is made up of two separate elements: a transparent plastic cavity shaped to the product and its blister board backing. These two elements are then joined together with a heat sealing process which allows the product to be hung or displayed. Exemplary types of "blister packages" include: Face seal blister packages, gang run blister packages, mock blister packages, interactive blister packages, slide blister packages.

Blister packs, clamshells or trays are forms of packaging used for goods; thus, the invention provides for blister packs, clamshells or trays comprising a composition (e.g., a (the multi-ingredient combination of drugs of the invention) combination of active ingredients) of the invention. Blister packs, clamshells or trays can be designed to be non-reclosable, so consumers can tell if a package has already opened. They are used to package for sale goods where product tampering is a consideration, such as the pharmaceuticals of the invention. In one aspect, a blister pack of the invention comprises a moulded PVC base, with raised areas (the "blisters") to contain the tablets, pills, etc. comprising the combinations of the invention, covered by a foil laminate. Tablets, pills, etc. are removed from the pack either by peeling the foil back or by pushing the blister to force the tablet to break the foil. In one aspect, a specialized form of a blister pack is a strip pack. In one aspect, in the United Kingdom, blister packs adhere to British Standard 8404.

In one aspect, a blister packs also comprise a method of packaging where the compositions comprising combinations of ingredients of the invention are contained inbetween a card and a clear PVC. The PVC can be transparent so the item (pill, tablet, geltab, etc.) can be seen and examined easily; and in one aspect, can be vacuum-formed around a mould so it can contain the item snugly and have room to be opened upon purchase. In one aspect, the card is brightly coloured and designed depending on the item (pill, tablet, geltab, etc.) inside, and the PVC is affixed to the card using pre-formed tabs where the adhesive is placed. The adhesive can be stong enough so that the pack may hang on a peg, but weak enough so that this way one can tear open the join and access the item. Sometimes with large items or multiple enclosed pills, tablets, geltabs, etc., the card has a perforated window for access. In one aspect, more secure blister packs, e.g., for items such as pills, tablets, geltabs, etc. of the invention are used, and they can comprise of two vacuum-formed PVC sheets meshed together at the edges, with the informative card inside. These can be hard to open by hand, so a pair of scissors or a sharp knife may be required to open.

In one aspect, blister packaging comprises at least two components (e.g., is a multi-ingredient combination of drugs of the invention): a thermoformed "blister" which houses the product (e.g., a pharmaceutical combination of the invention), and then a "blister card" that is a printed card with an adhesive coating on the front surface. During the assembly process, the blister component, which is most commonly made out of PVC, is attached to the blister card using a blister machine. This machine introduces heat to the flange area of the blister which activates the glue on the card in that specific area and ultimately secures the PVG blister to the printed blister card. The thermoformed PVG blister and the printed blister card can be as small or as large as you would like, but there are limitations and cost considerations in going to an oversized blister card. Conventional blister packs can also be sealed (e.g., using an AERGO 8 DUO™, SCA Consumer Packaging, Inc., DeKalb IL) using regular heat seal tooling. This alternative aspect, using heat seal tooling, can seal common types of thermoformed packaging.

For example, in one aspect, the invention provides a blister package, a clamshell, a tray or a shrink wrap comprising at least one beta adrenergic receptor antagonist (a beta blocker) and an angiotensin-converting enzyme (ACE) inhibitor, an angiotensin receptor blocker (ARB), a non-steroidal anti-inflammatory drug (a NSAID), an anabolic steroid, a natural oil, a fatty acid or a combination thereof. In one aspect, the therapeutic combination in the blister package, clamshell, tray or shrink wrap comprises at least one beta adrenergic receptor antagonist (a beta blocker) and at least one non-steroidal anti-inflammatory drug (a NSAID), such as an aspirin, dichlofenac, diflunisal, etodolac, fenoprofen, flurbiprofen, ibuprofen, indomethacin, ketoprofen; ketorolac, meclofenamate, mefenamic acid, meloxicam, nabumetone, naproxen, oxaprozin, piroxicam, salsalate, sulindac, tolmetin, a COX-2 inhibitor (e.g., a COX-2-selective inhibitor) inhibitor or a combination thereof. In one aspect, the therapeutic combination in the blister package, clamshell, tray or shrink wrap comprises at least one beta adrenergic receptor antagonist (a beta blocker) and a COX-2 inhibitor (e.g., a COX-2-selective inhibitor), such as celecoxib rofecoxib, etoricoxib, valdecoxib, parecoxib, meloxicam and/or lumiracoxib. In one aspect, the therapeutic combination in the blister package, clamshell, tray or shrink wrap comprises an atenolol, nadolol, metoprolol, propranolol, carteolol, carvedolol, labetalol, oxprenolol, penbutolol, pindolol, sotalol, timolol or a combination thereof, and at least one non-steroidal anti-inflammatory drug (a NSAID).

The following examples are offered to illustrate, but not to limit the claimed invention.

### EXAMPLES

### Example 1: Exemplary treatment for non-lung, non-hematological metastatic cancer

The following example describes an exemplary treatment protocol of the invention, using an exemplary composition of the invention.

Population: Patients with advanced cancer, Stage III or IV, with evidence of weight loss. Exclude lung cancer patients with pulmonary function disease and patients with hematological cancer.

Drug classes: Beta Adrenergic Antagonist (beta blocker) and Non-steroidal anti-inflammatory (NSAID).

Specific Drugs (this combination designated "VB 122"): Propranolol (e.g., INDERAL™), a non selective antagonist that inhibits beta1, beta2 and beta3 subclasses of beta adrenergic receptors (the drug is contraindicated for patients with bronchospastic disease, e.g., chronic bronchitis, emphysema, asthma); and, etodolac (e.g., LODINE™), an NSAID that inhibits both Cox-1 and Cox-2 enzymes with some preference towards Cox-2 causing less gastrointestinal problems.

Dosing Route: multiple including intravenous, topical, and oral by inhalation, infusion or injection, (e.g., intraperitoneal, intramuscular, subcutaneous, intra-aural, intra-articular, intra-mammary, etc.), topical application (e.g., on areas, such as eyes, ears, skin or on afflictions such as wounds, burns, etc.), and by absorption through epithelial or mucocutaneous linings (e.g. vaginal and other epithelial linings, gastrointestinal mucosa, etc.).

Dosing: Dose for propranolol is individualized. Doses include from 10 to 320 mg per day based on heart rate and blood pressure Since the effect of propranolol and etodolac are opposite on blood pressure it is very important that patient compliance be maintained for safety. Therefore, in one aspect, the drugs are supplied in a single package or a plurality of packages, e.g., a blister pack, with one of each pill released upon opening. An exemplary protocol comprising an individualized course of dose escalation is:
- Start: AM, 10 mg: propranolol, 400 mg etodolac
- PM, 10 mg: propranolol, 400 mg etodolac
- Dose Escalation 1:: AM, 20 mg propranolol, 400 mg etodolac PM, 20 mg propranolol, 400 mg etodolac
- Dose Escalation 2:: AM, 40 mg propranolol, 400 mg etodolac PM, 40 mg propranolol, 400 mg etodolac
- Dose Escalation 3:: AM, 80 mg propranolol, 400 mg etodolac PM, 80 mg propranolol, 400 mg etodolac
- Dose Escalation 4:: AM, 160 mg propranolol, 400 mg etodolac PM, 160 mg propranolol, 400 mg etodolac

Dose is increased to obtain a heart rate of approximately 60 bpm with blood pressure maintained above 90/60.

In one aspect, chrono-dosing to match the optimal dose for the time of day is used. In this indication, a high dose of propranolol in the morning will provide the best dose for typical high morning blood pressure and to provide a good bolus dose to start the day. Afternoon dosing is lower and evening dose is lower again, or the same as afternoon. Propranolol has been linked to unusual and vivid dreams so a low dose in the evening is preferred. Etodolac is provided at a modest dose twice during the day and is increased in the evening to provide additional pain management for good rest in the night.

An exemplary protocol comprising an individualized chrono-dosing is:
- Start:: AM, 20 mg propranolol, 200mg etodolac; afternoon, 10 mg propranolol, 200 mg etodolac; PM 10 mg propranolol, 400 mg etodolac.

- Dose Escalation 1:: AM 40 mg propranolol, 200 mg etodolac; afternoon 20 mg propranolol, 200 mg etodolac; evening, 20 mg propranolol, 400 mg etodolac
- Dose escalation 2:: AM 80 mg propranolol, 200 mg etodolac; afternoon 40 mg propranolol, 200 mg etodolac, evening 40 mg, etodolac.

In one aspect, this release profile, or a substantially similar profile, is obtained by reformulating the active ingredient such that one or two pills are taken per day.

Another exemplary protocol is:
- Start:: AM, 20 mg propranolol, 200mg etodolac; afternoon, 10 mg propranolol, 200 mg etodolac; PM 5 mg propranolol, 400 mg etodolac.
- Dose Escalation 1:: AM 40 mg propranolol, 200 mg etodolac; afternoon 20 mg propranolol, 200 mg etodolac; evening, 10 mg propranolol, 400 mg etodolac
- Dose escalation 2:: AM 80 mg propranolol, 200 mg etodolac; afternoon 40 mg propranolol, 200 mg etodolac, evening 20 mg, etodolac.

In one aspect, this release profile, or a substantially similar profile, is obtained by reformulating the active ingredient such that one or two pills are taken per day.

### Example 2: An exemplary non-hematological metastatic cancer treatment

The following example describes an exemplary treatment protocol of the invention, using an exemplary composition of the invention.

Population: Patients with advanced cancer, Stage III or IV, with evidence of weight loss. Exclude patients with hematological cancer.

Drug classes: Beta Adrenergic Antagonist (beta blocker) and Non-steroidal anti-inflammatory (NSAID).

Specific Drugs (this combination designated "VB-132"): Atenolol (e.g., TENORMIN™), selective antagonist that inhibits beta1 subclass of receptors over beta2 and beta3 receptors; the drug is useful for patients with bronchospastic disease, e.g., chronic bronchitis, emphysema, asthma (atenolol is eliminated primarily by the kidneys through the urine; patients with impaired renal function (creatinine clearance 10-50) may be better to use the VB-142 combination, see below); and,

Etodolac (e.g., LODINE™), an NSAID that inhibits both Cox-1 and Cox-2 enzymes with some preference towards Cox-2 causing less gastrointestinal problems.

Dosing Route: multiple including intravenous, topical, and oral by inhalation, infusion or injection, (e.g., intraperitoneal, intramuscular, subcutaneous, intra-aural, intra-articular, intra-mammary, etc.), topical application (e.g., on areas, such as eyes, ears, skin or on afflictions such as wounds, bums, etc.), and by absorption through epithelial or mucocutaneous linings (e.g. vaginal and other epithelial linings, gastrointestinal mucosa, etc.).

Dosing: Dose for atenolol is individualized. Doses include from 12.5 to 100 mg per day based on heart rate and blood pressure Since the effect of atenolol and etodolac are opposite on blood pressure it is very important that patient compliance be maintained for safety. Therefore, the drugs may be supplied in a blister pack with one of each pill released upon opening.

An exemplary protocol comprising an individualized course of dose escalation is:
- Start: AM, 12.5 mg: atenolol, 400 mg etodolac
- PM, 12.5 mg: atenolol, 400 mg etodolac
- Dose Escalation 1:: AM, 25 mg atenolol, 400 mg etodolac PM, 25 mg atenolol, 400 mg etodolac
- Dose Escalation 2:: AM, 25 mg atenolol, 400 mg etodolac PM, 50 mg atenolol, 400 mg etodolac
- Dose Escalation 3:: AM, 50 mg atenolol, 400 mg etodolac PM, 50 mg atenolol, 400 mg etodolac

Dose is increased to obtain a heart rate of approximately 60 bpm with blood pressure maintained above 90/60.

In one aspect, chrono-dosing to match the optimal dose for the time of day is used. A high dose of atenolol in the morning will provide the best dose for typical high morning blood pressure and to provide a good bolus dose to start the day. Afternoon dosing is lower and evening dose is lower again, or the same as afternoon. Etodolac is provided at a modest dose twice during the day and is increased in the evening to provide additional pain management for good rest in the night.

An exemplary protocol comprising individualized chrono-dosing is:
- Start:: AM, 12.5 mg atenolol, 200mg etodolac; afternoon, 12.5 mg atenolol, 200 mg etodolac; PM 12.5 mg atenolol, 400 mg etodolac.
- Dose Escalation 1:: AM 25 mg atenolol, 200 mg etodolac; afternoon 12.5 mg atenolol, 200 mg etodolac; evening, 20 mg atenolol, 400 mg etodolac
- Dose escalation 2:: AM 50 mg atenolol, 200 mg etodolac; afternoon 25 mg atenolol, 200 mg etodolac, evening 25 mg atenolol, 400 mg etodolac.

In one aspect, this release profile, or a substantially similar profile, is obtained by reformulating the active ingredient such that one or two pills are taken per day.

Another exemplary protocol is:
- Start:: AM, 12.5 mg atenolol, 200mg etodolac; afternoon, 12.5 mg atenolol, 200 mg etodolac; PM 6.25 mg atenolol, 400 mg etodolac.

- Dose Escalation 1:: AM 25 mg atenolol, 200 mg etodolac; afternoon 12.5 mg atenolol, 200 mg etodolac; evening, 6.25mg atenolol, 400 mg etodolac
- Dose escalation 2:: AM 50 mg atenolol, 200 mg etodolac; afternoon 25 mg atenolol, 200 mg etodolac, evening 12.5 mg, etodolac.
- Dose escalation 3:: AM 50 mg atenolol, 200 mg etodolac; afternoon 25 mg atenolol, 200 mg etodolac, evening 25 mg, etodolac.

In one aspect, this release profile, or a substantially similar profile, is obtained by reformulating the active ingredient such that one or two pills are taken per day.

### Example 3: An exemplary treatment for non-hematological metastatic cancer

The following example describes an exemplary treatment protocol of the invention, using an exemplary composition of the invention.

Population: Patients with advanced cancer, Stage III or IV, with evidence of weight loss. Exclude patients with hematological cancer.

Drug classes: Beta Adrenergic Antagonist (beta blocker) and Non-steroidal anti-inflammatory (NSAID).

Specific Drugs (this combination designated "VB-142"): Metoprolol (e.g., TOPROL-XL™, metoprolol succinate), is a selective antagonist that inhibits beta1 subclass of receptors over beta2 and beta3 receptors (the drug is useful for patients with bronchospastic disease, e.g., chronic bronchitis, emphysema, asthma); and, metoprolol. Metoprolol is extensively metabolized so patients with impaired renal function may be put on this rather than the VB-132 combination (see above).

Etodolac (e.g., LODINE™), an NSAID that inhibits both Cox-1 and Cox-2 enzymes with some preference towards Cox-2 causing less gastrointestinal problems.

Dosing Route: multiple including intravenous, topical, and oral by inhalation, infusion or injection, (e.g., intraperitoneal, intramuscular, subcutaneous, intra-aural, intra-articular, intra-mammary, etc.), topical application (e.g., on areas, such as eyes, ears, skin or on afflictions such as wounds, burns, etc.), and by absorption through epithelial or mucocutaneous linings (e.g. vaginal and other epithelial linings, gastrointestinal mucosa, etc.).

Dosing: Dose for metoprolol is individualized. Doses include from 12.5 to 100 mg per day based on heart rate and blood pressure Since the effect of metoprolol and etodolac are opposite on blood pressure it is very important that patient compliance be maintained for safety. Therefore, the drugs may be supplied in a blister pack with one of each pill released upon opening.

An exemplary protocol, including a simple individualized course of dose escalation, comprises:
- Start: AM, 12.5 mg: metoprolol, 400 mg etodolac
- PM, 12.5 mg: metoprolol, 400 mg etodolac
- Dose Escalation 1:: AM, 25 mg metoprolol, 400 mg etodolac PM, 25 mg metoprolol, 400 mg etodolac
- Dose Escalation 2:: AM, 25 mg metoprolol, 400 mg etodolac PM, 50 mg metoprolol, 400 mg etodolac
- Dose Escalation 3:: AM, 50 mg metoprolol, 400 mg etodolac PM, 50 mg metoprolol, 400 mg etodolac

Dose is increased to obtain a heart rate of approximately 60 bpm with blood pressure maintained above 90/60.

In one aspect, chrono-dosing to match the optimal dose for the time of day is used. In this case, a high dose of metoprolol in the morning will provide the best dose for typical high morning blood pressure and to provide a good bolus dose to start the day. Afternoon dosing is lower and evening dose is lower again, or the same as afternoon. Etodolac is provided at a modest dose twice during the day and is increased in the evening to provide additional pain management for good rest in the night.

An exemplary protocol of individualized chrono-dosing is:
- Start:: AM, 12.5 mg metoprolol, 200mg etodolac; afternoon, 12.5 mg metoprolol, 200 mg etodolac; PM 12.5 mg metoprolol, 400 mg etodolac.
- Dose Escalation 1:: AM 25 mg metoprolol, 200 mg etodolac; afternoon 12.5 mg metoprolol, 200 mg etodolac; evening, 20 mg metoprolol, 400 mg etodolac
- Dose escalation 2:: AM 50 mg metoprolol, 200 mg etodolac; afternoon 25 mg metoprolol, 200 mg etodolac, evening 25 mg metoprolol, 400 mg etodolac.

In one aspect, this release profile, or a substantially similar profile, is obtained by reformulating the active ingredient such that one or two pills are taken per day.

Another exemplary protocol is:
- Start:: AM, 12.5 mg metoprolol, 200mg etodolac; afternoon, 12.5 mg metoprolol, 200 mg etodolac; PM 6.25 mg metoprolol, 400 mg etodolac.
- Dose Escalation 1:: AM 25 mg metoprolol, 200 mg etodolac; afternoon 12.5 mg metoprolol, 200 mg etodolac; evening, 6.25mg metoprolol, 400 mg etodolac
- Dose escalation 2:: AM 50 mg metoprolol, 200 mg etodolac; afternoon 25 mg metoprolol, 200 mg etodolac, evening 12.5 mg, etodolac.

- Dose escalation 3:: AM 50 mg metoprolol, 200 mg etodolac; afternoon 25 mg metoprolol, 200 mg etodolac, evening 25 mg, etodolac.

In one aspect, this release profile, or a substantially similar profile, is obtained by reformulating the active ingredient such that one or two pills are taken per day.

### Example 4: Exemplary Beta Blockers and Combinations of beta blockers

The following example describes exemplary treatment protocols of the invention, using exemplary therapeutic combination compositions of the invention.

### Beta Blockers and Combinations of beta blockers

In one aspect, a beta blocker and an NSAID are used in combination to treat cachexia. The beta blocker antagonizes the beta receptors resulting in normalization of energy metabolism as well as lipid and protein metabolism. The NSAID provides additional anti-inflammatory capacity to work in synergy with the beta blocker reducing inflammation and has offsetting activity blood pressure to attenuate any drop in blood pressure that may result from the beta blocker therapy. The use of beta blocker alone is inadequate because it primarily affects the autonomic system. The NSAID alone has its primary impact on the inflammatory system and needs the beta blocker to attenuate the adrenergic dysfunction. The dose of the NSAID is fixed and the dose of the beta blocker is increased periodically at approximately intervals:
a) In one aspect, the beta blocker is non-selective and the NSAID is non-selective
b) In one aspect, the beta blocker is non-selective and the NSAID is cox2 selective
c) In one aspect, the beta blocker is cardio-selective (beta1 selective) and the NSAID is non-selective
d) In one aspect, the beta blocker is cardio-selective (beta1 selective) and the NSAID is cox2 selective

In one aspect, a beta blocker and an NSAID are used in combination to treat cachexia. In one aspect, the dose of the NSAID is fixed and the dose of the beta blocker is increased periodically at approximately weekly intervals. Since the patient with cachexia is often overwhelmed with medications and may have difficulty swallowing pills and keeping track of medication dose and times the medication is provided in a convenient format for administration:
a) Any beta blocker and NSAID combination, e.g., as described herein.
b) In one aspect, the pills are provided in a blister pack in which the appropriate dose of each drug can be dispensed together.
c) The medications are provided in liquid formulation in separate compartments that are released by removing a single cap.
d) In one aspect, the medication is provided in liquid or solid form in a multi-dose blister pack where the dose of the beta blocker is available at increasing levels to provide the doses needed to reach target dose. The packages are also provided with a fixed dose beta blocker and NSAID for administration once the best dose for the patient is identified using the multi-dose pack.
e) In one aspect, the medications are provided at ½ and 80% of standard available doses of beta blocker to provide a broader range and reduced level of beta blockers for the cachexia patients that have reduced body mass.
f) In one aspect, the medication is provided in an extended release solid formulation such that medication must be taken only once daily.
g) In one aspect, the medication is provided as an extended release liquid formulation such that medication must be taken only once daily.
h) Since beta blockers may cause lower blood pressure and reduced energy some patients will benefit from having a lower dose in the day and can have a higher dose in the evening. In one aspect, a dose pack is provided that provides a constant level of NSAID in two daily doses whereas the beta blocker is provided at a lower dose for the morning administration and a higher dose for the evening administration.

In one aspect, a beta blocker and eicosapentaenoic acid (EPA) are used in combination to treat cachexia. The beta blocker antagonizes the beta receptors resulting in normalization of energy metabolism as well as lipid and protein metabolism. The EPA provides additional anti-inflammatory capacity to work in synergy with the beta blocker reducing inflammation and has offsetting activity blood pressure to attenuate any drop in blood pressure that may result from the beta blocker therapy. In one aspect, the use of beta blocker alone is inadequate because it primarily affects the autonomic system. The EPA alone has its primary impact on the inflammatory system and needs the beta blocker to attenuate the adrenergic dysfunction. In one aspect, the dose of the EPA is fixed and the dose of the beta blocker is increased periodically at approximately intervals:
a) In one aspect, the beta blocker is non-selective and the EPA is as a capsule
b) In one aspect, the beta blocker is non-selective and the EPA is administered as an intravenous infusion.
c) In one aspect, the beta blocker is cardio-selective (beta1 selective) and the EPA a capsule.
d) In one aspect, the beta-blocker is cardioselective (beta1 selective) and the EPA is administered as an intravenous infusion.

In one aspect, a selective beta1 blocker and a COX 1/2 inhibitor are used in combination to treat cachexia. The beta blocker antagonizes the beta receptors in the heart resulting in reduced energy expenditure, normalization of energy metabolism as well as lipid and protein metabolism. The NSAID provides additional anti-inflammatory capacity to work in synergy with the beta blocker reducing inflammation and has offsetting activity blood pressure to attenuate any drop in blood pressure that may result from the beta blocker therapy. The use of beta blocker alone is inadequate because it primarily affects the autonomic system. The NSAID alone has its primary impact on the inflammatory system and needs the beta blocker to attenuate the adrenergic dysfunction. In one aspect, the dose of the NSAID is fixed and the dose of the beta blocker is increased periodically at approximately intervals to obtain a desired reduction in heart rate.
a) In one aspect, any beta blocker that is not considered beta1 selective is combined with any NSAID that is not considered a cox-2 inhibitor.
b) In one aspect, the pills are provided in one package, e.g., a blister pack, in which the appropriate dose of each drug can be dispensed together.
c) In one aspect, the medications are provided in liquid formulation in separate compartments that are released by removing a single cap.
d) In one aspect, the medication is provided in liquid or solid form in a multi-dose blister pack where the dose of the beta blocker is available at increasing levels to provide the doses needed to reach target dose. The packages are also provided with a fixed dose beta blocker and NSAID for administration once the best dose for the patient is identified using the multi-dose pack.
e) In one aspect, the medications are provided at ½ and 80% of standard available doses of beta blocker to provide a broader range and reduced level of beta blockers for the cachexia patients that have reduced body mass.
f) In one aspect, the medication is provided in an extended release solid formulation such that medication must be taken only once daily
g) In one aspect, the medication is provided as an extended release liquid formulation such that medication must be taken only once daily.
h) Since beta blockers may cause lower blood pressure and reduced energy some patients will benefit from having a lower dose in the day and can have a higher dose in the evening. In one aspect, a dose pack is provided that provides a constant level of NSAID in two daily doses whereas the beta blocker is provided at a lower dose for the morning administration and a higher dose for the evening administration.

In one aspect, a non-selective beta blocker and a COX 2 inhibitor are used in combination to treat cachexia. The beta blocker antagonizes the beta receptors resulting in normalization of energy metabolism as well as lipid and protein metabolism. The NSAID provides additional anti-inflammatory capacity to work in synergy with the beta blocker reducing inflammation and has offsetting activity blood pressure to attenuate any drop in blood pressure that may result from the beta blocker therapy. The use of beta blocker alone is inadequate because it primarily affects the autonomic system. The NSAID alone has its primary impact on the inflammatory system and needs the beta blocker to attenuate the adrenergic dysfunction. In one aspect, the dose of the NSAID is fixed and the dose of the beta blocker is increased periodically at approximately intervals
a) In one aspect, any beta blocker that is not considered beta1 selective is combined with any NSAID that is not considered a cox-2 inhibitor.
b) The medication is provided in any of the formats described herein.

In one aspect, a selective beta1 blocker and a COX 2 inhibitor are used in combination to treat cachexia. The beta blocker antagonizes the beta receptors resulting in normalization of energy metabolism as well as lipid and protein metabolism. The NSAID provides additional anti-inflammatory capacity to work in synergy with the beta blocker reducing inflammation and has offsetting activity blood pressure to attenuate any drop in blood pressure that may result from the beta blocker therapy. The use of beta blocker alone is inadequate because it primarily affects the autonomic system. In one aspect, the NSAID alone has its primary impact on the inflammatory system and needs the beta blocker to attenuate the adrenergic dysfunction. In one aspect, the dose of the NSAID is fixed and the dose of the beta blocker is increased periodically at approximately intervals:
a) In one aspect, any beta blocker that is not considered beta1 selective is combined with any NSAID that is not considered a cox-2 inhibitor.
b) In one aspect, the medication is provided in any of the formats described herein.

In one aspect, the nonselective beta blocker propranolol and the non selective NSAID ibuprofen are used in combination to treat cachexia. The beta blocker antagonizes the beta receptors resulting in normalization of energy metabolism as well as lipid and protein metabolism. The NSAID provides additional anti-inflammatory capacity to work in synergy with the beta blocker reducing inflammation and has offsetting activity blood pressure to attenuate any drop in blood pressure that may result from the beta blocker therapy. The use of beta blocker alone is inadequate because it primarily affects the autonomic system. The NSAID alone has its primary impact on the inflammatory system and needs the beta blocker to attenuate the adrenergic dysfunction. In one aspect, the dose of the NSAID is fixed and the dose of the beta blocker is increased periodically at approximately intervals:
a) In one aspect, the propranolol and ibuprofen are provided in any of the formats described herein.

In one aspect, the selective beta1 blocker atenolol and the nonselective NSAID ibuprofen are used in combination to treat cachexia. The beta blocker antagonizes the beta receptors resulting in normalization of energy metabolism as well as lipid and protein metabolism. The NSAID provides additional anti-inflammatory capacity to work in synergy with the beta blocker reducing inflammation and has offsetting activity blood pressure to attenuate any drop in blood pressure that may result from the beta blocker therapy. The use of beta blocker alone is inadequate because it primarily affects the autonomic system. The NSAID alone has its primary impact on the inflammatory system and needs the beta blocker to attenuate the adrenergic dysfunction. In one aspect, the dose of the NSAID is fixed and the dose of the beta blocker is increased periodically at approximately intervals
a) In one aspect, the atenolol and ibuprofen are provided in any of the formats described herein.

In one aspect, the nonselective beta blocker propranolol and the COX 2 preferential NSAID etodolac are used in combination to treat cachexia. The beta blocker antagonizes the beta receptors resulting in normalization of energy metabolism as well as lipid and protein metabolism. The NSAID provides additional anti-inflammatory capacity to work in synergy with the beta blocker reducing inflammation and has offsetting activity blood pressure to attenuate any drop in blood pressure that may result from the beta blocker therapy. The use of beta blocker alone is inadequate because it primarily affects the autonomic system. The NSAID alone has its primary impact on the inflammatory system and needs the beta blocker to attenuate the adrenergic dysfunction. In one aspect, the dose of the NSAID is fixed and the dose of the beta blocker is increased periodically at approximately intervals
a) In one aspect, the propranolol and etodolac are provided in any of the formats described herein.
b) In one aspect, the beta blocker propranolol is provided at a starting dosage of 40 mg and etodolac at 500 mg twice daily. The patient receives both medications at the same time. In one aspect, the dose of the propranolol is increased weekly to obtain a reduction in heart rate of 20% as long as the blood pressure does not go below 90/60.

In one aspect, the selective beta1 blocker atenolol and cox-2 preferential NSAID etodolac are used in combination to treat cachexia. The beta blocker antagonizes the beta receptors resulting in normalization of energy metabolism as well as lipid and protein metabolism. The NSAID provides additional anti-inflammatory capacity to work in synergy with the beta blocker reducing inflammation and has offsetting activity blood pressure to attenuate any drop in blood pressure that may result from the beta blocker therapy. The use of beta blocker alone is inadequate because it primarily affects the autonomic system. The NSAID alone has its primary impact on the inflammatory system and needs the beta blocker to attenuate the adrenergic dysfunction. In one aspect, the dose of the NSAID is fixed and the dose of the beta blocker is increased periodically at approximately intervals
a) In one aspect, the atenolol and etodolac are provided in any of the formats described herein.
b) In one aspect, the atenolol is provided at a starting dosage of 25mg once per day. The NSAID etodolac is provided at 500 mg once daily. The patient receives both medications at the same time. In one aspect, the dose of the atenolol is increased weekly to obtain a reduction in heart rate of 20% as long as the blood pressure does not go below 90/60. In one aspect, a second dose of etodolac is taken after 12 hours.

The invention provides a therapeutic combination, e.g., a pharmaceutical composition, comprising a drug comprising a beta1 blocker that is useful in the treatment of patients with pulmonary stress (e.g., asthma, COPD). The use of a beta blocker in a patient with asthma or COPD or respiratory impairment is generally contraindicated. Thus, in one aspect, the beta1 selective blockers, such as atenolol, acebuterol, bisoprolol or metoprolol may be used more safely in cachexia patients with pulmonary complications.

The cardioselective beta blockers, including atenolol, acebutolol, bisoprolol, or metoprolol, are used in this embodiment of a composition of the invention. In this aspect, the drugs are prescribed initially at ½ the lowest recommended dose and the dose is gradually increased.
a) In one aspect, the medication is provided in any of the formats described herein.
b) In one aspect, a dose-pack is provided of atenolol at increasing dose levels sufficient to obtain an optimal dose. The pack contains a seven day supply of the medication at each set dose so the patient's therapeutic dose can be obtained. The pack contains 7 doses of 10 mg of atenolol, 7 doses of 20mg atenolol, 7 doses of 40 mg atenolol , 7 doses of 70 mg atenolol and 7 doses of 100 mg atenolol. The doses are selected to provide a lower minimal dose than is now available and provides additional incremental dosage increases than is available. After taking one dose for 5-7 days the patient is recommended to increase to the next dose upon monitoring by the physician. The dose can be increased if there is no adverse effect on the respiratory system and if the blood pressure is not below 90/60 and the heart rate is not 60 bpm or lower.
c) In one aspect, the invention provides a long acting cardioselective beta blocker that needs to be taken once per day. The beta blocker bisoprolol has a half-life of 10-12 hours and can be taken once per day. By taking the dose in the evening the highest concentration is available at night when there is less demand on the respiratory system. In one aspect, the drug is provided at 2mg, 4 mg and 10 mg for the cachexia patient.

The problem of cachexia patients taking medication is a serious hurdle in their treatment. Therapeutic combinations, e.g., pharmaceutical compositions comprising various formulations, and accommodations for this problem, as provided by the instant invention, will help the patient take medication as needed and therefore increase the likelihood of a positive response.
a) In one aspect, the beta blocker nadolol, a non-selective, kidney excreted product has a half-life of 10-24 hours and is taken once per day at 40 mg. In one aspect, the invention provides this product for cachexia as an easy to swallow pill or in a liquid formulation.
b) In one aspect, the liquid formulation of nadolol is also provided with a high calorie nutritional drink with high amino acid content and additional tryptophan, leucine and glutamine but with low sugar and added vitamins. In one aspect, the liquid nadolol is combined with the nutrient drink which is immediately taken by the patient. In one aspect, a second dose is administered with a second nutritional drink to provide additional calories, nutrients and drug effect.

In one aspect, the combination of pindolol and ibuprofen is used; this combination formulation of the invention has additional benefits for patients with low blood pressure. Since pindolol has intrinsic sympathomimetic activity it has less of an effect on blood pressure and resting heart rate. Pindolol does not suppress the heart rate increase during exercise which may allow for more comfortable physical activity. The use of ibuprofen has been shown to reduce inflammation and may be useful in maintaining blood pressure as described above.
a) In one aspect, the beta blocker pindolol is provided at a starting dosage of 10 mg and ibuprofen at 400 mg twice daily. In one aspect, the patient receives both medications at the same time. In one aspect, the dose of the pindolol is increased weekly to obtain a reduction in heart rate of 20% as long as the blood pressure does not go below 90/60.
b) The medication is provided in any of the formats described herein.

The invention provides a therapeutic combination, e.g., a pharmaceutical composition, comprising a beta blocker with approximately equal activity against beta2 and beta1 and approximate 25% of the relative activity against beta3 in combination with a non-steroidal anti-inflammatory drug that is not metabolized by the liver. The beta blocker has a long half-life in the patient so dosing is required only once per day. In this combination the NSAID is selected that is not liver metabolized. Both the beta blocker and the NSAID are excreted through the kidneys and do not induce metabolizing enzymes.
a) For example, in one aspect, the beta blocker nadolol is provided at a starting dosage of 40mg. The NSAID naproxen is provided at 500 mg once daily. In one aspect, the patient receives both medications at the same time. In one aspect, the dose of the nadolol is increased weekly to obtain a reduction in heart rate of 20% as long as the blood pressure does not go below 90/60. In one aspect, a second dose of naproxen may be taken after 12 hours.
b) In this combination the active ingredients are excreted through the kidney. This provides more reliable dosing between patients and prevents induction of metabolizing enzymes. The relative activity against the different receptor subtypes is balanced, thereby providing the most likely chance of obtaining a therapeutic effect. The presence of the naproxen serves to also maintain blood pressure and to reduce the inflammatory response caused by the disease.
c) In addition, both these drugs are long acting so dosing can be reduced to a single dose of nadolol and naproxen per day. This reduced frequency of dosing may be particularly attractive to patients who are unable or unwilling to take medications on a frequent basis and will promote patient compliance.
d) The medication is provided in any of the formats described herein.

The invention provides a therapeutic combination, e.g., a pharmaceutical composition, comprising a combination product that is useful in patients with impaired pulmonary function for which beta-blockers would be ordinarily contraindicated. Since beta blockers that impact the beta 2 receptor subtype are particularly susceptible to pulmonary constriction a selective beta-blocker, with more activity against beta-1 subtype, is selected.

For example, the invention provides a therapeutic combination comprising the beta1 blocker atenolol, which has low pulmonary activity and is more cardiac selective (more activity against beta-1 subtype receptors than beta-2 receptors), and the NSAID etodolac. Atenolol has approximately 16 fold higher relative activity against beta 1 receptor subtypes as compared to a nonselective beta blocker such as propranolol. This reduced activity against the pulmonary associated subtype is responsible for the reduced chance of negative effect in patients with pulmonary disease.
a) In one aspect, the atenolol is provided at a starting dosage of 25mg once per day. The NSAID etodolac is provided at 500 mg once daily. The patient receives both medications at the same time. In one aspect, the dose of the atenolol is increased weekly to obtain a reduction in heart rate of 20% as long as the blood pressure does not go below 90/60. A second dose of etodolac may be taken after 12 hours.
b) In one aspect, an extended release formulation of etodolac is taken once per day with the atenolol to make it easier for the patient. The adjustment of the dose needs to occur stepwise as described above.

The invention provides a therapeutic combination, e.g., a pharmaceutical composition, that is a combination product comprising a cardioselective beta blocker with an NSAID to target the heart more directly. In one aspect, the beta blocker metoprolol (6:1 beta1:beta2 selectivity) is used in combination with the NSAID ibuprofen. This combination provides a beta blocker that is cardioselective to reduce high blood pressure and reduce high heart rate. The presence of the additional beta blocker activity against beta2 and beta3 provides additional reduction in energy expenditure. In one aspect, the ibuprofen is used as an anti-inflammatory to reduce the counter-productive effects of the inflammatory response in these patients.
a) In one aspect, the metoprolol is provided at an initial dose of 25 mg twice daily. In one aspect, the dose is increased weekly to obtain at least a 20% reduction in heart rate but the blood pressure is maintained above 90/60.

The invention provides a therapeutic combination, e.g., a pharmaceutical composition, for hospitalized patients that is a combination product comprising a non-selective beta blocker in combination with the natural anti-inflammatory compound eicosapentaneoic acid (EPA, from fish oil). This therapeutic combination of the invention provides a very strong anti-inflammatory delivered directly into the blood stream. This alleviates the usual problem with EPA in that it is metabolized by the liver and is rapidly metabolized, so the actual dose is relatively low. The relative activity of the beta blocker against the different receptor subtypes is balanced, thereby providing the most likely chance of obtaining a therapeutic effect. The presence of the EPA serves to reduce inflammation and is know to have the side-benefit of being beneficial to the treatment of cancer. In one aspect, the propranolol is administered intravenously (e.g., 2 micrograms/kg/min infusion following 80 microgram/kg infusion).
a) In one aspect, the beta blocker propranolol is provided through a nasogastric tube at a starting dosage of 40 mg. In one aspect, the EPA is provided as a daily parenteral infusion for one week followed by oral EPA. In one aspect, the patient receives daily dosing of intravenous EPA (100mg/day). In one aspect, the dose of the propranolol is adjusted daily to obtain a reduction in heart rate of 20% as long as the blood pressure does not go below 90/60.

One of the side effects of beta blockers on some patient may be a feeling of tiredness. Thus, the invention provides a therapeutic combination, e.g., a pharmaceutical composition, that is a combination product of a non-selective beta blocker with an NSAID at a dosing schedule that will provide a higher dose of beta blocker in the night and a lower dose during the day. In one aspect, the same level of beta blocker is provided at both times.
a) In one aspect, the beta blocker metoprolol is provided in a dose-pack that is color coded (e.g., yellow) containing a low dose of the beta blocker in combination with the NSAID etodolac to indicate daytime administration and a high dose that is color coded (another color, e.g., navy blue) containing a higher dose of metoprolol with etodolac for bedtime administration. In one aspect, the dose pack is provided for a month supply with an increasing dose of metoprolol in the evening combination.
b) exemplary dose pack is in the following table.

| Week | Daytime Medication | Evening Medication |
|---|---|---|
| 1 | 25 mg metoprolol 500 mg etodolac | 25 mg metoprolol 500 mg etodolac |
| 2 | 25 mg metoprolol 500 mg etodolac | 50 mg metoprolol 500 mg etodolac |
| 3 | 50 mg metoprolol 500 mg etodolac | 50 mg metoprolol 500 mg etodolac |
| 4 | 50 mg metoprolol 500 mg etodolac | 100 mg metoprolol 500 mg etodolac |

In one aspect, after completing the initial dose pack the patient continues ongoing treatment with the maximum tolerated dose.

Patients with cachexia have several issues that need to be addressed in terms of their psychological and physical ability to take medications. In order to increase compliance with the therapy the invention provides therapeutic combinations in a variety of formats that can best fit with the patient's lifestyle.

In one aspect, the invention provides a therapeutic combination comprising a beta blocker and an NSAID in a single pill wherein the two components are physically separated. Combining both products into one pill relieves the patient of needing to take multiple pills at multiple times during the day. The beta blocker can be any of those listed in Table, below, and the NSAID can be any of those from Table, below.
a) In one aspect, combining the non-specific beta-blocker propranolol with the NSAID etodolac, both supplied as extended release formulations, provides a single pill that can be taken just once per 24 hour period thereby easing the burden on the patient. In one aspect, a series of pills are provided that will allow increased dosing of the beta blocker to achieve a 20% reduction in heart rate and blood pressure above 90/60 while keeping a constant amount of NSAID.
b) In one aspect, the two components of the pill are provided in a dual chamber capsule that does not allow intermixing of the two active ingredients.

In one aspect, the invention provides methods and compositions for making therapeutic combinations and methods for the taking of medicine in a more user friendly manner, e.g., the invention provides compositions in a form that is easier to take, such as in a liquid. In one aspect, the invention provides a product comprising a beta blocker and an NSAID in liquid form. In one aspect, the packaging is configured such that the beta blocker and NSAID are supplied in two compartments on a single unit. Removing the seal allows for easy dispensing of both drugs at the same time in a convenient format. In one aspect, the dose-packs are provided in multiple colors to indicate different doses of beta blocker (to allow increased dosing to achieve a 20% reduction in heart rate and maintain blood pressure over 90/60) with a constant amount of NSAID. In one aspect, combining both products in an easy to use and easy to recognize dose format provides a user-friendly way to provide proper doses while increasing patient compliance. In one aspect, the liquid format facilitates delivery to a patient who is experiencing difficulty in swallowing or in complying with taking more than one pill at more than one time per day. In one aspect, beta blockers are administered in this way are any of the beta blockers listed in the Tables shown herein, and in combination with any of NSAIDs, as described herein.

The cachectic patient has special needs with regards to dosing considering their weakened state and low body weight. The standard commercial doses available for beta blockers are based on patients of normal size, metabolism and physiology. The cachectic patient is underweight, has altered metabolic rate and dysregulated physiology. Thus, to accommodate these patients, the invention provides doses which are below the standard doses supplied. One exemplary dose is obtained by providing a dose that is 50% of the current minimum and providing increased doses that are each 20% less than the available dose. The following table provides an example of exemplary (e.g., recommended, depending on the condition and the individual) doses for select beta blockers useful in the treatment of cachexia. A similar dose range could be provided for any beta blocker, e.g., as listed in Tables described herein.

### Exemplary Doses (mg/capsule) of beta blockers used in the invention's treatment of cachexia

| Propranolol | Inderal LA / InnopranXL | Metoprolol | Nadolol | Atenolol | Pindolol |
|---|---|---|---|---|---|
| 10 | 30 | 6 | 25 | 6 | 2.5 |
| 16 | 48 | 20 | 36 | 20 | 8 |
| 32 | 64 | 40 | 64 | 40 | 12 |
| 64 | 96 | 80 | 96 | 80 | |
| 96 | 144 | | | | |

In one aspect, the doses are provided in an economical form so the physician can adjust dose on a regular basis as needed without the patient being required to obtain and fill a new prescription for each dose. During the adjustment period a dose pack is provided with 10 pills of each dose. The patient is prescribed the initial dose pack and starts at the first week dose. After approximately 7 days (5-9) the patient is checked by the doctor and the next dose is administered as warranted. When the proper dose is obtained the patient receives a monthly dose-pack with a constant level of the required dose sufficient to last one month.

In one aspect, the invention provides for the administration of beta blockers in a dose that restores the 1/*ƒ* scaling in heart rate. The healthy human heart rate has been known to exhibit 1/*ƒ*-type fluctuations to demonstrate multifractal scaling properties. The healthy 1/*ƒ* scaling in heart rate requires the balance between the antagonistic activity of the parasympathetic nervous system (PNS) and the sympathetic nervous system (SNS). However, in the patient with cachexia the 1/*ƒ* fluctuations are lost and instead show a "too regular" distribution. By recording the EKG of patients and determining the R-R interheartbeat variation it is possible to identify patients with dysregulated PNS/SNS that should be considered for further diagnosis. Patients that also have increased levels of epinephrine should be considered as candidates for treatment with beta blockers alone or in combination with another drug such as an NSAID. Patients with "too regular" heartbeat and increased levels of inflammatory cytokines (IL-6, IFN-γ or TNF-α) are also good secondary indicators of patients in need of therapy.

In one aspect, the invention provides for the administration of beta blockers at the appropriate dose to restore the 1/*ƒ* scaling in heart rate. In one aspect, the proper dose also maintains a heart rate that is 60 beats per minute (bpm) or above and blood pressure that is over 90/60.

### Example 5: Exemplary Therapeutic Combination Protocols of the Invention

The following example describes exemplary treatment protocols of the invention, using exemplary therapeutic combination compositions of the invention.

In these exemplary treatment protocols of the invention, the dosing schedule is designed to provide a higher dose of beta blocker, e.g., propranolol, in the morning (the AM) when sympathetic activity is high, with a lower long lasting beta blocker dose in the evening to reduce the impact on CNS such as "vivid dreams"; and, in the evening, a relatively higher dose of an anti-anxiety and/or an anti-inflammatory medication, such as an NSAID. For example, in one aspect, etodolac, or any other NSAID, e.g., aspirin, diclofenac; diflunisal, fenoprofen, flurbiprofen, ibuprofen, indomethacin, ketoprofen; ketorolac, meclofenamate, mefenamic acid, meloxicam, nabumetone, naproxen, oxaprozin, piroxicam, salsalate, sulindac, tolmetin, a COX-2 inhibitor, such as a COX-2-selective inhibitor (e.g., celecoxib rofecoxib, etoricoxib, valdecoxib, parecoxib, meloxicam or lumiracoxib) or a combination thereof, is given higher in the evening to provide pain relief and foster better sleep as well as the effect on systemic inflammatory response.

For example, the invention provides a product of manufacture comprising, e.g., a blister package, clamshell, tray or shrink wrap comprising at least two dosages of beta adrenergic receptor antagonist (a beta blocker) drug and at least two dosages of non-steroidal anti-inflammatory drug (a NSAID) and/or anti-anxiety drug, and the drugs are organized or labeled in the blister package, clamshell, tray or shrink wrap for usage by an individual for at least two administrations (e.g., clearly labeled for being taken by a patient at different times in the day), at least one of each (beta blocker and non-steroidal anti-inflammatory drug (a NSAID) and/or anti-anxiety drug) in the morning and at least one of each in the evening, wherein the dosage schedule provides a relatively higher dose of beta blocker in the morning (the AM) than in the evening, and a relatively higher dose of an anti-anxiety and/or an anti-inflammatory medication in the evening than in the morning. For example, in alternative embodiments, the packaging and/or labeling can have a higher dosage pill, tablet, geltab and the like, of beta blocker in the morning "section" of the blister package, clamshell, tray or shrink wrap, than the dosage of pill, tablet, geltab and the like, of beta blocker in the evening "section". Alternatively, two tablets can be provided in the morning "section" versus the evening "section". The reverse would be the case for the non-steroidal anti-inflammatory drug (a NSAID) and/or anti-anxiety drug.

Beta-adrenergic antagonists (beta-blockers) and non-steroidal anti-inflammatory drugs (NSAIDs) can reduce the level of inflammatory cytokines, epinephrine, acute phase proteins response, as well as normalize EKG patterns and attenuate weight loss in severely ill subjects. In addition, beta-blockers and NSAIDs have offsetting hypotensive and hypertensive side effects. Using this convergence of activities, the invention provides use of beta-blockers and NSAIDs together to modulate the dysregulated psycho-neuroendocrine-immune systems and the associated clinical manifestations of cachexia. Prior clinical trials have shown that these drugs (alone) support positive outcomes in cachectic patients.

This open pilot clinical trial is designed to evaluate the safety of a beta blocker (propranolol or metoprolol) in combination with a non-steroidal anti-inflammatory drug (etodolac) in cachectic metastatic cancer patients. In addition, to determine whether a twelve week treatment with this drug combination will decrease their weight loss, inflammatory markers and improve quality of life. Propranolol, metoprolol and etodolac have been selected for this clinical trial based on their well known safety records and previous positive clinical trials in cachexia related indications.

The primary goal is to evaluate the safety of this drug combination, which has been utilized clinically, but not previously assessed in a clinical trial. Secondarily, individual subjects will be analyzed to determine the comparison differences in measurements of blood and urine analysis, C reactive protein (CRP), heart rate, blood pressure, weight, lean body mass, and quality of life before, during and at the conclusion of the combination drug treatment. Because of the variation in types and stages of cancer, as well as the small number of patients involved, this is a pilot longitudinal study designed to generate data to support a larger trial to definitively determine the efficacy of the treatment.

Thus, the invention provides compositions and methods for ameliorating and/or preventing cachexia, a complex syndrome that combines weight loss, loss of muscle and visceral protein, lipolysis, anorexia, chronic nausea, and weakness. In addition, the invention provides compositions and methods for ameliorating and/or preventing cachexia is associated with anemia, immunosuppression, insulin resistance, anxiety, and sleep disturbances. These syndromes treated by the compositions and methods of the invention can be broadly characterized as Chronic Systemic Inflammatory Response Syndrome or Chronic SIRS. The compositions and methods can also be used to treat and/or prevent severe cachexia occurring in most patients with advanced cancer, AIDS and other end-stage chronic diseases. At the moment of diagnosis, about 80% of patients with upper gastrointestinal cancers and 60% of patients with lung cancer have substantial weight loss. In general, patients with solid tumors (with the exception of breast cancer) have a higher frequency of cachexia. The compositions and methods can also be used to treat and/or prevent cachexia in children and elderly patients, addressing a cachexia that becomes more pronounced as disease progresses.

Results of clinical studies (a CRP, in more detail below): data from 4 subjects, 3 were very successful (weight losing (5 lb 2 mo) and high heart rate (>=80bpm) to weight stabilization (gain of 2-3 lbs over 3 weeks) with reduced heart rate in the low 70s), one is a "technical success" in that the catecholamines were reduced, but he is not eating, so of course is not gaining weight. In all patients enrolled the CRP is elevated but other cytokines (IL-1, IL-2, IL-6, IFN-gamma, TNF alpha are all normal.

Patient selection can be an important criteria for selection of patient for this protocol. All cachexia studies to date have used weight as the only criteria. By using heart rate (greater than 80 beats per minute (bpm) or 6 bpm increase over two months) with weight loss (5 lbs over 2 months) a population with cachexia as opposed to anorexia or just malnourished (due to treatment, depression or whatever) can be selected.

In one aspect, in practicing the invention, anorexia is distinguished from cachexia driven weight loss. In one aspect, in practicing the invention, anorexia is identified as an adaptive nutritional state secondary to lack of nutrition. Anorexia is responsive to increased nutrition. In one aspect, in practicing the invention, cachexia is identified as a maladaptive nutritional state secondary to a maladaptive severe chronic systemic inflammatory response and autonomic dysregulation. Cachexia is non-responsive to increased nutrition.

Within the analytical framework of this invention, while the invention is not limited by any particular mechanism of action, the natural history of a healthy inflammatory response is characterized by a sequence of feedback responses beginning with tissue breakdown leading to a stress response of inflammation and immune activation and finally restoration of tissue integrity. If the sequence of events is within normal adaptive range, both in terms of the extent of trauma and duration of the response, then tissue integrity is restored. However, in some trauma (e.g., cancer, congestive heart failure (CHF), AIDS, burn, etc) a positive feedback cycle of escalating stress response and maladaptive immune system feedback leads to a severe dysregulated psycho-neuroendocrine-immune state.

The complexity of cachexia, in its many forms and from many causes, has made it difficult to manage. With the identification of the maladaptive autonomic nervous system (ANS) and an inflammatory response as the primary underlying causes of the disease, this invention utilizes compounds that regulate the autonomic nervous system, in particular the adrenergic system, with the beta blocker in combination with the non-steroidal anti-inflammatory drug (NSAID), for treatment of chronic SIRS, and specifically cancer cachexia.

While the invention is not limited by any particular mechanism of action, the cachexia syndrome appears to be associated with malign patterns of eicosanoid production and the presence of a chaotic population of cytokines with consequent aberrations in neuro-endocrine-immune control systems, hypothalamic control of appetite, and intermediary metabolism. Metabolic changes may be associated with changes in autonomic control and an increase in body energy consumption, with further mismatches of food intake with need. The systemic aberrations are similar to the acute stress reaction commonly observed in patients with life-threatening infections or major trauma. When this potentially life-saving "switch" is left on, over time the chronic stress inflammatory reaction may enhance tumor growth while inducing a devastating effect on a variety of organs and tissues.

In practicing this invention the wasting in cancer patients is slowed or reversed - the process where muscle synthesis and repair decline is slowed or reversed, and the increased muscle proteolysis (possibly due to tumor-produced proteolysis factors) is slowed or reversed. The wasting syndrome is commonly noted at the time of diagnosis in many forms of cancer, notably those of the upper gastrointestinal tract and lung, progresses through the course of illness, finally leaving the patient in a severe state of malnutrition. A recent review details current concepts of this pathophysiology and the relevance of these concepts to anorexia-cachexia research (e.g., see MacDonald (2003) J. Am. Coll. Surg. 197:143-61.

Markers of chronic inflammation (e.g., C reactive protein [CRP]) and increased production of certain cytokines, notably IL-6, TNFα and (IFN)-γ correlate with both cachexia and with poor prognosis in cancer patients and other illnesses. Although evidence has been presented for circulatory levels of tumor catabolic products in humans, there is less evidence for circulating cytokines. In many cases where serum levels of cytokines such as TNF α are elevated, these levels correlate with the stage of the disease, reflecting tumor size and metastasis and not specifically with cachexia. Chronic inflammation correlates with other symptoms, such as fatigue, which are common in cancer and other chronic illnesses.

The autonomic nervous system helps to control arterial pressure, gastrointestinal motility, urinary bladder emptying, sweating, body temperature, and many other activities. The autonomic nervous system is primarily activated by centers in the spinal cord, brain stem, and hypothalamus. The efferent autonomic signals are transmitted to the various organs of the body through either the sympathetic nervous system or the parasympathetic nervous system. The neurotransmitter of all preganglionic autonomic fibers, all postganglionic parasympathetic fibers, and a few post-ganglionic sympathetic fibers is acetylcholine. These are referred to as cholinergic fibers. The adrenergic fibers compose the majority of the postganglionic sympathetic fibers and use the norepinephrine (noradrenaline) as the neurotransmitter. In cancer patients elevated resting energy expenditure is not likely due to tumor metabolism, but more likely it is due to an interaction between the cancer and the neuroendocrine-immune system. The elevated resting energy metabolism is primarily due to the increased adrenergic activity in cancer patients.

While the invention is not limited by any particular mechanism of action, the normal adaptive nutritional states control of the use of energy stores (protein, fat and carbohydrates) is by a number of factors including insulin/glucagon, catecholamines, ghrelin and related orexigenic and anorectic peptide signals from the neuroendocrine-immune system. The result of this regulation is the use of carbohydrates first, followed by mobilization and oxidation of fatty acids and finally degradation of protein and release of gluconeogenic amino acids. In cachexia (stressed state) there is a dysregulation of this system such that protein is not spared at the expense of carbohydrates or fat and there is no reduction in metabolic rate.

While the invention is not limited by any particular mechanism of action, the natural history of healthy inflammation is characterized by a sequence of flux imbalances and positive feedback responses. Thus, the compositions and methods of the invention address - treat, ameliorate and/or prevent - a breakdown in tissue integrity due to trauma, infection, cancer, etc.; stress, with associated imbalanced metabolic and signaling fluxes; a stress response characterized by a cycle of immune activation, inflammation and healing, and practicing the invention results in the ultimate restoration of tissue integrity, balanced fluxes (homeostasis), and immune inactivation. As long as the trauma is within the adaptive range and the immune system correctly partitions, escalates and de-escalates its immune response, the process is adaptive and restores tissue integrity.

The compositions and methods of the invention treat, ameliorate and/or prevent a maladaptive immune response in cancer. If not treated, the cancer progresses, leading to a positive feedback cycle of escalating cancer induced flux imbalances and escalating maladaptive immune system feedback responses. The escalating flux imbalances and maladaptive feedback responses leads to a severe dysregulated neuroendocrine-immune state. The specific pattern of neuroendocrine-immune imbalances appears to partition into three broad classes of maladaptive infection response (dominated by gamma interferon), maladaptive trauma response (dominated by IL-6) or maladaptive wound healing response (dominated by TNF or IL-1). The overall state is characterized by a chronic SIRS.

In the normal state a weight-stable adult takes in energy that matches the amount used by the body. If energy supply is too low (fasting state) the body compensates by releasing orexigenic hormones to stimulate feeding and reducing total energy expenditure through the sympathetic nervous system, or more generally the neuroendocrine-immune system. If energy supply is in excess (overfed state) the body stores the excess primarily as fat. In normal adaptive nutritional states control of the use of energy stores (protein, fat and carbohydrates) is by a number of factors including insulin/glucagon, catecholamines, ghrelin and related orexigenic and anorectic peptide signals from the neuroendocrine-immune system. The result of this regulation is the use of carbohydrates first, followed by mobilization and oxidation of fatty acids and finally degradation of protein and release of gluconeogenic amino acids. In cachexia (stressed state) there is a dysregulation of this system such that protein is not spared at the expense of carbohydrates or fat and there is no reduction in metabolic rate.

In the retrospective study (45 records, untreated control) if 5 pound (lb) weight loss over 2 months is used as the criteria for entry we have 32% return to normal weight in 12 weeks with an average weight loss of 5.6 lbs. If we use 5 lb weight loss in previous 2 months with heart rate > = 80 bpm the regression to mean is only 23% and the patients lose 6 lbs. Finally, if we use 5 lb weight loss and an increase of 6 beats/min heart rate we have 14% regression to mean and 8 lb weight loss.

### Study A

This clinical trial was done to evaluate the effect of beta blocker (propranolol or metoprolol) in combination with an NSAID (etodolac) to assess the influence of these compounds on weight and quality of life in subjects diagnosed with metastatic cancer.

In general, it is not difficult to identify affected cancer patients, and patients that would benefit by taking the therapeutic combinations of medications of the invention. In North Central Cancer Treatment Group (NCCTG) research trials involving over 2,300 patients, very simple criteria for anorexia/cachexia has been used:
- A 5-lb weight loss in the preceding 2 months and/or an estimated daily caloric intake < 20 calories/kg
- A desire by the patient to increase his or her appetite and gain weight
- The physician's opinion that weight gain would be beneficial for the patient

As noted above, the primary objective of this study was to evaluate the effect of beta blocker (propranolol or metoprolol) in combination with an NSAID (etodolac) to assess the influence of these compounds on weight and quality of life in subjects diagnosed with metastatic cancer. Another goal is to determine the effects of a 12-week treatment of combination therapy with a beta-blocker and an NSAID on weight, and quality of life.

### Materials and Methods

Age: 18 years and over
Sex: Male and Female
Diagnosis: Metastatic Cancer, non-hematological
Trial sample: A total of 80 subjects will participate in this trial. Recruitment period for the trial is 12 months. The overall duration should not exceed 18 months. Forty (40) subjects will be treated and 40 will be followed for case controls.

### Inclusion Criteria

- Subject having confirmed malignancy
- Subjects demonstrating average weight loss of 5 or more pounds (2.3kg) within 2 months prior to trial recruitment
- Subjects demonstrating average heart rate increase of 6 bpm or more within 2 months prior to trial recruitment
- Negative pregnancy test, (female patients of child bearing age)
- Able to give Informed Consent
- Subjects are able to swallow medication tablets
- Subjects are able to ingest food or food supplements
- Subjects who are not on or who have not taken beta-blockers for more than one-month prior to entry into this trial
- Subjects who have not received radiation therapy or surgery for at least 2 weeks prior to entry into this trial

### Exclusion Criteria

- Contraindication to propranolol or metoprolol or etodolac
- Hypersensitivity reaction to propranolol or metoprolol or etodolac
- History of myocardial infraction within the past 3 months
- Evidence of congestive heart failure
- Unstable angina
- History of GI bleeding
- Uncontrolled diabetes
- History of psychiatric disorders other than depression
- A positive pregnancy test
- No evidence of infection
- History of bleeding disorder
- Not on digoxin or other chronotropic drugs

### Study Medications

- Propranolol (Inderal, Inderal LA) with etodolac (Lodine) or if contraindication to propranolol physician may use metoprolol (Lopressor, Toprol XL).
- 2x/day dosing, flexible dosing adjusts weekly to reach 20% reduction in heart rate (minimum heart rate of 60 bpm) with blood pressure over 90/60.

### Dosage Form and Range

Propranolol: Propranolol is a synthetic beta-adrenergic receptor blocking agent chemically described as 2-Propanol, 1-[(1-methylethyl)amino]-3-(1-naphthalenyloxy)-, hydrochloride, (+)-. Propranolol is a stable, white, crystalline solid which is readily soluble in water and ethanol. The immediate release product is available as 10 mg, 20 mg, 40 mg, 60 mg, and 80 mg tablets for oral administration. The inactive ingredients contained in each tablet are: lactose, magnesium stearate, microcrystalline cellulose, and stearic acid. In addition, 10 mg and 80 mg tablets contain FD&C Yellow No. 6 and D&C Yellow No. 10; 20 mg tablets contain FD&C Blue No.1; 40 mg tablets contain FD&C Blue No. 1, FD&C Yellow No. 6, and D&C Yellow No. 10; 60 mg tablets contain D&C Red No. 30.

The LA product is available on 60 mg, 80 mg, 120 mg and 160 mg capsules. Inactive ingredients include: cellulose, ethylcellulose, gelatin capsules, hypromellose, and titanium dioxide. In addition, Inderal LA 60 mg, 80 mg, and 120 mg capsules contain D&C Red No. 28 and FD&C Blue No. 1; Inderal LA 160 mg capsules contain FD&C Blue No. 1.

Subjects will be prescribed propranolol from 80 mg/per day to a maximum of 160 mg/per day in divided daily dosages.

Metoprolol: The immediate release tablet, metoprolol tartrate, is a selective betal-adrenergic receptor blocking agent, chemically described as (±)-1-(isopropylamino)-3-(p-(2-(methoxyethyl) phenoxy)-2-propanol (2:1) dextrotartrate salt. Metoprolol tartrate is a white, practically odorless, crystalline powder which is very soluble in water; freely soluble in methylene chloride, in chloroform, and in alcohol; slightly soluble in acetone; and insoluble in ether. It is available as 25 mg, 50 mg, and 100 mg tablets for oral administration.

The inactive ingredients in the tablets include cellulose compounds, colloidal silicon dioxide, D&C red no. 30 aluminum lake (50-mg tablets), FD&C blue no. 2 aluminum lake (100-mg tablets), lactose, magnesium stearate, polyethylene glycol, propylene glycol, povidone, sodium starch glycolate, talc, and titanium dioxide.

The extended release tablet metoprolol succinate, is a betal-selective (cardioselective) adrenoceptor blocking agent, for oral administration, available as extended release tablets. TOPROL-XL has been formulated to provide a controlled and predictable release of metoprolol for once-daily administration. The tablets comprise a multiple unit system containing metoprolol succinate in a multitude of controlled release pellets. Each pellet acts as a separate drug delivery unit and is designed to deliver metoprolol continuously over the dosage interval. The tablets contain 23.75, 47.5, 95 and 190 mg of metoprolol succinate equivalent to 25, 50, 100 and 200 mg of metoprolol tartrate, USP, respectively. Its chemical name is (±)1-(isopropylamino)-3-[p-(2-methoxyethyl) phenoxy]-2-propanol succinate (2:1) (salt). Metoprolol succinate is a white crystalline powder. It is freely soluble in water; soluble in methanol; sparingly soluble in ethanol; slightly soluble in dichloromethane and 2-propanol; practically insoluble in ethyl-acetate, acetone, diethylether and heptane. Inactive ingredients: silicon dioxide, cellulose compounds, sodium stearyl fumarate, polyethylene glycol, titanium dioxide, paraffin.

Subjects will be prescribed metoprolol from 50 mg/per day to a maximum of 150 mg/per day in divided daily dosages.

Etodolac: Etodolac is a pyranocarboxylic acid chemically designated as (+) 1, 8-diethyl-1,3,4,9-tetrahydropyrano-[3,4-b]indole-1-acetic acid. It is a white crystalline compound, insoluble in water but soluble in alcohols, chloroform, dimethyl sulfoxide, and aqueous polyethylene glycol. It will be provided as 200 mg capsule and 500 mg tablet. The inactive ingredients contained in each tablet are: cellulase, hydroxypropyl, methylcellulose, lactose, magnesium stearate, polyethylene glycol, polysorbate 80, povidone, sodium starch glycolate, and titanium dioxide. Subjects will be prescribed 400 to 700 mg/per day of Etodolac in divided daily dosages.

### Exemplary Dosage Administrations of the invention (see also table below)

In one aspect, doses of propranolol are given in 20 or 40 mg tablets immediate release on a bid basis. In one aspect, the first dose week the doses for propranolol will be started 20 mg in the morning and 20 mg at bedtime. After 1 week the dosage should be adjusted to 20 mg of the immediate release product in the morning and 60 mg of the extended release at bedtime. If after an additional week the subject shows no improvement or has not obtained a 20% reduction in heart rate, without decreasing heart rate below 60 bpm or blood pressure below 90/60, the dose may be adjusted to 40 mg of the immediate release product in the morning and 120 mg of the extended release product at bedtime.

In one aspect, doses of metoprolol are given in 25 or 50 mg tablets on a bid basis. Doses for metoprolol will be started at 25 mg of the immediate release product in the morning and at bedtime. After 1 week the dosage should be adjusted to 25 mg of the immediate release product in the morning and 50 mg of the extended release product at bedtime. If after an additional week the subject shows no improvement or has not obtained a 20% reduction in heart rate, without decreasing heart rate below 60 bpm or blood pressure below 90/60, the dose may be adjusted to 50 mg of the immediate release product in the morning and 100 mg of the extended release product at bedtime.

In one aspect, doses of etodolac are given in 200 mg capsules or 500 mg tablets on a bid basis. Doses for etodolac will be started at 200 mg in the morning and at bedtime. After 1 week the dosage should be adjusted to 200 mg in the morning and 500 mg at bedtime. No additional change in the dosage of etodolac is made.

These exemplary dosage regimens of the invention are governed by the investigators observations noted in the metabolic changes occurring in the cardiovascular and neuroendocrine systems.

### Propranolol

| Week 1 | | | |
|---|---|---|---|
| Time | Propranolol, Immediate Release | Propranolol, Extended Release | Etodolac |
| Morning | 20 | 0 | 200 |
| Evening | 20 | 0 | 200 |

| Week 2 | | | |
|---|---|---|---|
| Time | Propranolol, Immediate Release | Propranolol, Extended Release | Etodolac |
| Morning | 20 | 0 | 200 |
| Evening | 0 | 60 | 500 |

| Week 3 | | | |
|---|---|---|---|
| Time | Propranolol, Immediate Release | Propranolol, Extended Release | Etodolac |
| Morning | 40 | 0 | 200 |
| Evening | 0 | 120 | 500 |

### Metoprolol

| Week 1 | | | |
|---|---|---|---|
| Time | Metoprolol, Immediate Release | Metoprolol, Extended Release | Etodolac |
| Morning | 25 | 0 | 200 |
| Evening | 25 | 0 | 200 |

| Week 2 | | | |
|---|---|---|---|
| Time | Metoprolol, Immediate Release | Metoprolol, Extended Release | Etodolac |
| Morning | 25 | 0 | 200 |
| Evening | 0 | 050 | 500 |

| Week 3 | | | |
|---|---|---|---|
| Time | Metoprolol, Immediate Release | Metoprolol, Extended Release | Etodolac |
| Morning | 50 | 0 | 200 |
| Evening | 0 | 100 | 500 |

Duration of therapy: Treatment will be given for a maximum of 12 weeks.

### Concomitant Medications:

The following concomitant medications will not be allowed during the clinical trial:
- No corticosteroids unless used intermittently as part of a pre-chemotherapy
- No estrogens
- No progestins or other steroids
- No oral anti-therapeutic anticoagulants
- No anticonvulsants
- No thioridazine (Mellaril^{®})

### Study B

This study is an open pilot clinical trial to evaluate the safety of propranolol in combination with etodolac in subjects diagnosed with metastatic cancer and secondary outcomes of weight, inflammatory markers and quality of life. Another goal of the study is to determine the effects of a 6-week treatment of combination therapy with a beta-blocker and an NSAID on weight due to lack of appetite, inflammatory markers and quality of life.

### Materials and Methods

Age: 18 years and over
Sex: Male and Female
Diagnosis: Metastatic Cancer
Trial sample: A total of 20 subjects will participate in this trial. Recruitment period for the trial is 12 months. The overall duration should not exceed 18 months.

### Inclusion Criteria

- Subject having histologically or cytologically confirmed malignancy with a life expectancy of 3 months minimum
- Subjects demonstrating weight loss of minimum of > 5(2.3 kg) within 2 months prior to trial recruitment and/or an estimated caloric intake of less than 20 calories/kg daily
- Subjects with a Body Mass Index no greater than 30
- Negative pregnancy test, (female patients of child bearing age)
- Able to give Informed Consent
- Subjects are able to swallow medication tablets
- Subjects are able to ingest food or food supplements
- Subjects who are not on or who have not taken beta-blockers for more than one-month prior to entry into this trial
- Subjects who have not received radiation therapy for at least 2 weeks prior to entry into this trial
- Subjects who have not received Megestrol acetate or Marinol or other appetite stimulants within 2 months prior to trial entry
- Subjects having a heart rate of 80 bpm or greater

### Exclusion Criteria

- Contraindication to beta-blockers
- History of hypersensitivity reaction to propranolol or etodolac
- A history of myocardial infraction within the past 3 months
- Evidence of congestive heart failure
- Unstable angina
- Present history of pulmonary edema, Chronic Obstructive Pulmonary Disease (COPD), asthma or bronchospastic diseases
- History of GI bleeding
- Unstable diabetes
- History of psychiatric disorders other than depression
- Subjects with a history of marijuana use within two months of trial entry
- A positive pregnancy test

### Study Medications:

Each subject will be administered Propranolol in combination with Etodolac. Propranolol (INDERAL®) or etodolac (LODINE®) will be supplied.

### Dosage Form:

Propranolol (INDERAL®): Propranolol is a synthetic beta-adrenergic receptor blocking agent chemically described as 2-Propanol, 1-[(1-methylethyl)amino]-3-(1-naphthalenyloxy)-, hydrochloride, (+)-. It is a stable, white, crystalline solid which is readily soluble in water and ethanol. It is available as 20 mg, 40 mg, 60 mg, and 80 mg tablets for oral administration. The inactive ingredients contained in each tablet are: lactose, magnesium stearate, microcrystalline cellulose, and stearic acid. In addition, 10 mg and 80 mg tablets contain FD&C Yellow No. 6 and D&C Yellow No. 10; 20 mg tablets contain FD&C Blue No.1; 40 mg tablets contain FD&C Blue No. 1, FD&C Yellow No. 6, and D&C Yellow No. 10; 60 mg tablets contain D&C Red No. 30. Subjects will be prescribed Propranolol from 80 mg/per day to a maximum of 160 mg/per day in divided daily dosages.

Etodolac (LODINE®): Etodolac is a pyranocarboxylic acid chemically designated as (+) 1, 8-diethyl-1,3,4,9-tetrahydropyrano-[3,4-b]indole-1-acetic acid. It is a white crystalline compound, insoluble in water but soluble in alcohols, chloroform, dimethyl sulfoxide, and aqueous polyethylene glycol. It will be provided as 200 mg tablets. The inactive ingredients contained in each tablet are: cellulase, hydroxypropyl, methylcellulose, lactose, magnesium stearate, polyethylene glycol, polysorbate 80, povidone, sodium starch glycolate, and titanium dioxide. Subjects will be prescribed 800 mg/per day of etodolac in divided daily dosages.

### Exemplary Dosage and Administration

In one aspect, doses are given in 20, 40 or 80 mg tablets on a tid basis (an exemplary protocol of the invention). In one aspect, doses for propranolol will be started at 40 mg at 8 am, 20 mg at 2 pm, and 20 mg at bedtime (an exemplary protocol of the invention). In one aspect, if after 3 weeks the subject shows no improvement or has not obtained a 20% reduction in heart rate, without decreasing heart rate below 60 bpm or blood pressure below 90/60, doses should be adjusted to 80 mg at 8 am, 40 mg at 2 pm, and 40 mg at bedtime.

In one aspect, doses of etodolac are given at 200 mg at 8 am, 200 mg at 2 pm, and 400 mg at bedtime. These dosage regimens are governed by the investigators observations noted in the metabolic changes occurring in the cardiovascular and neuroendocrine systems.

Duration of therapy: Treatment will be given for a maximum of 6 weeks.

Concomitant Medications: The following concomitant medications will not be allowed during the clinical trial:
- No corticosteroids unless used intermittently as part of a pre-chemotherapy
- No estrogens
- No progestins or other steroids
- No oral anticoagulants
- No anticonvulsants
- No thioridazine (Mellaril^{®})

### Study C

This study is an open pilot clinical trial to evaluate the effect of propranolol in combination with etodolac to evaluate the influence of these compounds on weight and quality of life in subjects diagnosed with metastatic cancer. Another goal of this study is to determine the effect of 6-week treatment of combination therapy of a beta-blocker with an NSAID on resting energy expenditure (REE), weight, inflammatory markers and quality of life.

### Materials and Methods

Age: 18 years and over
Sex: Male and Female
Diagnosis: Metastatic Cancer
Trial sample: A total of 20 subjects will participate in this trial. Recruitment period for the trial is 6 months. The overall duration should not exceed 10 months.

### Inclusion Criteria

- Subject having histologically or cytologically confirmed malignancy with a life expectancy of 3 months minimum
- Subjects demonstrating weight loss of minimum of > 5 pounds (2.3 kg) within 2 months prior to trial recruitment and/or an estimated caloric intake of less than 20 calories/kg daily
- Subjects with a Body Mass Index no greater than 30
- Negative pregnancy test (if applicable) [this test must be done on female patients of childbearing age before they can enroll?]
- Able to give Informed Consent
- Subjects are able to swallow medication tablets
- Subjects are able to ingest food or food supplements
- Subjects who are not on or who have not taken beta-blockers for more than one-month prior to entry into this trial
- Subj ects who have not received radiation therapy for at least 2 weeks prior to entry into this trial
- Subjects who have not received Megestrol acetate or Marinol within 2 months prior to trial entry

### Exclusion Criteria

- Contraindication to beta-blockers
- History of hypersensitivity reaction to propranolol or etodolac
- A history of myocardial infraction within the past 3 months
- Evidence of congestive heart failure
- Unstable angina
- Present history of pulmonary edema, Chronic Obstructive Pulmonary Disease (COPD), asthma or bronchospastic diseases
- History of GI bleeding
- Unstable diabetes
- History of psychiatric disorders other than depression
- Subj ects with a history of marijuana use

### Exemplary Study Medications (exemplary combinations of the invention):

Each subject will be administered Propranolol in combination with Etodolac, an exemplary therapeutic combination of the invention. In one aspect, propranolol is prescribed starting with doses of 40 mg per day up to 160 mg per day. In one aspect, doses are given in 20, 40 or 80 mg tablets on a bid basis.

### Exemplary Dosage Range (exemplary dosages of the invention):

Propranolol 40 mg to 160 mg per day;
Etodolac 800 mg per day.

### Dose Administration (exemplary administration regimens of the invention)

In one aspect, doses for propranolol are adjusted up to three times to a maximum of 160 mg per day in order to obtain a 20% reduction in heart rate without decreasing heart rate below 60 bpm or blood pressure below 90/60. Doses of Etodolac will be fixed at 400 mg bid.

Concomitant Medications: The following concomitant medications will not be allowed during the clinical trial:
No corticosteroids unless used intermittently as part of a pre-chemotherapy
   No estrogens
   No progestins or other steroids
   No oral anticoagulants
   No anticonvulsants
   No thioridazine (Mellaril^{®})

### REFERENCES

The following references are also hereby incorporated herein in their entirety:
(1989). "Parenteral nutrition in patients receiving cancer chemotherapy. American College of Physicians." Ann Intern Med 110(9): 734-6.
(1991). "Perioperative total parenteral nutrition in surgical patients. The Veterans Affairs Total Parenteral Nutrition Cooperative Study Group." N Engl J Med 325(8): 525-32.
Balkwill, F. and A. Mantovani (2001). "Inflammation and cancer: back to Virchow?" Lancet 357(9255): 539-45.
Banta, S., T. Yokoyama, et al. (2004). "Quantitative effects of thermal injury and insulin on the metabolism of the skeletal muscle using the perfused rat hindquarter preparation." Biotechnol Bioeng 88(5): 613-29.
Barber, M. D. and K. C. Fearon (2001). "Tolerance and incorporation of a high-dose eicosapentaenoic acid diester emulsion by patients with pancreatic cancer cachexia." Lipids 36(4): 347-51.
Barber, M. D., K. C. Fearon, et al. (2001). "Effect of a fish oil-enriched nutritional supplement on metabolic mediators in patients with pancreatic cancer cachexia." Nutr Cancer 40(2): 118-24.
Barber, M. D., J. A. Ross, et al. (1999). "Fish oil-enriched nutritional supplement attenuates progression of the acute-phase response in weight-losing patients with advanced pancreatic cancer." J Nutr 129(6): 1120-5.
Barber, M. D., J. A. Ross, et al. (1999). "The effect of an oral nutritional supplement enriched with fish oil on weight-loss in patients with pancreatic cancer." Br J Cancer 81(1): 80-6.
Barton, B. E. (2001). "IL-6-like cytokines and cancer cachexia: consequences of chronic inflammation." Immunol Res 23(1): 41-58.
Basaria, S., J. T. Wahlstrom, et al. (2001). "Clinical review 138: Anabolic-androgenic steroid therapy in the treatment of chronic diseases." J Clin Endocrinol Metab 86(11): 5108-17.
Berg, M., D. L. Fraker, et al. (1994). "Characterization of differentiation factor/leukaemia inhibitory factor effect on lipoprotein lipase activity and mRNA in 3T3-L1 adipocytes." Cytokine 6(4): 425-32.
Bosaeus, I., P. Daneryd, et al. (2001). "Dietary intake and resting energy expenditure in relation to weight loss in unselected cancer patients." Int J Cancer 93(3): 380-3.
Bruera, E., S. Ernst, et al. (1998). "Effectiveness of megestrol acetate in patients with advanced cancer: a randomized, double-blind, crossover study." Cancer Prev Control 2(2): 74-8.
Bruera, E., K. Macmillan, et al. (1990). "A controlled trial of megestrol acetate on appetite, caloric intake, nutritional status, and other symptoms in patients with advanced cancer." Cancer 66(6): 1279-82.
Busquets, S., N. Carbo, et al. (2001). "Hyperlipemia: a role in regulating UCP3 gene expression in skeletal muscle during cancer cachexia?" FEBS Lett 505(2): 255-8.
Busquets, S., M. T. Figueras, et al. (2004). "Anticachectic effects of formoterol: a drug for potential treatment of muscle wasting." Cancer Res 64(18): 6725-31.
Candelore, M. R., L. Deng, et al. (1999). "Potent and selective human beta(3)-adrenergic receptor antagonists." J Pharmacol Exp Ther 290(2): 649-55.
Carbo, N., J. Lopez-Soriano, et al. (1997). "Comparative effects of beta2-adrenergic agonists on muscle waste associated with tumor growth." Cancer Lett 115(1): 113-8.
Castan, I., P. Valet, et al. (1994). "Antilipolytic effects of alpha 2-adrenergic agonists, neuropeptide Y, adenosine, and PGE1 in mammal adipocytes." Am J Physiol 266(4 Pt 2): R1141-7.
Chance, W. T., L. Cao, et al. (1991). "Clenbuterol plus acivicin decrease tumor growth and increase muscle mass in rats maintained on total parenteral nutrition." Am J Surg 161(1): 51-6.
Chlebowski, R. T., L. Bulcavage, et al. (1987). "Hydrazine sulfate in cancer patients with weight loss. A placebo-controlled clinical experience." Cancer 59(3): 406-10.
Cohen, M. L., W. Bloomquist, et al. (1999). "Aryl propanolamines: comparison of activity at human beta3 receptors, rat beta3 receptors and rat atrial receptors mediating tachycardia." Br J Pharmacol 126(4): 1018-24.
Conraads, V. M., J. M. Bosmans, et al. (2002). "Chronic heart failure: an example of a systemic chronic inflammatory disease resulting in cachexia." Int J Cardiol 85(1): 33-49.
Costelli, P., M. Llovera, et al. (1995). "Enhanced leucine oxidation in rats bearing an ascites hepatoma (Yoshida AH-130) and its reversal by clenbuterol." Cancer Lett 91(1): 73-8.
Davis, T. W., B. S. Zweifel, et al. (2004). "Inhibition of cyclooxygenase-2 by celecoxib reverses tumor-induced wasting." J Pharmacol Exp Ther 308(3): 929-34.
De Conno, F., C. Martini, et al. (1998). "Megestrol acetate for anorexia in patients with far-advanced cancer: a double-blind controlled clinical trial." Eur J Cancer 34(11): 1705-9.
Della Cuna, G. R., A. Pellegrini, et al. (1989). "Effect of methylprednisolone sodium succinate on quality of life in preterminal cancer patients: a placebo-controlled, multicenter study. The Methylprednisolone Preterminal Cancer Study Group." Eur J Cancer Clin Oncol 25(12): 1817-21.
Dezube, B. J., J. L. Fridovich-Keil, et al. (1990). "Pentoxifylline and wellbeing in patients with cancer." Lancet 335(8690): 662.
Drott, C., H. Persson, et al. (1989). "Cardiovascular and metabolic response to adrenaline infusion in weight-losing patients with and without cancer." Clin Physiol 9(5): 427-39.
Emery, P. W., R. H. Edwards, et al. (1984). "Protein synthesis in muscle measured in vivo in cachectic patients with cancer." Br Med J (Clin Res Ed) 289(6445): 584-6.
Fearon, K. C. (1992). "The Sir David Cuthbertson Medal Lecture 1991. The mechanisms and treatment of weight loss in cancer." Proc Nutr Soc 51(2): 251-65.
Fearon, K. C. and A. G. Moses (2002). "Cancer cachexia." Int J Cardiol 85(1): 73-81.
Fearon, K. C., M. F. Von Meyenfeldt, et al. (2003). "Effect of a protein and energy dense N-3 fatty acid enriched oral supplement on loss of weight and lean tissue in cancer cachexia: a randomised double blind trial." Gut 52(10): 1479-86.
Feliu, J., M. Gonzalez-Baron, et al. (1992). "Usefulness of megestrol acetate in cancer cachexia and anorexia. A placebo-controlled study." Am J Clin Oncol 15(5): 436-40.
Gambardella, A., R. Tortoriello, et al. (1999). "Intralipid infusion combined with propranolol administration has favorable metabolic effects in elderly malnourished cancer patients." Metabolism 48(3): 291-7.
Gauthier, C., G. Tavernier, et al. (1999). "Interspecies differences in the cardiac negative inotropic effects of beta(3)-adrenoceptor agonists." J Pharmacol Exp Ther 290(2): 687-93.
Gilman, A. (2001). The Pharmacological Basis of Therapeutics. New York, McGraw-Hill.
Gogos, C. A., P. Ginopoulos, et al. (1998). "Dietary omega-3 polyunsaturated fatty acids plus vitamin E restore immunodeficiency and prolong survival for severely ill patients with generalized malignancy: a randomized control trial." Cancer 82(2): 395-402.
Goldberg, R. M., C. L. Loprinzi, et al. (1995). "Pentoxifylline for treatment of cancer anorexia and cachexia? A randomized, double-blind, placebo-controlled trial." J Clin Oncol 13(11): 2856-9.
Guttridge, D. C., M. W. Mayo, et al. (2000). "NF-kappaB-induced loss of MyoD messenger RNA: possible role in muscle decay and cachexia." Science 289(5488): 2363-6.
Hansell, D. T., J. W. Davies, et al. (1986). "The relationship between resting energy expenditure and weight loss in benign and malignant disease." Ann Surg 203(3): 240-5.
Hart, D. W., S. E. Wolf, et al. (2002). "Energy expenditure and caloric balance after bum: increased feeding leads to fat rather than lean mass accretion." Ann Surg 235(1): 152-61.
Herndon, D. N., D. W. Hart, et al. (2001). "Reversal of catabolism by beta-blockade after severe burns." N Engl J Med 345(17): 1223-9.
Herndon, D. N., T. T. Nguyen, et al. (1994). "Lipolysis in burned patients is stimulated by the beta 2-receptor for catecholamines." Arch Surg 129(12): 1301-4; discussion 1304-5.
Herndon, D. N. and R. G. Tompkins (2004). "Support of the metabolic response to burn injury." Lancet 363(9424): 1895-902.
Hogarth, M. B., R. Gallimore, et al. (1997). "Acute phase proteins, C-reactive protein and serum amyloid A protein, as prognostic markers in the elderly inpatient." Age Ageing 26(2): 153-8.
Hryniewicz, K., A. S. Androne, et al. (2003). "Partial reversal of cachexia by beta-adrenergic receptor blocker therapy in patients with chronic heart failure." J Card Fail 9(6): 464-8.
Hyltander, A., P. Daneryd, et al. (2000). "Beta-adrenoceptor activity and resting energy metabolism in weight losing cancer patients." Eur J Cancer 36(3): 330-4.
Hyltander, A., C. Drott, et al. (1991). "Elevated energy expenditure in cancer patients with solid tumours." Eur J Cancer 27(1): 9-15.
Hyltander, A., U. Korner, et al. (1993). "Evaluation of mechanisms behind elevated energy expenditure in cancer patients with solid tumours." Eur J Clin Invest 23(1): 46-52.
Inui, A. (2002). "Cancer anorexia-cachexia syndrome: current issues in research and management." CA Cancer J Clin 52(2): 72-91.
Jatoi, A., H. E. Windschitl, et al. (2002). "Dronabinol versus megestrol acetate versus combination therapy for cancer-associated anorexia: a North Central Cancer Treatment Group study." J Clin Oncol 20(2): 567-73.
Kardinal, C. G., C. L. Loprinzi, et al. (1990). "A controlled trial of cyproheptadine in cancer patients with anorexia and/or cachexia." Cancer 65(12): 2657-62.
Klein, S. and R. R. Wolfe (1990). "Whole-body lipolysis and triglyceride-fatty acid cycling in cachectic patients with esophageal cancer." J Clin Invest 86(5): 1403-8.
Lepor, H., W. Zhang, et al. (1994). "A comparison of the binding and functional properties of alpha-1 adrenoceptors and area density of smooth muscle in the human, canine and rat prostates." J Pharmacol Exp Ther 270(2): 722-7.
Lissoni, P., F. Paolorossi, et al. (1996). "Is there a role for melatonin in the treatment of neoplastic cachexia?" Eur J Cancer 32A(8): 1340-3.
Lopez-Soriano, J., N. Carbo, et al. (1996). "alpha-Adrenergic receptors may contribute to the hypertriglyceridemia associated with tumour growth." Cancer Lett 110(1-2): 213-6.
Loprinzi, C. L., N. M. Ellison, et al. (1990). "Controlled trial of megestrol acetate for the treatment of cancer anorexia and cachexia." J Natl Cancer Inst 82(13): 1127-32.
Loprinzi, C. L., R. M. Goldberg, et al. (1994). "Placebo-controlled trial of hydrazine sulfate in patients with newly diagnosed non-small-cell lung cancer." J Clin Oncol 12(6): 1126-9.
Loprinzi, C. L., J. W. Kugler, et al. (1999). "Randomized comparison of megestrol acetate versus dexamethasone versus fluoxymesterone for the treatment of cancer anorexia/cachexia." J Clin Oncol 17(10): 3299-306.
Loprinzi, C. L., S. A. Kuross, et al. (1994). "Randomized placebo-controlled evaluation of hydrazine sulfate in patients with advanced colorectal cancer." J Clin Oncol 12(6): 1121-5.
Loprinzi, C. L., D. J. Schaid, et al. (1993). "Body-composition changes in patients who gain weight while receiving megestrol acetate." J Clin Oncol 11(1): 152-4.
Lundholm, K., P. Daneryd, et al. (2004). "Evidence that long-term COX-treatment improves energy homeostasis and body composition in cancer patients with progressive cachexia." Int J Oncol 24(3): 505-12.
Lundholm, K., J. Gelin, et al. (1994). "Anti-inflammatory treatment may prolong survival in undernourished patients with metastatic solid tumors." Cancer Res 54(21): 5602-6.
MacDonald, N. (2003). "Is there evidence for earlier intervention in cancer-associated weight loss?" J Support Oncol 1(4): 279-86.
MacDonald, N., A. M. Easson, et al. (2003). "Understanding and managing cancer cachexia." J Am Coll Surg 197(1): 143-61.
McCarthy, D. O., P. Whitney, et al. (2004). "Indomethacin and ibuprofen preserve gastrocnemius muscle mass in mice bearing the colon-26 adenocarcinoma." Res Nurs Health 27(3): 174-84.
McMillan, D. C., E. Leen, et al. (1995). "Effect of extended ibuprofen administration on the acute phase protein response in colorectal cancer patients." Eur J Surg Oncol 21(5): 531-4.
McMillan, D. C., P. O'Gorman, et al. (1997). "A pilot study of megestrol acetate and ibuprofen in the treatment of cachexia in gastrointestinal cancer patients." Br J Cancer 76(6): 788-90.
McMillan, D. C., S. J. Wigmore, et al. (1999). "A prospective randomized study of megestrol acetate and ibuprofen in gastrointestinal cancer patients with weight loss." Br J Cancer 79(3-4): 495-500.
Megeney, L. A., B. Kablar, et al. (1996). "MyoD is required for myogenic stem cell function in adult skeletal muscle." Genes Dev 10(10): 1173-83.
Moldawer, L. L. and E. M. Copeland, 3rd (1997). "Proinflammnatory cytokines, nutritional support, and the cachexia syndrome: interactions and therapeutic options." Cancer 79(9): 1828-39.
Newman-Tancredi, A., J. P. Nicolas, et al. (1998). "Actions of alpha2 adrenoceptor ligands at alpha2A and 5-HT1A receptors: the antagonist, atipamezole, and the agonist, dexmedetomidine, are highly selective for alpha2A adrenoceptors." Naunyn Schmiedebergs Arch Pharmacol 358(2): 197-206.
Ohsaka, Y., T. Murakami, et al. (1998). "Comparison of atypical beta3-adrenoceptor agonists with their respective metabolic activities in rat white adipocytes." Jpn J Pharmacol 77(1): 41-51.
Palmer, R., R. Weiss, et al. (2003). "Effects of nabumetone, celecoxib, and ibuprofen on blood pressure control in hypertensive patients on angiotensin converting enzyme inhibitors." Am J Hypertens 16(2): 135-9.
Piffar, P. M., R. Fernandez, et al. (2003). "Naproxen, clenbuterol and insulin administration ameliorates cancer cachexia and reduce tumor growth in Walker 256 tumor-bearing rats." Cancer Lett 201(2): 139-48.
Popiela, T., R. Lucchi, et al. (1989). "Methylprednisolone as palliative therapy for female terminal cancer patients. The Methylprednisolone Female Preterminal Cancer Study Group." Eur J Cancer Clin Oncol 25(12): 1823-9.
Preston, T., K. C. Fearon, et al. (1995). "Effect of ibuprofen on the acute-phase response and protein metabolism in patients with cancer and weight loss." Br J Surg 82(2): 229-34.
Ross, J. A. and K. C. Fearon (2002). "Eicosanoid-dependent cancer cachexia and wasting." Curr Opin Clin Nutr Metab Care 5(3): 241-8.
Rowland, K. M., Jr., C. L. Loprinzi, et al. (1996). "Randomized double-blind placebo-controlled trial of cisplatin and etoposide plus megestrol acetate/placebo in extensive-stage small-cell lung cancer: a North Central Cancer Treatment Group study." J Clin Oncol 14(1): 135-41.
Salpeter, S., T. Ormiston, et al. (2002). "Cardioselective beta-blockers for chronic obstructive pulmonary disease." Cochrane Database of Systematic Reviews (Issue 1): CD003566.
Sandstrom, R., C. Drott, et al. (1993). "The effect of postoperative intravenous feeding (TPN) on outcome following major surgery evaluated in a randomized study." Ann Surg 217(2): 185-95.
Scott, H. R., D. C. McMillan, et al. (2003). "A prospective study of the impact of weight loss and the systemic inflammatory response on quality of life in patients with inoperable non-small cell lung cancer." Lung Cancer 40(3): 295-9.
Scott, H. R., D. C. McMillan, et al. (2002). "The systemic inflammatory response, weight loss, performance status and survival in patients with inoperable non-small cell lung cancer." Br J Cancer 87(3): 264-7.
Shaw, J. H. and R. R. Wolfe (1987). "Fatty acid and glycerol kinetics in septic patients and in patients with gastrointestinal cancer. The response to glucose infusion and parenteral feeding." Ann Surg 205(4): 368-76.
Sheen-Chen, S. M., W. J. Chen, et al. (1997). "Serum concentration of tumor necrosis factor in patients with breast cancer." Breast Cancer Res Treat 43(3): 211-5.
Simons, J. P., N. K. Aaronson, et al. (1996). "Effects of medroxyprogesterone acetate on appetite, weight, and quality of life in advanced-stage non-hormone-sensitive cancer: a placebo-controlled multicenter study." J Clin Oncol 14(4): 1077-84.
Simons, J. P., A. M. Schols, et al. (1999). "Weight loss and low body cell mass in males with lung cancer: relationship with systemic inflammation, acute-phase response, resting energy expenditure, and catabolic and anabolic hormones." Clin Sci (Lond) 97(2): 215-23.
Simons, J. P., A. M. Schols, et al. (1998). "Effects of medroxyprogesterone acetate on food intake, body composition, and resting energy expenditure in patients with advanced, nonhormone-sensitive cancer: a randomized, placebo-controlled trial." Cancer 82(3): 553-60.
Smith, C. and M. Teitler (1999). "Beta-blocker selectivity at cloned human beta 1-and beta 2-adrenergic receptors." Cardiovasc Drugs Ther 13(2): 123-6.
Stallion, A., T. Foley-Nelson, et al. (1993). "Effects of increased beta 2-agonist dose in tumor-bearing animals." Nutr Cancer 20(3): 251-60.
Stallion, A., F. S. Zhang, et al. (1991). "Reversal of cancer cachexia in rats by cimaterol and supplemental nutrition." Surgery 110(4): 678-84.
Struzik, Z. R., J. Hayano, et al. (2004). "1/f scaling in heart rate requires antagonistic autonomic control." Phys Rev E Stat Nonlin Soft Matter Phys 70(5 Pt 1): 050901.
Szabo, K. (1979). "Clinical experiences with beta adrenergic blocking therapy on burned patients." Scand J Plast Reconstr Surg 13(1): 211-5.
Tchekmedyian, N. S., M. Hickman, et al. (1992). "Megestrol acetate in cancer anorexia and weight loss." Cancer 69(5): 1268-74.
Tisdale, M. J. (1993). "Cancer cachexia." Anticancer Drugs 4(2): 115-25.
Tisdale, M. J. (1996). "Inhibition of lipolysis and muscle protein degradation by EPA in cancer cachexia." Nutrition 12(1 Suppl): S31-3.
Tisdale, M. J. (2000). "Biomedicine. Protein loss in cancer cachexia." Science 289(5488): 2293-4.
Weir, M. R., R. S. Sperling, et al. (2003). "Selective COX-2 inhibition and cardiovascular effects: a review of the rofecoxib development program." Am Heart J 146(4): 591-604.
Wigmore, S. J., M. D. Barber, et al. (2000). "Effect of oral eicosapentaenoic acid on weight loss in patients with pancreatic cancer." Nutr Cancer 36(2): 177-84.
Wigmore, S. J., J. S. Falconer, et al. (1995). "Ibuprofen reduces energy expenditure and acute-phase protein production compared with placebo in pancreatic cancer patients." Br J Cancer 72(1): 185-8.
Wigmore, S. J., K. C. Fearon, et al. (1997). "Down-regulation of the acute-phase response in patients with pancreatic cancer cachexia receiving oral eicosapentaenoic acid is mediated via suppression of interleukin-6." Clin Sci (Lond) 92(2): 215-21.
Wigmore, S. J., J. A. Ross, et al. (1996). "The effect of polyunsaturated fatty acids on the progress of cachexia in patients with pancreatic cancer." Nutrition 12(1 Suppl): S27-30.
Zhang, H. H., M. Halbleib, et al. (2002). "Tumor necrosis factor-alpha stimulates lipolysis in differentiated human adipocytes through activation of extracellular signal-related kinase and elevation of intracellular cAMP." Diabetes 51(10): 2929-35.

A number of aspects of the invention have been described. Nevertheless, it will be understood that various modifications may be made without departing from the spirit and scope of the invention. Accordingly, other aspects are within the scope of the following claims.

The present invention also relates to the following aspects:
1. A therapeutic combination comprising at least one member of a first group and at least one member of a second group;
   wherein members of the first group are selected from the group consisting of beta adrenergic receptor antagonists (beta blockers); and
   members of the second group are selected from the group consisting of angiotensin-converting enzyme (ACE) inhibitors, angiotensin receptor blockers (ARBs), non-steroidal anti-inflammatory drugs (NSAID), anabolic steroids, natural oils and fatty acids and a combination thereof.
2. The therapeutic combination of aspect 1, comprising:
   at least one beta adrenergic receptor antagonist (beta blocker) and at least one angiotensin-converting enzyme (ACE) inhibitors;
   at least one beta adrenergic receptor antagonist (beta blocker) and at least one non-steroidal anti-inflammatory drug (NSAID);
   at least one beta adrenergic receptor antagonist (beta blocker) and at least one angiotensin receptor blocker (ARB);
   at least one beta adrenergic receptor antagonist (beta blocker) and at least one anabolic steroid; or
   at least one beta adrenergic receptor antagonist (beta blocker) and at least one natural oil or fatty acid.
3. The therapeutic combination of aspect 1, further comprising at least one member of a third group, wherein the member of the third group is a different composition than the selected member of the first or second group, and members of the third group are selected from the group consisting of angiotensin-converting enzyme (ACE) inhibitors, angiotensin receptor blockers (ARBs), non-steroidal anti-inflammatory drugs (NSAIDs), anabolic steroids, natural oils and fatty acids and a combination thereof.
4. The therapeutic combination of aspect 3, comprising:
   at least one beta adrenergic receptor antagonist (beta blocker), at least one angiotensin-converting enzyme (ACE) inhibitor and at least one angiotensin receptor blocker (ARB);
   at least one beta adrenergic receptor antagonist (beta blocker), at least one angiotensin-converting enzyme (ACE) inhibitor and at least one non-steroidal anti-inflammatory drug (NSAID);
   at least one beta adrenergic receptor antagonist (beta blocker), at least one angiotensin-converting enzyme (ACE) inhibitor and at least one anabolic steroid;
   at least one beta adrenergic receptor antagonist (beta blocker), at least one angiotensin-converting enzyme (ACE) inhibitor and at least one natural oil or fatty acid;
   at least one beta adrenergic receptor antagonist (beta blocker), at least one angiotensin receptor blocker (ARB) and at least one non-steroidal anti-inflammatory drug (NSAID);
   at least one beta adrenergic receptor antagonist (beta blocker), at least one angiotensin receptor blocker (ARB) and at least one anabolic steroid;
   at least one beta adrenergic receptor antagonist (beta blocker), at least one angiotensin receptor blocker (ARB) and at least one natural oil or fatty acid;
   at least one beta adrenergic receptor antagonist (beta blocker), at least one non-steroidal anti-inflammatory drug (NSAID) and at least one anabolic steroid;
   at least one beta adrenergic receptor antagonist (beta blocker), at least one non-steroidal anti-inflammatory drug (NSAID) and at least one natural oil or fatty acid; or
   at least one beta adrenergic receptor antagonist (beta blocker), at least one anabolic steroid and at least one natural oil or fatty acid.
5. The therapeutic combination of aspect 3, further comprising at least one member of a fourth group, wherein the member of the fourth group is a different composition than the selected member of the first, second or third group, and members of the fourth group are selected from the group consisting of angiotensin-converting enzyme (ACE) inhibitors, angiotensin receptor blockers (ARBs), non-steroidal anti-inflammatory drugs (NSAIDs), anabolic steroids, natural oils and fatty acids and a combination thereof.
6. The therapeutic combination of aspect 5, further comprising at least one member of a fifth group, wherein the member of the fifth group is a different composition than the selected member of the first, second, third or fourth group, and members of the fifth group are selected from the group consisting of angiotensin-converting enzyme (ACE) inhibitors, angiotensin receptor blockers (ARBs), non-steroidal anti-inflammatory drugs (NSAIDs), anabolic steroids, natural oils and fatty acids and a combination thereof.
7. The therapeutic combination of aspect 5, further comprising at least one member of a sixth group, wherein the member of the sixth group is a different composition than the selected member of the first, second, third, fourth or fifth group, and members of the sixth group are selected from the group consisting of angiotensin-converting enzyme (ACE) inhibitors, angiotensin receptor blockers (ARBs), non-steroidal anti-inflammatory drugs (NSAIDs), anabolic steroids, natural oils and fatty acids and a combination thereof.
8. The therapeutic combination of aspect 1, wherein the at least one member of the first group and the at least one member of the second group are formulated as separate compositions.
9. The therapeutic combination of aspect 1, wherein the at least one member of the first group and the at least one member of the group are formulated in the same composition.
10. The therapeutic combination of any of aspects 3 to 7, wherein each member of each selected group is formulated as a separate composition.
11. The therapeutic combination of any of aspects 3 to 7, wherein all selected members are formulated in the same composition.
12. A pharmaceutical composition comprising the therapeutic combination of any of aspects 1 to 11.
13. The pharmaceutical composition of aspect 12, further comprising a pharmaceutically acceptable excipient.
14. The pharmaceutical composition of aspect 12 or aspect 13, wherein the therapeutic combination or pharmaceutical composition is formulated or manufactured as a feed, a food, a liquid, an elixir, an aerosol, a spray, a powder, a tablet, a pill, a capsule, a gel, a geltab, a nanosuspension, a nanoparticle, a microgel or a suppository.
15. The therapeutic combination of any of aspects 1 to 11, or the pharmaceutical composition of any of aspects 12 to 15, wherein the beta adrenergic receptor antagonist (beta blocker) comprises atenolol, nadolol, metoprolol, propranolol, carteolol, carvedolol, labetalol, oxprenolol, penbutolol, pindolol, sotalol, timolol or a combination thereof.
16. The therapeutic combination of any of aspects 1 to 11, or the pharmaceutical composition of any of aspects 12 to 15, wherein the angiotensin-converting enzyme (ACE) inhibitor comprises benazepril, captopril, cilazapril, enalapril, enalaprilat, fosinopril, imidapril, lisinopril, moexipril, perindopril, quinapril, ramipril, trandolapril or a combination thereof.
17. The therapeutic combination of any of aspects 1 to 11, or the pharmaceutical composition of any of aspects 12 to 15, wherein the angiotensin receptor blocker comprises candesartan, eprosartan, irbesartan, losartan, olmesartan, telmisartan, valsartan or a combination thereof.
18. The therapeutic combination of any of aspects 1 to 11, or the pharmaceutical composition of any of aspects 12 to 15, wherein the non-steroidal anti-inflammatory drugs (NSAID) comprises aspirin, diclofenac; diflunisal, etodolac, fenoprofen, flurbiprofen, ibuprofen, indomethacin, ketoprofen; ketorolac, meclofenamate, mefenamic acid, meloxicam, nabumetone, naproxen, oxaprozin, piroxicam, salsalate, sulindac, tolmetin, a COX-2-selective inhibitor or a combination thereof,
   wherein optionally the COX-2-selective inhibitor comprises celecoxib rofecoxib, etoricoxib, valdecoxib, parecoxib, meloxicam or lumiracoxib.
19. The therapeutic combination of any of aspects 1 to 11, or the pharmaceutical composition of any of aspects 12 to 15, wherein the anabolic steroid comprises andarine, ethylestrenol, mesterolone, methandrostenolone, methenolone, methyltestosterone, oxandrolone, oxymetholone stanozolol, boldenone, hexoxymestrolum, methandrostenolone, methenolone enanthate, nandrolone decanoate, nandrolone phenproprionate, stanozolol, stenbolone, testosterone cypionate, testosterone enanthate, testosteron, testosterone nicotinate, therobolin, trenbolone, trenbolone, trophobolene or a combination thereof.
20. The therapeutic combination of any of aspects 1 to 11, or the pharmaceutical composition of any of aspects 12 to 15, wherein the natural oil or fatty acid comprises an omega-3 fatty acid, a fish oil, a long-chain polyunsaturated fatty acid, an n-3 and/or n-6 highly unsaturated fatty acid, eicosapentaenoic acid (EPA), docosahexaenoic acid (DHA) or a combination thereof.
21. The therapeutic combination of any of aspects 1 to 11, or the pharmaceutical composition of any of aspects 12 to 15, formulated for use as a medicament in the treatment of chronic systemic inflammatory stress; bums, chronic obstructive pulmonary disease; congestive heart failure; chronic kidney disease; surgery; cancer; sepsis; ageing; acute respiratory distress syndrome; acute lung injury; infection; a CNS disorder or injury; anemia; immunosuppression; insulin resistance; anorexia; anxiety; sleep disturbances; weakness; fatigue; gastrointestinal distress; sleep disturbances; wake disturbances; pain; listlessness; shortness of breath; lethargy; depression; malaise; or, a combination thereof.
22. The therapeutic combination of any of aspects 1 to 11, or the pharmaceutical composition of any of aspects 12 to 15, formulated for use in the treatment or amelioration of a condition or disease comprising a chronic Systemic Inflammatory Response State (SIRS).
23. The therapeutic combination of any of aspects 1 to 11, for use as a medicament.
24. The therapeutic combination of any of aspects 1 to 11, for use in the treatment of cachexia or anorexia.
25. The therapeutic combination of aspect 24, wherein the cachexia is defined as at least two of the symptoms selected from the group consisting of: 1) a hyper-inflammatory state, 2) altered hormone levels and cytokine levels; 3) increased heart rate variability; 4) weight loss, and 5) increased heart rate, wherein optionally the increased heart rate is having a sustained elevated heart rate of at least about 6 bpm.
26. The therapeutic combination of aspect 25, wherein the cachexia is defined by an individual having at least a sustained elevated heart rate of at least about 6 bpm and weight loss.
27. Use of the therapeutic combination of any of aspects 1 to 11, in the manufacture of a medicament or pharmaceutical composition for treating, ameliorating or preventing a trauma, condition or disease comprising: a chronic Systemic Inflammatory Response State (SIRS); chronic systemic inflammatory stress; bums, chronic obstructive pulmonary disease; congestive heart failure; chronic kidney disease; surgery; cancer; sepsis; ageing; acute respiratory distress syndrome; acute lung injury; infection; a CNS disorder or injury; anemia; immunosuppression; insulin resistance; anorexia; anxiety; sleep disturbances; weakness; fatigue; gastrointestinal distress; sleep disturbances; wake disturbances; pain; listlessness; shortness of breath; lethargy; depression; malaise; or, a combination thereof.
28. The use of aspect 27, wherein the trauma, condition or disease comprises a maladaptive nutritional state secondary to the SIRS.
29. The use of aspect 28, wherein the maladaptive nutritional state comprises cachexia, and optionally the cachexia comprises cachexia secondary to cancer.
30. The use of aspect 29, wherein the cachexia is defined as at least two of the symptoms selected from the group consisting of: 1) a hyper-inflammatory state, 2) altered hormone levels and cytokine levels; 3) increased heart rate variability; 4) weight loss, and 5) increased heart rate, wherein optionally the increased heart rate is having a sustained elevated heart rate of at least about 6 bpm.
31. The use of aspect 30, wherein the cachexia is defined by an individual having at least a sustained elevated heart rate of at least about 6 bpm and weight loss.
32. The use of aspect 27, wherein the CNS disorder comprises Parkinson's disease or Alzheimer's disease.
33. A method for treating or ameliorating a trauma, condition or disease comprising a chronic Systemic Inflammatory Response State (SIRS), chronic systemic inflammatory stress; bums, chronic obstructive pulmonary disease; congestive heart failure; chronic kidney disease; surgery; cancer; sepsis; ageing; acute respiratory distress syndrome; acute lung injury; infection; a CNS disorder or injury; anemia; immunosuppression; insulin resistance; anorexia; anxiety; sleep disturbances; weakness; fatigue; gastrointestinal distress; sleep disturbances; wake disturbances; pain; listlessness; shortness of breath; lethargy; depression; malaise; or, a combination thereof, the method comprising the steps of:
   (a) providing the therapeutic combination of any of aspects 1 to 11, or the pharmaceutical composition of any of aspects 12 to 15; and,
   (b) administering a therapeutically effective amount of the therapeutic combination of step (a), thereby treating or ameliorating the trauma, condition or disease.
34. The method of aspect 33, wherein the condition or disease comprises a maladaptive nutritional state secondary to the SIRS.
35. The method of aspect 34, wherein the maladaptive nutritional state comprises cachexia or anorexia.
36. The method of aspect 35, wherein the cachexia comprises cachexia secondary to cancer.
37. The method of aspect 35, wherein the cachexia is defined as at least two of the symptoms selected from the group consisting of: 1) a hyper-inflammatory state, 2) altered hormone levels and cytokine levels; 3) increased heart rate variability; 4) weight loss, and 5) increased heart rate, wherein optionally the increased heart rate is having a sustained elevated heart rate of at least about 6 bpm.
38. The method of aspect 36, wherein the cachexia is defined by an individual having at least a sustained elevated heart rate of at least about 6 bpm and weight loss.
39. The method of aspect 33, wherein the therapeutic combination of any of aspects 1 to 11, or the pharmaceutical composition of any of aspects 12 to 15, is administered in single or multiple doses, and optionally the pharmaceutical compositions are packaged in a single or a plurality of packages or packets.
40. The method of aspect 33, wherein the therapeutic combination of any of aspects 1 to 11, or the pharmaceutical composition of any of aspects 12 to 15, is administered intravenously, topically, orally, by inhalation, by infusion, by injection, intraperitoneally, intramuscularly, subcutaneously, intra-aurally, by intra-articular administration, by intra-mammary administration, by topical administration or by absorption through epithelial or mucocutaneous linings.
41. The method of aspect 33, wherein the therapeutic combination or the pharmaceutical composition comprises administration of the beta adrenergic receptor antagonist (beta blocker) intravenously in a dose of about 1 mg/kg, and optionally the beta adrenergic receptor antagonist (beta blocker) comprises atenolol, nadolol, metoprolol, propranolol, carteolol, carvedolol, labetalol, oxprenolol, penbutolol, pindolol, sotalol, timolol or a combination thereof.
42. A kit comprising the therapeutic combination of any of aspects 1 to 11, or the pharmaceutical composition of any of aspects 12 to 15.
43. The kit of aspect 42, comprising at least one blister pack comprising the therapeutic combination of any of aspects 1 to 11, or the pharmaceutical composition of any of aspects 12 to 15.
44. A kit for the treatment, amelioration or prevention of a chronic Systemic Inflammatory Response State (SIRS) or a maladaptive nutritional state in a patient population, the kit comprising the therapeutic combination of any of aspects 1 to 11, or the pharmaceutical composition of any of aspects 12 to 15, and instructions for use of the therapeutic combination or pharmaceutical composition.
45. The kit of aspect 44, wherein the maladaptive nutritional state comprises cachexia, and optionally the cachexia comprises cachexia secondary to cancer.
46. The kit of aspect 45, wherein the cachexia is defined as at least two of the symptoms selected from the group consisting of: 1) a hyper-inflammatory state, 2) altered hormone levels and/or cytokine levels; 3) increased heart rate variability; 4) weight loss, and 5) sustained increased heart rate, wherein optionally the sustained increased heart rate is having a sustained elevated heart rate of at least about 6 bpm.
47. The kit of aspect 46, wherein the cachexia is defined by an individual having at least a sustained elevated heart rate of at least about 6 bpm and weight loss.
48. A kit for the treatment, amelioration or prevention of a CNS disorder, wherein the kit comprises the therapeutic combination of any of aspects 1 to 11, or the pharmaceutical composition of any of aspects 12 to 15.
49. The kit of aspect 48, wherein the CNS disorder comprises Parkinson's disease or Alzheimer's disease.
50. The therapeutic combination of any of aspects 1 to 11, or the pharmaceutical composition of any of aspects 12 to 15, formulated for administration intravenously, topically, orally, by inhalation, by infusion, by injection, intraperitoneally, intramuscularly, subcutaneously, intra-aurally, for intra-articular administration, for intra-mammary administration, for topical administration or for absorption through epithelial or mucocutaneous linings.
51. The therapeutic combination of any of aspects 1 to 11, or the pharmaceutical composition of any of aspects 12 to 15, formulated for chrono-dosing.
52. The therapeutic combination of any of aspects 1 to 11, or the pharmaceutical composition of any of aspects 12 to 15, formulated for administration once a day, b.i.d. or t.i.d.
53. The therapeutic combination of any of aspects 1 to 11, or the pharmaceutical composition of any of aspects 12 to 15, formulated and dosaged as set forth in any one of exemplary ingredient combinations 1 to 90.
54. A product of manufacture comprising a blister package, a clamshell, a tray or a shrink wrap comprising a therapeutic combination of any of aspects 1 to 11, or the pharmaceutical composition of any of aspects 12 to 15.
55. The product of manufacture of aspect 54, comprising at least one beta adrenergic receptor antagonist (a beta blocker) and an angiotensin-converting enzyme (ACE) inhibitor, an angiotensin receptor blocker (ARB), a non-steroidal anti-inflammatory drug (a NSAID), an anabolic steroid, a natural oil, a fatty acid or a combination thereof.
56. The product of manufacture of aspect 55, comprising at least one beta adrenergic receptor antagonist (a beta blocker) and at least one non-steroidal anti-inflammatory drug (a NSAID).
57. The product of manufacture of aspect 55, wherein the at least one non-steroidal anti-inflammatory drug (a NSAID) comprises an aspirin, dichlofenac, diflunisal, etodolac, fenoprofen, flurbiprofen, ibuprofen, indomethacin, ketoprofen; ketorolac, meclofenamate, mefenamic acid, meloxicam, nabumetone, naproxen, oxaprozin, piroxicam, salsalate, sulindac, tolmetin, a COX-2-selective inhibitor or a combination thereof.
58. The product of manufacture of aspect 57, wherein the COX-2-selective inhibitor comprises celecoxib rofecoxib, etoricoxib, valdecoxib, parecoxib, meloxicam and/or lumiracoxib.
59. A product of manufacture comprising a blister package, a clamshell, a tray or a shrink wrap comprising a therapeutic combination comprising an atenolol, nadolol, metoprolol, propranolol, carteolol, carvedolol, labetalol, oxprenolol, penbutolol, pindolol, sotalol, timolol or a combination thereof, and at least one non-steroidal anti-inflammatory drug (a NSAID).
60. A method for ameliorating cachexia, anorexia, anorexia-cachexia, stress or anxiety related to a cancer comprising administering to an individual in need thereof a pharmaceutically effective amount of the therapeutic combination of any of aspects 1 to 11, or the pharmaceutical composition of any of aspects 12 to 15.
61. The method of aspect 60, wherein the cancer is a metastatic cancer.
62. The method of aspect 61, wherein the metastatic cancer is a non-lung, non-hematological metastatic cancer.
63. The method of aspect 60, wherein the therapeutic combination comprises at least one beta blocker and at least one non-steroidal anti-inflammatory drug (a NSAID).
64. The method of aspect 63, wherein the at least one beta blocker is an inhibitor of beta1, beta2 or beta3 subclass of beta adrenergic receptor.
65. The method of aspect 63, wherein the at least one beta blocker is propranolol, and optionally the propranolol is INDERAL™.
66. The method of aspect 63, wherein the at least one non-steroidal anti-inflammatory drug is a Cox-1 or a Cox-2 enzyme inhibitor.
67. The method of aspect 66, wherein the Cox-1 or a Cox-2 enzyme inhibitor is celecoxib rofecoxib, etoricoxib, valdecoxib, parecoxib, meloxicam or lumiracoxib.
68. The method of aspect 60, wherein the wherein the therapeutic combination is formulated and administered by intravenous, topical, oral by inhalation, infusion or injection, intraperitoneal, intramuscular, subcutaneous, intra-aural, intra-articular, intra-mammary, topical application on eyes, ears, skin, wounds or bums, by absorption through epithelial or mucocutaneous linings in vaginal epithelial linings, gastrointestinal mucosa routes.
69. A product of manufacture comprising a blister package, a clamshell, a tray or a shrink wrap comprising a therapeutic combination comprising a propranolol and a non-steroidal anti-inflammatory drug (a NSAID).
70. The product of manufacture of aspect 69, wherein the NSAID comprises aspirin, diclofenac; diflunisal, etodolac, fenoprofen, flurbiprofen, ibuprofen, indomethacin, ketoprofen; ketorolac, meclofenamate, mefenamic acid, meloxicam, nabumetone, naproxen, oxaprozin, piroxicam, salsalate, sulindac, tolmetin, a COX-2-selective inhibitor or a combination thereof, wherein optionally the COX-2-selective inhibitor comprises celecoxib rofecoxib, etoricoxib, valdecoxib, parecoxib, meloxicam or lumiracoxib.
71. The therapeutic combination of any of aspects 1 to 11, or the pharmaceutical composition of aspect 12 or aspect 13, formulated for at least two administrations, one in the morning and one in the evening, wherein the dosage schedule provides a relatively higher dose of beta blocker in the morning (the AM) than in the evening, and a relatively higher dose of an anti-anxiety and/or an anti-inflammatory medication in the evening than in the morning.
72. The product of manufacture of aspect 69, wherein the blister package, clamshell, tray or shrink wrap comprises at least two dosages of beta adrenergic receptor antagonist (a beta blocker) drug and at least two dosages of non-steroidal anti-inflammatory drug (a NSAID) and/or anti-anxiety drug, and the drugs are organized or labeled in the blister package, clamshell, tray or shrink wrap for usage by an individual for at least two administrations, one in the morning and one in the evening, wherein the dosage schedule provides a relatively higher dose of beta blocker in the morning (the AM) than in the evening, and a relatively higher dose of an anti-anxiety and/or an anti-inflammatory medication in the evening than in the morning.
73. The product of manufacture of aspect 69, or the therapeutic combination of aspect 57, wherein a dose of propranolol is given in 20 or 40 mg tablets immediate release on a bid basis, and in the first dose week the doses for propranolol are 20 mg in the morning and 20 mg at bedtime, and after 1 week the dosage is adjusted to 20 mg of the immediate release product in the morning and 60 mg of the extended release at bedtime, and optionally if after an additional week the subject shows no improvement or has not obtained a 20% reduction in heart rate, without decreasing heart rate below 60 bpm or blood pressure below 90/60, the dose is adjusted to 40 mg of the immediate release propranolol in the morning and 120 mg of the extended release propranolol at bedtime.
74. The product of manufacture of aspect 69, or the therapeutic combination of aspect 57, wherein doses of metoprolol are given in 25 or 50 mg tablets on a bid basis, and doses for metoprolol are started at 25 mg of the immediate release product in the morning and at bedtime, and after 1 week the dosage is adjusted to 25 mg of the immediate release metoprolol in the morning and 50 mg of the extended release metoprolol at bedtime; and optionally if after an additional week the subject shows no improvement or has not obtained a 20% reduction in heart rate, without decreasing heart rate below 60 bpm or blood pressure below 90/60, the dose is adjusted to 50 mg of the immediate release product in the morning and 100 mg of the extended release product at bedtime.
75. The product of manufacture of aspect 69, or the therapeutic combination of aspect 57, wherein doses of etodolac are given in 200 mg capsules or 500 mg tablets on a bid basis, and doses for etodolac are started at 200 mg in the morning and at bedtime, and after 1 week the dosage are adjusted to 200 mg in the morning and 500 mg at bedtime.
76. A product of manufacture comprising a blister package, a clamshell, a tray or a shrink wrap comprising therapeutic combination of any of aspects 1 to 11, or the pharmaceutical composition of aspect 12 or aspect 13, wherein the therapeutic combination or pharmaceutical composition are formulated for at least two dosage administrations.
77. The product of manufacture of aspect 76, wherein the therapeutic combination or pharmaceutical composition are formulated as one dosage administration in the morning and one dosage administration in the evening.
78. The product of manufacture of aspect 77, wherein the dosage schedule provides a relatively higher dose of one drug in the morning (the AM) than in the evening, and a relatively higher dose of another medication in the evening than in the morning.
79. A therapeutic combination of any of aspects 1 to 11, or the pharmaceutical composition of aspect 12 or aspect 13, further comprising a nutritional supplement.
80. A method for treating, ameliorating or preventing a chronic Systemic Inflammatory Response State (SIRS), wherein a therapeutic combination of any of aspects 1 to 11, or the pharmaceutical composition of aspect 12 or aspect 13, are administered to an individual in need thereof, and the therapeutic combination or the pharmaceutical composition are formulated for at least two administrations, one in the morning and one in the evening, wherein the dosage schedule provides a relatively higher dose of beta blocker in the morning (the AM) than in the evening, and a relatively higher dose of an anti-anxiety and/or an anti-inflammatory medication in the evening than in the morning.
81. The method of aspect 60 or aspect 80, wherein the administration regimen comprises at least two dosages of beta adrenergic receptor antagonist (a beta blocker) drug and at least two dosages of non-steroidal anti-inflammatory drug (a NSAID) and/or anti-anxiety drug, and the drugs are organized or labeled in the blister package, clamshell, tray or shrink wrap for usage by an individual for at least two administrations, one in the morning and one in the evening, wherein the dosage schedule provides a relatively higher dose of beta blocker in the morning (the AM) than in the evening, and a relatively higher dose of an anti-anxiety and/or an anti-inflammatory medication in the evening than in the morning.
82. The method of aspect 60 or aspect 80, wherein the administration regimen comprises doses of propranolol given in 20 or 40 mg tablets immediate release on a bid basis, and in the first dose week the doses for propranolol are 20 mg in the morning and 20 mg at bedtime, and after 1 week the dosage is adjusted to 20 mg of the immediate release product in the morning and 60 mg of the extended release at bedtime, and optionally if after an additional week the subject shows no improvement or has not obtained a 20% reduction in heart rate, without decreasing heart rate below 60 bpm or blood pressure below 90/60, the dose is adjusted to 40 mg of the immediate release propranolol in the morning and 120 mg of the extended release propranolol at bedtime.
83. The method of aspect 60 or aspect 80, wherein the administration regimen comprises doses of metoprolol given in 25 or 50 mg tablets on a bid basis, and doses for metoprolol are started at 25 mg of the immediate release product in the morning and at bedtime, and after 1 week the dosage is adjusted to 25 mg of the immediate release metoprolol in the morning and 50 mg of the extended release metoprolol at bedtime; and optionally if after an additional week the subject shows no improvement or has not obtained a 20% reduction in heart rate, without decreasing heart rate below 60 bpm or blood pressure below 90/60, the dose is adjusted to 50 mg of the immediate release product in the morning and 100 mg of the extended release product at bedtime.
84. The method of aspect 60 or aspect 80, wherein the administration regimen comprises doses of etodolac are given in 200 mg capsules or 500 mg tablets on a bid basis, and doses for etodolac are started at 200 mg in the morning and at bedtime, and after 1 week the dosage are adjusted to 200 mg in the morning and 500 mg at bedtime.
85. A method for diagnosing cachexia comprising measuring at least two of the symptoms selected from the group consisting of: 1) a hyper-inflammatory state, 2) altered hormone levels and/or cytokine levels; 3) increased heart rate variability over normal; 4) weight loss, and 5) sustained increased heart rate over normal, wherein optionally the sustained increased heart rate is having a sustained elevated heart rate of at least about 6 bpm over normal.
86. The method of aspect 85, wherein the cachexia diagnosis comprises diagnosis of early onset cachexia or early stage of cachexia.
87. A computer implemented method for diagnosing cachexia, wherein the computer implemented method comprises analysis of data representing measurements of at least two of symptoms selected from the group consisting of: 1) a hyper-inflammatory state, 2) hormone levels and/or cytokine levels; 3) heart rate variability over normal; 4) weight loss, and 5) sustained increased heart rate over normal are analyzed and the presence of at least two of the symptoms indicates a readout of a diagnosis of cachexia.
88. The computer implemented method of aspect 87, wherein the cachexia diagnosis comprises diagnosis of early onset cachexia or early stage of cachexia.
89. A computer program product for implementing the computer implemented method of aspect 87.
90. A method for distinguishing anorexia from cachexia comprising measuring increased heart rate variability over normal and weight loss, wherein optionally the sustained increased heart rate is having a sustained elevated heart rate of at least about 6 bpm over normal, wherein increased heart rate variability over normal and weight loss identifies cachexia.

## Claims

1. A therapeutic combination, for use in the treatment of cachexia or of a maladaptive nutritional state secondary to a chronic Systemic Inflammatory Response State (SIRS), wherein the combination comprises at least one member of a first group and at least one member of a second group;
wherein members of the first group are selected from the group consisting of angiotensin receptor blockers (ARBs); and
members of the second group are selected from the group consisting of non-steroidal anti-inflammatory drugs (NSAIDs).

2. The therapeutic combination of claim 1, further comprising
at least one member of a third group, wherein the member of the third group is different from the selected members of the first and second groups, and members of the third group are selected from the group consisting of statins, beta adrenergic receptor antagonists (beta blockers), angiotensin-converting enzyme (ACE) inhibitors, anabolic steroids, natural oils, fatty acids, and combinations thereof; and optionally
at least one member of a fourth group, wherein the member of the fourth group is different from the selected members of the first, second and third groups, and members of the fourth group are selected from the group consisting of statins, beta adrenergic receptor antagonists (beta blockers), angiotensin-converting enzyme (ACE) inhibitors, anabolic steroids, natural oils, fatty acids, and combinations thereof; and optionally
at least one member of a fifth group, wherein the member of the fifth group is different from the selected members of the first, second, third and fourth groups, and members of the fifth group are selected from the group consisting of statins, beta adrenergic receptor antagonists (beta blockers), angiotensin-converting enzyme (ACE) inhibitors, anabolic steroids, natural oils, fatty acids, and combinations thereof; and optionally
at least one member of a sixth group, wherein the member of the sixth group is different from the selected members of the first, second, third, fourth and fifth groups, and members of the sixth group are selected from the group consisting of statins, beta adrenergic receptor antagonists (beta blockers), angiotensin-converting enzyme (ACE) inhibitors, anabolic steroids, natural oils, fatty acids, and combinations thereof.

3. The therapeutic combination of claim 1 to 2, wherein each member of each selected group is formulated as a separate composition, or wherein all selected members are formulated in the same composition.

4. The therapeutic combination of any of claims 1 to 3, the angiotensin receptor blocker (ARB) comprises candesartan, eprosartan, irbesartan, losartan, olmesartan, telmisartan, valsartan or a combination thereof.

5. The therapeutic combination of any of claims 1 to 4, wherein the non-steroidal anti-inflammatory drugs (NSAID) comprises aspirin, diclofenac; diflunisal, etodolac, fenoprofen, flurbiprofen, ibuprofen, indomethacin, ketoprofen; ketorolac, meclofenamate, mefenamic acid, meloxicam, nabumetone, naproxen, oxaprozin, piroxicam, salsalate, sulindac, tolmetin, a COX-2-selective inhibitor or a combination thereof,
wherein optionally the COX-2-selective inhibitor comprises celecoxibrofecoxib, etoricoxib, valdecoxib, parecoxib, meloxicam or lumiracoxib.

6. The therapeutic combination of any of claims 2 to 5, wherein the beta adrenergic receptor antagonist (beta blocker) comprises atenolol, nadolol, metoprolol, propranolol, carteolol, carvedolol, labetalol, oxprenolol, penbutolol, pindolol, sotalol, timolol or a combination thereof, wherein the angiotensin-converting enzyme (ACE) inhibitor comprises benazepril, captopril, cilazapril, enalapril, enalaprilat, fosinopril, imidapril, lisinopril, moexipril, perindopril, quinapril, ramipril, trandolapril or a combination thereof, the anabolic steroid comprises andarine, ethylestrenol, mesterolone, methandrostenolone, methenolone, methyltestosterone, oxandrolone, oxymetholonestanozolol, boldenone, hexoxymestrolum, methandrostenolone, methenoloneenanthate, nandrolonedecanoate, nandrolonephenproprionate, stanozolol, stenbolone, testosterone cypionate, testosterone enanthate, testosteron, testosterone nicotinate, therobolin, trenbolone, trenbolone, trophobolene or a combination thereof, and/or the natural oil or fatty acid comprises an omega-3 fatty acid, a fish oil, a long-chain polyunsaturated fatty acid, an n-3 and/or n-6 highly unsaturated fatty acid, eicosapentaenoic acid (EPA), docosahexaenoic acid (DHA) or a combination thereof.

7. The therapeutic combination of any of claims 1 to 6, wherein the combination comprises at least one member of a third group selected from the group consisting of statins.

8. The therapeutic combination of any of claims 2 to 7, wherein the statin comprises atorvastatin, fluvastatin, lovastatin, pravastatin, rosuvastatin or simvastatin.

9. The therapeutic combination of any of claims 1 to 8, for use in the treatment of cachexia.

10. The therapeutic combination of any one of claims 1 to 8, for use in the treatment of a maladaptive nutritional state secondary to a chronic Systemic Inflammatory Response State (SIRS).

11. The combination of claim 10, wherein the maladaptive nutritional state comprises cachexia.

12. The combination of claim 9, wherein the cachexia comprises cachexia secondary to cancer.

13. A product of manufacture comprising a blister package, a clamshell, a tray or a shrink wrap comprising a therapeutic combination as defined in any of claims 1 to 12,
wherein optionally the blister package, clamshell, tray or shrink wrap comprises at least two dosages of a member of the first group and at least two dosages of a member of the second group,
and optionally the drugs are organized or labelled in the blister package, clamshell, tray or shrink wrap for usage by an individual for at least two administrations, one in the morning and one in the evening.
